# EUROPÄISCHE PATENTANMELDUNG

(11) **EP 2 149 551 A1**
(43) Veröffentlichungstag der Anmeldung: **03.02.2010**
(21) Anmeldenummer: 08161438.0
(22) Anmeldetag: 30.07.2008
(51) Int. Cl.: C07D 209/16, C07D 209/42, A61K 31/4045, C07D 401/12, C07D 403/12, C07D 405/12, C07D 409/12, C07D 417/12, C07D 471/04

(54) **N-(Indol-3-ylalkyl)-(hetero)arylamidderivate als Modulatoren des EP2-Rezeptors**

(71) Anmelder: Bayer Schering Pharma Aktiengesellschaft, 13353 Berlin (DE)
(72) Erfinder: Buchmann, Dr. Bernd, 16540 Hohen Neuendorf (DE); Bräuer, Dr. Nico, 14612 Falkensee (DE); Koppitz, Dr. Marcus, 10115 Berlin (DE); Peters, Dr. Olaf, 99891 Tabarz (DE); Laak, Dr. Antonius ter, 12159 Berlin (DE); Lindenthal, Dr. Bernhard, 13507 Berlin (DE); Langer, Dr. Gernot, 14612 Falkensee (DE); Wintermantel, Dr. Tim, 50670 Köln (DE)

(57) **Zusammenfassung**

Die vorliegende Erfindung betrifft erweiterte Benzamid-Derivate der allgemeinen Formel I, 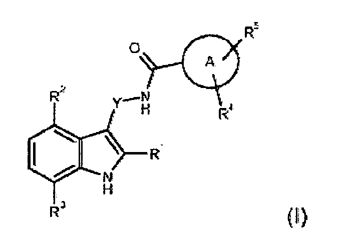 in der
A ein C₆-C₁₂-Aryl- oder C₅-C₁₂-Heteroarylrest, der gegebenenfalls ein- oder mehrfach mit R⁴ und/oder R⁵ substituiert sein kann,
R¹ ein C₁-C₆-Alkylrest, der gegebenenfalls substituiert sein kann,
R² ein Wasserstoff, Halogen, Cyano, ein -S(O)_{q}-CH₃, wobei q für 0 - 2 steht, ein C₁-C₄-Alkoxyrest oder C₁-C₆-Alkyl, wobei dieser Rest beliebig substituiert sein kann,
R³ ein Wasserstoff, ein C₁-C₆-Alkylrest, Cyano, Chlor oder Brom, und
Y eine -(CH₂)ₙ-Gruppe, wobei n für 1 - 3 steht, bedeuten,
Verfahren zu ihrer Herstellung sowie deren Verwendung zur Herstellung von pharmazeutischen Mitteln zur Behandlung von Erkrankungen und Indikationen, die im Zusammenhang stehen mit dem EP₂ Rezeptor.

## Beschreibung

Die vorliegende Erfindung betrifft erweiterte Benzamid-Derivate als EP₂ Rezeptor Modulatoren, Verfahren zu ihrer Herstellung sowie ihre Verwendung als Arzneimittel.

Es ist schon lange bekannt, dass Prostaglandine Schlüsselmoleküle bei den Prozessen der weiblichen Reproduktionsbiologie, wie z. B. der Regulation der Ovulation, der Fertilisation, der Nidation, der Dezidualisierung (z.B. Plazentabildung) und der Menstruation, sind. Prostaglandine spielen ebenfalls eine wichtige Rolle bei den pathologischen Veränderungen im reproduktiven Trakt, einschließlich der Menorrhagie, Dysmenorrhoe, Endometriose und Krebs. Bisher ist der Mechanismus, durch den Prostaglandine diese Veränderungen bewirken, noch nicht vollständig geklärt. Neuere Erkenntnisse weisen darauf hin, dass Prostaglandine, ihre Rezeptoren und deren Signaltransduktionswege an Prozessen wie Angiogenese, Apoptose, Proliferation sowie an inflammatorischen/antiinflammatorischen und immunologischen Prozessen beteiligt sind.

Die Wirkungen der Prostaglandine werden durch ihre G-Protein-gekoppelten Rezeptoren, die auf der Zelloberfläche lokalisiert sind, vermittelt. Von besonderem Interesse ist das Prostaglandin E₂ (PGE₂), das unterschiedlichste zelluläre Wirkungen erzielt, indem es an funktionell verschiedene Rezeptorensubtypen, nämlich die EP₁, EP₂, EP₃ und EP₄ Rezeptoren, bindet. Die Rezeptor-Subtypen, an denen das Prostaglandin E₂ bindet, scheinen für die Rezeptor vermittelten Wirkungen, die bei der Fertilitätsregulation eine Rolle spielen, von besonderem Interesse zu sein. So konnte gezeigt werden, dass die reproduktiven Funktionen bei EP₂ Knock-out Mäusen (EP₂ ^{-/-}), d.h. bei Mäusen, die keinen funktionstüchtigen PGE₂-Rezeptor Subtyp EP₂ mehr besitzen, beeinträchtigt sind, und dass diese Tiere eine kleinere "Wurfanzahl" haben (Matsumoto et al., 2001, Biology of Reproduction 64, 1557-1565). Ebenso konnte gezeigt werden, dass diese EP₂ Knock-out Mäuse (Hizaki et al. Proc Natl Acad Sci U. S. A. 1999 Aug 31; 96(18):10501-10506) eine deutlich verminderte Kumulus Expansion und starke Subfertilität aufweisen, was in ursächlichem Zusammenhang mit verminderten Reproduktionsprozessen wie der Ovulation und Fertilisierung zu sehen ist.

Der EP₂-Rezeptor stellt demnach ein wichtiges Ziel für die Entwicklung von Arzneimitteln für die Regulation der weiblichen Fertilität dar. Die Existenz der 4 Subklassen des PGE₂-Rezeptors eröffnet die Möglichkeit zur gezielten Entwicklung selektiv wirksamer Verbindungen. Bisher sind allerdings kaum selektive EP₂-Rezeptor-Liganden bekannt, die an den EP₂-Subtypen des PGE₂-Rezeptors binden, da die meisten bekannten Verbindungen auch an die anderen PGE₂-Rezeptor-Subtypen, wie beispielsweise an den EP₄-Rezeptor, binden.

EP₂-Rezeptor Antagonisten werden beispielsweise in den Anmeldungen US2005059742 und EP1467738 beschrieben (Jabbour, Medical Research Concil). Beansprucht wird eine Methode, bei der ein EP₂ und/oder ein EP₄ Antagonist zur Behandlung von Menorrhagie und Dysmenorrhoe eingesetzt werden kann. AH6809 wird als Antagonist des EP₂ oder EP₄ Rezeptors offenbart, es werden keine anderen spezifischen Antagonisten und keine neuen Verbindungen offenbart.

Ono Pharmaceutical beansprucht in der Anmeldung WO03/016254 die Herstellung von Benzolsäure oder gesättigten Carbonsäure-Derivaten, die mit Aryl oder Heterocyclen substituiert sind, unter anderem als PGE₂-Rezeptor Antagonisten. Die offenbarten Verbindungen werden für die Behandlung einer Vielzahl von Erkrankungen beansprucht, darunter auch allergische Erkrankungen, Morbus Alzheimer, Schmerz, Abort, Menstruationsbeschwerden, Menorrhagie und Dysmenorrhoe, Endometriose, Erkrankungen der Knochen, Ischämie usw. Die beschriebenen Verbindungen zeichnen sich jedoch durch eine besonders hohe Affinität zum EP₃ Rezeptor aus. In einer weiteren Anmeldung (WO04/032964) werden neue Verbindungen beschrieben, die sich ebenfalls durch eine besonders hohe Affinität zum EP₃ Rezeptor auszeichnen, aber auch als EP₂ Antagonisten, für die Behandlung und Prophylaxe von allergischen Erkrankungen Verwendung finden.

In der Anmeldung WO04/39807 der Firma Merck Frosst, Canada, wird die Herstellung von Pyridopyrrolizinen und Pyridoindolizinen offenbart. Diese Verbindungen zeichnen sich jedoch durch gute Bindung an den PGD₂ Rezeptor aus, dieser Rezeptor stellt einen anderen Subtyp des Prostaglandinrezeptors dar.

Naphathalinderivate als EP₄-Rezeptor Liganden werden von der SmithKline Beecham Corporation in der Anmeldung US2004102508 offenbart. Die beanspruchten Verbindungen finden ihre Verwendung für die Behandlung oder Prophylaxe von Schmerz, allergischen Reaktionen und neurodegenerativer Erkrankungen.

EP₄-Antagonisten (y-Lactame) werden in der Anmeldung WO03/103604 beansprucht (Applied Research Systems). Die Verbindungen binden ca. 60fach besser an den EP₄- als an den EP₂-Rezeptor und werden unter anderem zur Behandlung von vorzeitigen Wehen, Dysmenorrhoe, Asthma, Unfruchtbarkeit oder Fertilitätsstörungen beansprucht. Von derselben Firma werden in den Anmeldungen WO03/053923 (substituierte Pyrrolidine) oder WO03/035064 (substituierte Pyrazolidinone) Verbindungen für die Behandlung von Erkrankungen, die mit Prostaglandinen assoziiert sind, wie beispielsweise Infertilität, Hypertension und Osteoporose, beansprucht. Die Verbindungen binden an den EP₄-und an den EP₂ Rezeptor-Subtypen. In der Anmeldung WO03/037433 werden ω-Cycloalkyl, 17 Heteroaryl-Prostaglandinderivate als EP₂-Rezeptor Antagonisten, insbesondere für die Behandlung von erhöhtem Augeninnendruck, beansprucht.

In der Anmeldung WO03/064391 (Pfizer Products) werden Metabolite von [3-[[N-(4-tert-butylbenzyl)(pyridin-3-ylsulfonyl)amino]methyl] Essigsäure beschrieben, die die Bindung von [³H] Prostaglandin-E₂ an den EP₂-Rezeptor inhibieren. Die Verwendung dieser Metabolite zur Behandlung von Osteoporose wird offenbart.

Tani *et al*. beanspruchen in der Anmeldung US2005124577 8-Azaprostaglandinderivate für die Behandlung von immunologischen Erkrankungen, allergischen Erkrankungen, vorzeitigen Wehen, Abort usw. Die Verbindungen binden an den EP₂ und an den EP₄ Rezeptor.

In der europäischen Patentanmeldung EP 1306087 werden EP₂-Rezeptor Agonisten beschrieben, die ihre Verwendung bei der Behandlung der erektilen Dysfunktion finden (Ono Pharmaceuticals). Die gleiche Strukturklasse wird in dem europäischen Patent EP 860430 (Ono Pharmaceuticals) beschrieben, ihre Verwendung für die Herstellung eines Medikaments für die Behandlung von immunologischen Erkrankungen, Asthma und Abort wird beansprucht. In der WO04/009117 werden EP₂- und EP₄-Rezeptor Agonisten für die Behandlung von Erkrankungen beschrieben, die verursacht werden durch die Uteruskontraktion, beispielsweise Menstruationsbeschwerden (Ono Pharmaceuticals).

In den Anmeldungen WO03/74483 und WO03/09872 werden Agonisten beschrieben, die gleichermaßen an den EP₂ und an den EP₄ Rezeptor binden (Ono Pharmaceuticals).

Die Agonisten des EP₂ und des EP₄ Rezeptors werden häufig im Zusammenhang mit der Behandlung der Osteoporose beschrieben (WO99/19300 (Pfizer), US2003/0166631 (Dumont Francis), WO03/77910 (Pfizer), WO03/45371 (Pfizer), WO03/74483 und WO03/09872 (Ono Pharmaceuticals)) und für die Glaukombehandlung (WO04/37813, WO04/37786, WO04/19938, WO03/103772, WO03/103664, WO03/40123, WO03/47513, WO03/47417 (Merck Frosst Canada)) und US6410591 und US6747037 (Allergan).

In den Anmeldungen WO05/035514 (Vertex) und JP2007045752 (Takeda) werden Indolylamine offenbart, allerdings nicht als Liganden des EP₂ Rezeptors. In der Anmeldung WO05/035514 werden die Substanzen als Modulatoren von ATP Bindungskassetten-Transporter beschrieben.

In der Patentanmeldung WO04/12656 (Applied Research Systems) werden EP₂-Rezeptor Agonisten im Zusammenhang mit Inflammation beansprucht. In der Patentanmeldung WO03/77919 (Merck & Co. Inc.) werden EP₄-Rezeptor Agonisten für die Behandlung der Fertilität beansprucht.

Bisher sind jedoch keine selektiven EP₂-Rezeptor Agonisten und Antagonisten bekannt, die die Prozesse regulieren, die letztendlich für die Ovulation, Fertilisierung, Nidation und Dezidualisierung verantwortlich sind und somit zur Förderung oder Hemmung der Fertilität beitragen.
In den Patentanmeldungen der Bayer Schering Pharma AG (WO2007/057232, W02007/071456, WO2008/028689, WO2008/028690 WO2008/028691 werden erstmalig selektive Antagonisten des EP₂ Rezeptors beansprucht. Jedoch binden die beanspruchten Verbindungen nur mit einer Bindungsaffinität im mikromolaren Bereich.

Daher sind bisher nur schwach wirksame EP₂-Rezeptor Agonisten und Antagonisten bekannt. Für die Regulation der Prozesse, die letztendlich für die Ovulation, Fertilisierung, Nidation und Dezidualisierung verantwortlich sind und die somit zur Förderung oder Hemmung der Fertilität beitragen, werden jedoch Verbindungen benötigt, die mit einer hohen Bindungsaffinität selektiv an den EP₂-Rezeptor binden.

Aufgabe der vorliegenden Erfindung war es daher, potentere, selektive Antagonisten des EP₂-Rezeptor zur Verfügung zu stellen.

Diese Aufgabe wurde gelöst durch die Bereitstellung der Verbindungen der allgemeinen Formel I 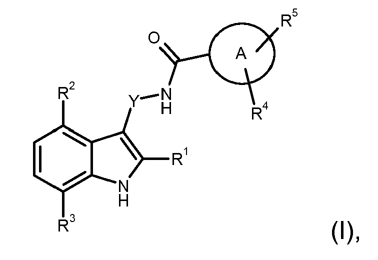 in der
- A: ein C₆-C₁₂-Aryl- oder C₅-C₁₂-Heteroarylrest, der gegebenenfalls ein- oder mehrfach mit R⁴ und/oder R⁵ substituiert sein kann,
- R¹: ein C₁-C₆-Alkylrest, der gegebenenfalls substituiert sein kann,
- R²: ein Wasserstoff, Halogen, Cyano, ein -S(O)_{q}-CH₃, wobei q für 0 - 2 steht, ein C₁-C₄-Alkoxyrest oder C₁-C₆-Alkyl, wobei dieser Rest beliebig substituiert sein kann,
- R³: ein Wasserstoff, ein C₁-C₆-Alkylrest, Cyano, Chlor oder Brom,
- R⁴: ein Wasserstoff, Halogen, Amino, eine -S(O)ₚ-C₁-C₆-Alkylgruppe, wobei p 0 - 2 bedeutet,
ein C₁-C₆-Acyl-, NH-CO-NH₂, -O-CO-NH(C₁-C₆-Alkyl), -O-CO-N(C₁-C₆-Alkyl)₂ oder NH-CO-C₁-C₆-Alkylrest,
ein C₁-C₆-Alkyl, welches gegebenenfalls ein- oder mehrfach, gleich oder verschieden mit C₁-C₆-Acyl, C₁-C₆-Alkoxy, Hydroxy, Cyano, CO₂-(C₁-C₆-Alkyl), N-(C₁-C₆-Alkyl)₂, C₅-C₁₂-Heteroaryl, COOH, CO-NH₂, CO-NH(C₁-C₆-Alkyl) oder mit CO-N(C₁-C₆-Alkyl)₂ substituiert sein kann,
ein C₁-C₆-Alkoxy, welches gegebenenfalls ein- oder mehrfach, gleich oder verschieden mit Hydroxy, Cyano, CO₂-(C₁-C₆-Alkyl), N-(C₁-C₆-Alkyl)₂, NH-C₃-C₆-Cycloalkyl, COOH, CO-NH₂, CO-NH(C₁-C₆-Alkyl) oder mit CO-N(C₁-C₆-Alkyl)₂ substituiert sein kann, ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₆-Alkyl), CO-NH(C₅-C₁₂-Heteroaryl), NH-(C₁-C₆-Alkyl), N-(C₁-C₆-Alkyl)₂,
ein C₆-C₁₂-Aryl, welches gegebenenfalls ein- oder mehrfach, gleich oder verschieden mit Halogen, mit C₁-C₆-Alkyl, C₃-C₆-Cycloalkyl, C₁-C₆-Acyl, C₁-C₆-Alkoxy, C₆-C₁₂-Aryl, C₅-C₁₂-Heteroaryl, Hydroxy, CH₂-OH, Cyano, CH₂-CN, Amino, CO₂-(C₁-C₆-Alkyl), N-(C₁-C₆-Alkyl)₂, NHSO₂CH₃, SO₂NH₂, SO₂NH(C₁-C₆-Alkyl), SO₂N(C₁-C₆-Alkyl)₂, COOH, CO-NH₂, CO-NH(C₁-C₆-Alkyl), CO-N(C₁-C₆-Alkyl)₂, CO-NH(C₅-C₁₂-Heteroaryl), NH-CO(C₁-C₆-Alkyl), CH₂-NH-CO(C₁-C₆-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₆-Alkyl substituiert sein kann,
unter der Voraussetzung, dass R² Cyano bedeutet oder R¹ und/oder R² gleich oder verschieden einen C₁-C₆-Akylrest bedeuten, wobei mindestens einer der Reste mindestens einfach substituiert ist oder
unter der Voraussetzung, dass R⁵ ein -S(O)ₚ-C₁-C₆-Alkyl bedeutet, wobei p für 0 - 2 steht, ein C₁-C₆-Acyl-, -O-CO- NH(C₁-C₆-Alkyl), -O-CO-N(C₁-C₆-Alkyl)₂, C₆-C₁₂-Aryloxy, C₅-C₁₆-Heteroaryloxy, Hydroxy, Cyano oder N-(C₁-C₆-Alkyl)₂ ist,
ein monocyclisches C₅-C₇-Heteroaryl, welches mindestens ein- oder mehrfach, gleich oder verschieden mit Halogen, CF₃, C₁-C₆-Acyl, C₁-C₆-Alkoxy, Hydroxy, CH₂-OH, Cyano, CO₂-(C₁-C₆-Alkyl), N-(C₁-C₆-Alkyl)₂, COOH, CO-NH₂, CO-NH(C₁-C₆-Alkyl) oder CON(C₁-C₆-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₆-Alkyl, C₁-C₆-Acyl, C₁-C₆-Alkoxy, Hydroxy, Cyano, CO₂-(C₁-C₆-Alkyl), N-(C₁-C₆-Alkyl)₂, COOH, CO-NH₂, CONH(C₁-C₆-Alkyl) oder CO-N(C₁-C₆-Alkyl)₂ substituiert sein kann oder
ein C₃-C₆-Cycloalkyl, welches gegebenenfalls ein- oder mehrfach, gleich oder verschieden mit Halogen, mit C₁-C₆-Alkyl, Hydroxy, Cyano, CO₂-(C₁-C₆-Alkyl), C₁-C₆-Acyl, N-(C₁-C₆-Alkyl)₂, COOH, CO-NH₂, CO-NH(C₁-C₆-Alkyl), CO-N(C₁-C₆-Alkyl)₂ oder C₁-C₆-Alkoxy substituiert sein kann,
- R⁵: ein Wasserstoff, Halogen, Amino, -S(O)ₚ-C₁-C₆-Alkyl, wobei p 0 - 2 bedeutet,
eine C₁-C₆-Acyl-, NH-CO-NH₂-, NH-CO-C₁-C₆-Alkyl-, -O-CO- NH(C₁-C₆-Alkyl)-, -O-CO-N(C₁-C₆-Alkyl)₂ oder C₁-C₆-Alkylgruppe, die gegebenenfalls ein- oder mehrfach, gleich oder verschieden mit C₁-C₆-Acyl, C₁-C₆-Alkoxy, Hydroxy, Cyano, CO₂-(C₁-C₆-Alkyl), N-(C₁-C₆-Alkyl)₂, C₅-C₁₂-Heteroaryl, COOH, CO-NH₂, CO-NH(C₁-C₆-Alkyl) oder mit CO-N(C₁-C₆-Alkyl)₂ substituiert sein kann,
ein C₁-C₆-Alkoxy, welches gegebenenfalls ein- oder mehrfach, gleich oder verschieden mit Hydroxy, Cyano, CO₂-(C₁-C₆-Alkyl), N-(C₁-C₆-Alkyl)₂, NH-C₃-C₆-Cycloalkyl, COOH, CO-NH₂, CO-NH(C₁-C₆-Alkyl) oder mit CO-N(C₁-C₆-Alkyl)₂substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₆-Alkyl), CO-NH(C₅-C₁₂-Heteroaryl), NH-(C₁-C₆-Alkyl), N-(C₁-C₆-Alkyl)₂,
ein C₆-C₁₂-Aryl, welches gegebenenfalls ein- oder mehrfach, gleich oder verschieden mit Halogen, mit C₁-C₆-Alkyl, C₃-C₆-Cycloalkyl, C₁-C₆-Acyl, C₁-C₆-Alkoxy, C₆-C₁₂-Aryl, C₅-C₁₂-Heteroaryl, Hydroxy, CH₂-OH, Cyano, CH₂-CN, Amino, CO₂-(C₁-C₆-Alkyl), N-(C₁-C₆-Alkyl)₂, NHSO₂CH₃, SO₂NH₂, SO₂NH(C₁-C₆-Alkyl), SO₂N(C₁-C₆-Alkyl)₂, COOH, CO-NH₂, CO-NH(C₁-C₆-Alkyl), CO-N(C₁-C₆-Alkyl)₂, CO-NH(C₅-C₁₂-Heteroaryl), NH-CO(C₁-C₆-Alkyl), CH₂-NH-CO(C₁-C₆-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₆-Alkyl substituiert sein kann, wenn R² gleich Cyano ist oder unter der Voraussetzung, dass R¹ und/oder R² gleich oder verschieden ein C₁-C₆-Akylrest bedeuten, wobei mindestens einer der Reste mindestens einfach substituiert ist,
ein monocyclisches C₅-C₇-Heteroaryl, welches mindestens ein- oder mehrfach, gleich oder verschieden mit Halogen, CF₃, C₁-C₆-Acyl, C₁-C₆-Alkoxy, Hydroxy, CH₂-OH, Cyano, CO₂-(C₁-C₆-Alkyl), N-(C₁-C₆-Alkyl)₂, COOH, CO-NH₂, CO-NH(C₁-C₆-Alkyl) oder CON(C₁-C₆-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₆-Alkyl, C₁-C₆-Acyl, C₁-C₆-Alkoxy, Hydroxy, Cyano, CO₂-(C₁-C₆-Alkyl), N-(C₁-C₆-Alkyl)₂, COOH, CO-NH₂, CONH(C₁-C₆-Alkyl) oder CO-N(C₁-C₆-Alkyl)₂ substituiert sein kann oder
ein C₃-C₆-Cycloalkyl, welches gegebenenfalls ein- oder mehrfach, gleich oder verschieden mit Halogen, mit C₁-C₆-Alkyl, Hydroxy, Cyano, CO₂-(C₁-C₆-Alkyl), C₁-C₆-Acyl, N-(C₁-C₆-Alkyl)₂, COOH, CO-NH₂, CO-NH(C₁-C₆-Alkyl), CO-N(C₁-C₆-Alkyl)₂ oder C₁-C₆-Alkoxy substituiert sein kann,
- R⁴ und R⁵: entweder in ortho-, meta- oder meta,para-Stellung zueinander stehen und gemeinsam die Bedeutung haben -O-CO-S-, -S-CO- O-, CH₂-CO-O-, O-CO-CH₂-, -CH₂-CO-NH-, -NH-CO-CH₂-, -O-CO- NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
- Y: eine -(CH₂)ₙ-Gruppe, wobei n für 1 - 3 steht,
bedeuten, sowie deren Isomere, Diastereomere, Enantiomere und Salze bzw. Cyclodextrinclathrate, die die bekannten Nachteile überwinden und verbesserte Eigenschaften aufweisen, d.h. mit hoher Bindungsaffinität an den EP₂-Rezeptor binden, eine gute Wirksamkeit, gute Löslichkeit und Stabilität aufweisen, wobei die folgenden Verbindungen ausgeschlossen sind:
4-Chlor-1,3-dimethyl-N-[2-(2-methyl-1H-indol-3-yl)ethyl]-1H-pyrazol-5-carbonsäureamid
N-[2-(2-Methyl-1 H-indol-3-yl)ethyl]-6-chinoxalincarbonsäureamid
3,5-Dimethyl-N-[2-(2-methyl-1 H-indol-3-yl)ethyl]-4-isoxazolcarbonsäureamid
5,7-Dimethyl-N-[2-(2-methyl-1 H-indol-3-yl)ethyl]-pyrazolo[1,5-a]pyrimidin-2-carbonsäureamid
N-[2-(2-Methyl-1 H-indol-3-yl)ethyl]-2-methylbenzamid
N-[2-(2-Methyl-1 H-indol-3-yl)ethyl]-2-(difluoromethyl)benzamid
3-lod-1-methyl-N-[2-(2-methyl-1H-indol-3-yl)ethyl]-1H-pyrol-2-carbonsäureamid 1-Methyl-N-[2-(2-methyl-1H-indol-3-yl)ethyl]-4-(trifluormethyl)-1H-pyrol-3-carbonsäureamid
2-lod-N-[2-(2-methyl-1 H-indol-3-yl)ethyl]-3-thiophencarbonsäureamid
3-(Difluormethyl)-1-methyl-N-[2-(2-methyl-1H-indol-3-yl)ethyl]-1H-pyrazol-4-carbonsäureamid
3-(Trifluormethyl)-1-methyl-N-[2-(2-methyl-1H-indol-3-yl)ethyl]-1H-pyrazol-4-carbonsäureamid
5-Fluor-1,3-dimethyl-N-[2-(2-methyl-1H-indol-3-yl)ethyl]-1H-pyrazol-4-carbonsäureamid
4-(Difluormethyl)-2-methyl-N-[2-(2-methyl-1 H-indol-3-yl)ethyl]-5-thiazolcarbonsäureamid
4-(Trifluormethyl)-2-methyl-N-[2-(2-methyl-1 H-indol-3-yl)ethyl]-5-thiazolcarbonsäureamid
2-lod-N-[2-(2-methyl-1 H-indol-3-yl)ethyl]-3-furancarbonsäureamid
2,5-Dimethyl-N-[2-(2-methyl-1 H-indol-3-yl)ethyl]-3-furancarbonsäureamid
5-Methyl-N-[2-(2-methyl-1 H-indol-3-yl)ethyl]-2-(trifluormethyl)-3-furancarbonsäureamid
2-Methyl-N-[2-(2-methyl-1 H-indol-3-yl)ethyl]-3-furancarbonsäureamid
3-Methyl-N-[2-(2-methyl-1H-indol-3-yl)ethyl]-2-thiophencarbonsäureamid
3-lod-N-[2-(2-methyl-1H-indol-3-yl)ethyl]-2-thiophencarbonsäureamid
3-Brom-N-[2-(2-methyl-1H-indol-3-yl)ethyl]-2-thiophencarbonsäureamid
4-Methoxy-N-[2-(2-methyl-1H-indol-3-yl)ethyl]-benzamid
N-[2-(7-Chlor-2-methyl-1H-indol-3-yl)ethyl]-2-methyl-7-benzoxazolcarbonsäureamid
4-(1,1-Dimethylethyl)-N-[2-(2-methyl-1H-indol-3-yl)ethyl]-benzamid
N-[2-(2,7-Dimethyl-1H-indol-3-yl)ethyl]-3,4-dimethoxy-benzamid
2-Ethoxy-N-[2-(2-methyl-1H-indol-3-yl)ethyl]-benzamid
2-(1-Methylethoxy)-N-[2-(2-methyl-1H-indol-3-yl)ethyl]-benzamid
2-Chlor-4,5-difluor-N-[2-(2-methyl-1H-indol-3-yl)ethyl]-benzamid
4-Brom-N-[2-(4-fluoro-2,7-dimethyl-1H-indol-3-yl)ethyl]-benzamid
2,6-Difluor-N-[2-(2-methyl-1H-indol-3-yl)ethyl]-benzamid
3,4-Difluor-N-[2-(2-methyl-1H-indol-3-yl)ethyl]-benzamid
N-[2-(2-Methyl-1H-indol-3-yl)ethyl]-4-(trifluormethyl)-benzamid
N-[2-(2-Methyl-1H-indol-3-yl)ethyl]-4-methyl-benzamid
2,4-Dichlor-N-[2-(2-methyl-1H-indol-3-yl)ethyl]-benzamid
2-Brom-N-[2-(2-Methyl-1H-indol-3-yl)ethyl]-benzamid
4-Fluor-N-[2-(2-methyl-1H-indol-3-yl)ethyl]-benzamid
N-[2-(4-Fluor-2,7-dimethyl-1H-indol-3-yl)ethyl]-2-methyl-benzamid
N-[2-(4-Fluor-2,7-dimethyl-1H-indol-3-yl)ethyl]-2-methoxy-benzamid
3,4-Dimethoxy-N-[2-(4-Fluor-2,7-dimethyl-1H-indol-3-yl)ethyl]-benzamid
4-[[[2-(4-Fluor-2,7-dimethyl-1H-indol-3-yl)ethyl]amino]carbonyl]-benzoesäure methyl ester
N-[2-(4-Fluor-2,7-dimethyl-1H-indol-3-yl)ethyl]-1-methyl-1H-pyrazol-5-carbonsäureamid
N-[2-(7-Ethyl-2-methyl-1H-indol-3-yl)ethyl]-2-methoxy-benzamid
4-Chlor-N-[2-(2-methyl-1H-indol-3-yl)ethyl]-benzamid
2,6-Dichlor-N-[2-(2-methyl-1H-indol-3-yl)ethyl]-benzamid
5-Chlor-2-methoxy-N-[2-(2-methyl-1H-indol-3-yl)ethyl]-benzamid
3,4-Dichlor-N-[2-(2-methyl-1H-indol-3-yl)ethyl]-benzamid
2-Fluor-N-[2-(2-methyl-1H-indol-3-yl)ethyl]-benzamid
4-Chlor-1-methyl-N-[2-(2-methyl-1H-indol-3-yl)ethyl]-1H-pyrazol-5-carbonsäureamid
4-[[[2-(2-Methyl-1H-indol-3-yl)ethyl]amino]carbonyl]-benzoesäure methylester
N-[2-(2-Methyl-1H-indol-3-yl)ethyl]-2-thiophencarbonsäureamid
3,4,5-Trimethoxy-N-[2-(2-methyl-1H-indol-3-yl)ethyl]-benzamid
3-Methoxy-N-[2-(2-methyl-1H-indol-3-yl)ethyl]-benzamid
N-[2-(2-methyl-1H-indol-3-yl)ethyl]-4-pyridincarbonsäureamid
N-[2-(2-methyl-1H-indol-3-yl)ethyl]-3-pyridincarbonsäureamid

Die erfindungsgemäßen Verbindungen haben eine antagonistische Wirkung am EP₂-Rezeptor und dienen somit der weiblichen Fertilitätskontrolle.

Unter C₁-C₄-Alkyl bzw. C₁-C₆-Alkyl ist jeweils ein geradkettiger oder verzweigter Alkylrest, wie beispielsweise Methyl, Ethyl, Propyl, Isopropyl, Butyl, Isobutyl, sek. Butyl, tert. Butyl, Pentyl, Isopentyl und Hexyl, zu verstehen.
Die Alkylreste können gegebenenfalls ein- oder mehrfach, gleich oder verschieden durch Halogen substituiert sein.

Unter C₁-C₄-Alkoxy bzw. C₁-C₆-Alkoxy ist jeweils ein geradkettiger oder verzweigter Alkoxyrest, wie beispielsweise Methoxy-, Ethoxy-, n-Propoxy-, iso-Propoxy-, n-Butoxy-, sec-Butoxy-, iso-Butoxy-, tert. Butyloxy-, Pentoxy-, iso-Pentoxy- und Hexoxy-, zu verstehen.
Die Alkoxyreste können gegebenenfalls ein- oder mehrfach, gleich oder verschieden durch Halogen substituiert sein.

Unter C₁-C₄-Acyl bzw. C₁-C₆-Acyl ist jeweils ein geradkettiger oder verzweigter Rest, wie beispielsweise Formyl, Acetyl, Propionyl, Butyroyl, iso-Butyroyl, Valeroyl und Benzoyl zu verstehen.
Die Acylreste können gegebenenfalls ein- oder mehrfach, gleich oder verschieden durch Halogen substituiert sein.

Unter C₃-C₆-Cycloalkyl sind monocyclische Alkylringe wie Cyclopropyl, Cyclobutyl, Cyclopentyl und Cyclohexyl zu verstehen.

Die Cycloalklyreste können anstelle der Kohlenstoffatome ein oder mehrere Heteroatome, wie Sauerstoff, Schwefel und/oder Stickstoff enthalten. Bevorzugt sind solche Heterocycloalkyle mit 3 bis 6 Ringatomen, wie beispielsweise Aziridinyl, Azetidinyl, Pyrrolidinyl, Piperidinyl, Piperazinyl, Morpholinyl. Unter den Ringsystemen, bei denen gegebenenfalls ein- oder mehrere mögliche Doppelbindungen im Ring enthalten sein können, sind zum Beispiel Cycloalkenyle wie Cyclopropenyl, Cyclobutenyl, Cyclopentenyl, Cyclopentadienyl, Cyclohexenyl, zu verstehen, wobei die Anknüpfung sowohl an der Doppelbindung wie auch an den Einfachbindungen erfolgen kann.

Unter Halogen ist jeweils Fluor, Chlor, Brom oder Jod zu verstehen.

Der C₆-C₁₂-Arylrest umfasst jeweils 6 - 12 Kohlenstoffatome und kann beispielsweise benzokondensiert sein. Beispielsweise genannt seien: Phenyl, Tropyl, Cyclooctadienyl, Indenyl, Naphthyl, Biphenyl, Fluorenyl, Anthracenyl etc. Ein entsprechender Aryloxyrest umfasst einen Arylrest, der über eine Sauerstoffbrücke verknüpft ist.

Unter dem monocyclischen C₅-C₇-Heteroarylrest, dem bicyclischen und tricyclischen C₈-C₁₂-Heteroarylrest und dem C₅-C₁₂ bzw. C₅-C₁₆-Heteroarylrest sind Ringsysteme zu verstehen, die jeweils 5 - 16 Ringatome enthalten und die anstelle des Kohlenstoffs ein- oder mehrere, gleiche oder verschiedene Heteroatome, wie Sauerstoff, Schwefel oder Stickstoff enthalten können, und
wobei der C₈-C₁₂- bzw. der C₅-C₁₆-Heteroarylrest mono-, bi- oder tricyclisch sein kann und zusätzlich jeweils benzokondensiert sein kann.
Beispielsweise seien genannt:
Thienyl, Furanyl, Pyrrolyl, Oxazolyl, Imidazolyl, Pyrazolyl, Isoxazolyl, Isothiazolyl, Oxadiazolyl, Triazolyl, Thiadiazolyl, etc. und Benzoderivate davon, wie z.B. Benzofuranyl, Benzothienyl, Benzooxazolyl, Benzimdazolyl, Indazolyl, Indolyl, Isoindolyl, etc; oder Pyridinyl, Pyridazinyl, Pyrimidinyl, Pyrazinyl, Triazinyl, etc. und Benzoderivate davon wie z.B. Chinolyl, Isochinolyl, etc; oder Azocinyl, Indolizinyl, Purinyl, etc. und Benzoderivate davon; oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, Oxepinyl, Benzotriazol, etc.

Der Heteroarylrest kann jeweils benzokondensiert sein. Beispielsweise seien als 5-Ringheteroaromaten genannt: Thiophen, Furan, Oxazol, Thiazol, Imidazol, Pyrazol und Benzoderivate davon und als 6-Ring-Heteroaromaten Pyridin, Pyrimidin, Triazin, Chinolin, Isochinolin und Benzoderivate.

Unter Heteroatomen sind Sauerstoff-, Stickstoff- oder Schwefel-Atome zu verstehen.
Ein entsprechender Heteroaryloxyrest umfasst einen Heteroarylrest, der über eine Sauerstoffbrücke verknüpft ist.

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethylglukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Bevorzugt sind solche Verbindungen der allgemeinen Formel (I),
wobei
- A: ein C₆-C₁₂-Aryl- oder C₅-C₁₂-Heteroarylrest, der gegebenenfalls ein- oder mehrfach mit R⁴ und/oder R⁵ substituiert sein kann,
- R¹: ein C₁-C₄-Alkylrest, der gegebenenfalls substituiert sein kann,
- R²: ein Wasserstoff, Halogen, ein C₁-C₄-Alkoxyrest oder C₁-C₆-Alkyl, wobei dieser Rest beliebig substituiert sein kann,
- R³: ein Wasserstoff, ein C₁-C₆-Alkylrest, Cyano, Chlor oder Brom, und die Reste R⁴, R⁵, R⁶, R⁷ und Y die oben angegebenen Bedeutung haben,
bedeuten, sowie deren Isomere, Diastereomere, Enantiomere und Salze bzw. Cyclodextrinclathrate.

Ebenfalls bevorzugt sind solche Verbindungen der allgemeinen Formel (I),
wobei
- A: ein C₆-C₁₂-Aryl- oder C₅-C₁₂-Heteroarylrest, der gegebenenfalls ein- oder mehrfach mit R⁴ und/oder R⁵ substituiert sein kann,
- R¹: ein C₁-C₄-Alkylrest,
- R²: ein Wasserstoff, Halogen oder C₁-C₄-Alkyl, wobei dieser Rest beliebig substituiert sein kann,
- R³: ein Wasserstoff, ein C₁-C₄-Alkylrest, Cyano, Chlor oder Brom,
- R⁴: ein Wasserstoff, Halogen, Amino, eine -S(O)ₚ-C₁-C₄-Alkylgruppe, wobei p 0 - 2 bedeutet,
ein C₁-C₄-Acyl-, NH-CO-NH₂, -O-CO-NH(C₁-C₄-Alkyl), -O-CO-N(C₁-C₄-Alkyl)₂ oder NH-CO-C₁-C₄-Alkylrest,
ein C₁-C₄-Alkyl, welches gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann, ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SP₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann,
unter der Voraussetzung, dass R² Cyano oder R¹ und/oder R² gleich oder verschieden einen C₁-C₄-Akylrest bedeuten, wobei mindestens einer der Reste mindestens einfach substituiert ist oder
unter der Voraussetzung, dass R⁵ ein -S(O)ₚ-C₁-C₄-Alkyl bedeutet, wobei p für 0 - 2 steht, ein C₁-C₆-Acyl-, -O-CO-NH(C₁-C₄-Alkyl), -O-CO-N(C₁-C₄-Alkyl)₂, C₆-C₁₂-Aryloxy, C₅-C₁₆-Heteroaryloxy, Hydroxy, Cyano oder N-(C₁-C₄-Alkyl)₂ bedeutet,
ein monocyclisches C₅-C₇-Heteroaryl, welches mindestens ein-oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CON(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CONH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
- R⁵: ein Wasserstoff, Halogen, Amino, -S(O)ₚ-C₁-C₄-Alkyl, wobei p 0 - 2 bedeutet,
eine C₁-C₄-Acyl-, NH-CO-NH₂-, NH-CO-C₁-C₄-Alkyl-, -O-CO-NH(C₁-C₄-Alkyl)-, -O-CO-N(C₁-C₄-Alkyl)₂ oder C₁-C₄-Alkylgruppe, die gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂- Heteroaryl), Styryl, oder ein -S(O)ᵣ-CH₃, wobei r für 0 - 2 steht, substituiert sein kann oder zwei benachbarte Positionen durch -O- CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann, unter derVoraussetzung, dass R² Cyano bedeutet oder wenn R¹ und/oder R² gleich oder verschieden ein C₁-C₄-Akylrest ist, wobei mindestens einer der Reste mindestens einfach substituiert ist,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CON(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CONH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
- R⁴ und R⁵: entweder in ortho-, meta- oder meta,para-Stellung zueinander stehen und gemeinsam die Bedeutung haben -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
- Y: eine -(CH₂)ₙ-Gruppe, wobei n für 1 - 3 steht,
bedeuten, sowie deren Isomere, Diastereomere, Enantiomere und Salze bzw. Cyclodextrinclathrate.

Ebenfalls bevorzugt sind solche Verbindungen der allgemeinen Formel (I),
wobei
- A: einen Phenyl-, Naphthyl- oder Heteroarylrest, der gegebenenfalls ein- oder zweifach mit R⁴ und/oder R⁵ substituiert sein kann,
- R¹: ein C₁-C₄-Alkylrest,
- R²: ein Wasserstoff, Halogen oder C₁-C₄-Alkyl, wobei dieser Rest beliebig substituiert sein kann,
- R³: ein Wasserstoff, ein C₁-C₄-Alkylrest, Cyano, Chlor oder Brom,
- R⁴: ein Wasserstoff, Halogen, Amino, eine -S(O)ₚ-C₁-C₄-Alkylgruppe, wobei p 0 - 2 bedeutet,
ein C₁-C₄-Acyl-, NH-CO-NH₂, -O-CO-NH(C₁-C₄-Alkyl), -O-CO-N(C₁-C₄-Alkyl)₂ oder NH-CO-C₁-C₄-Alkylrest,
ein C₁-C₄-Alkyl, welches gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂substituiert sein kann, ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann,
unter der Voraussetzung, dass R² Cyano oder R¹ und/oder R² gleich oder verschieden einen C₁-C₄-Akylrest bedeuten, wobei mindestens einer der Reste mindestens einfach substituiert ist oder
unter der Voraussetzung, dass R⁵ ein -S(O)ₚ-C₁-C₄-Alkyl bedeutet, wobei p für 0 - 2 steht, ein C₁-C₆-Acyl-, -O-CO-NH(C₁-C₄-Alkyl), -O-CO-N(C₁-C₄-Alkyl)₂, C₆-C₁₂-Aryloxy, C₅-C₁₆-Heteroaryloxy, Hydroxy, Cyano oder N-(C₁-C₄-Alkyl)₂ bedeutet,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CON(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CONH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,

- R⁵: ein Wasserstoff, Halogen, Amino, -S(O)ₚ-C₁-C₄-Alkyl, wobei p 0 - 2 bedeutet,
eine C₁-C₄-Acyl-, NH-CO-NH₂-, NH-CO-C₁-C₄-Alkyl-, -O-CONH(C₁-C₄-Alkyl)-, -O-CO-N(C₁-C₄-Alkyl)₂ oder C₁-C₄-Alkylgruppe, die gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder ein -S(O)ᵣ-CH₃, wobei r für 0 - 2 steht, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann, unter derVoraussetzung, dass R² Cyano bedeutet oder wenn R¹ und/oder R² gleich oder verschieden ein C₁-C₄-Akylrest ist, wobei mindestens einer der Reste mindestens einfach substituiert ist,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CON(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CONH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
- R⁴ und R⁵: entweder in ortho-, meta- oder meta,para-Stellung zueinander stehen und gemeinsam die Bedeutung haben -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
- Y: eine -(CH₂)₂-Gruppe steht,
bedeuten, sowie deren Isomere, Diastereomere, Enantiomere und Salze bzw. Cyclodextrinclathrate,

Ebenfalls bevorzugt sind solche Verbindungen der allgemeinen Formel (I),
wobei
- A: einen Phenyl-, Naphthyl- oder C₅-C₁₂-Heteroarylrest, der gegebenenfalls ein- oder zweifach mit R⁴ und/oder R⁵ substituiert sein kann,
- R¹: ein C₁-C₄-Alkylrest,
- R²: ein Wasserstoff, Fluor, Chlor, Brom oder C₁-C₄-Alkyl,
- R³: ein Wasserstoff, Methyl, Ethyl, Trifluormethyl, Cyano, Chlor oder Brom,
- R⁴: ein Wasserstoff, Halogen, Amino, eine -S(O)ₚ-C₁-C₄-Alkylgruppe,
wobei p 0 - 2 bedeutet,
ein C₁-C₄-Acyl-, NH-CO-NH₂, -O-CO-NH(C₁-C₄-Alkyl), -O-CO-N(C₁-C₄-Alkyl)₂ oder NH-CO-C₁-C₄-Alkylrest,
ein C₁-C₄-Alkyl, welches gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂substituiert sein kann, ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann,
unter der Voraussetzung, dass R² Cyano oder R¹ und/oder R² gleich oder verschieden einen C₁-C₄-Akylrest bedeuten, wobei
mindestens einer der Reste mindestens einfach substituiert ist oder
unter der Voraussetzung, dass R⁵ ein -S(O)ₚ-C₁-C₄-Alkyl bedeutet, wobei p für 0 - 2 steht, ein C₁-C₆-Acyl-, -O-CO-NH(C₁-C₄-Alkyl), -O-CO-N(C₁-C₄-Alkyl)₂, C₆-C₁₂-Aryloxy, C₅-C₁₆-Heteroaryloxy, Hydroxy, Cyano oder N-(C₁-C₄-Alkyl)₂ bedeutet,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CON(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CONH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
- R⁵: ein Wasserstoff, Halogen, Amino, -S(O)ₚ-C₁-C₄-Alkyl, wobei p 0 - 2 bedeutet,
eine C₁-C₄-Acyl-, NH-CO-NH₂-, NH-CO-C₁-C₄-Alkyl-, -O-CO-NH(C₁-C₄-Alkyl)-, -O-CO-N(C₁-C₄-Alkyl)₂ oder C₁-C₄-Alkylgruppe, die gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder ein -S(O)ᵣ-CH₃, wobei r für 0 - 2 steht, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann, unter derVoraussetzung, dass R² Cyano bedeutet oder wenn R¹ und/oder R² gleich oder verschieden ein C₁-C₄-Akylrest ist, wobei mindestens einer der Reste mindestens einfach substituiert ist,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CON(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CONH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
- R⁴ und R⁵: entweder in ortho-, meta- oder meta,para-Stellung zueinander stehen und gemeinsam die Bedeutung haben -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
- Y: eine -(CH₂)₂-Gruppe steht,
bedeuten, sowie deren Isomere, Diastereomere, Enantiomere und Salze bzw. Cyclodextrinclathrate.

Ebenfalls bevorzugt sind solche Verbindungen der allgemeinen Formel (I),
wobei
- A: einen Phenyl-, Naphthyl- oder C₅-C₁₂-Heteroarylrest, der gegebenenfalls ein- oder zweifach mit R⁴ und/oder R⁵ substituiert sein kann,
- R¹: ein C₁-C₄-Alkylrest,
- R²: ein Wasserstoff, Fluor, Chlor, Brom oder C₁-C₄-Alkyl,
- R³: ein Wasserstoff, Methyl, Ethyl, Trifluormethyl, Cyano, Chlor oder Brom,
- R⁴: ein Wasserstoff, Halogen, Amino, eine -S(O)ₚ-C₁-C₄-Alkylgruppe, wobei p 0 - 2 bedeuten,
ein C₁-C₄-Acyl-, NH-CO-NH₂, -O-CO-NH(C₁-C₄-Alkyl), -O-CO-N(C₁-C₄-Alkyl)₂ oder NH-CO-C₁-C₄-Alkylrest,
ein C₁-C₄-Alkyl, welches gegebenenfalls ein-, zwei- oder dreifach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein-, zwei- oder dreifach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls ein- oder zweifach mit Hydroxy, Cyano, COOH oder CO-NH₂ substituiert sein kann,
ein CH₂O-C₆-C₁₂-Aryl, welches gegebenenfalls ein- oder zweifach mit Hydroxy, Cyano, COOH oder CO-NH₂ substituiert sein kann,
ein O-C₅-C₁₆-Heteroaryl, welches gegebenenfalls ein- oder zweifach mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein-, zwei- oder dreifach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), S02N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein-, zwei- oder dreifach, gleich oder verschieden mit C₁-C₄-Alkyl substituiert sein kann,
unter der Voraussetzung, dass R² Cyano ist oder wenn R¹ und/oder R² gleich oder verschieden einen C₁-C₄-Akylrest bedeuten, wobei mindestens einer der Reste mindestens einfach substituiert ist oder
unter der Voraussetzung, dass R⁵ ein -S(O)ₚ-C₁-C₄-Alkyl bedeutet, wobei p für 0 - 2 steht, ein C₁-C₆-Acyl-, -O-CO-NH(C₁-C₄-Alkyl), -O-CO-N(C₁-C₄-Alkyl)₂, C₆-C₁₂-Aryloxy, C₅-C₁₆-Heteroaryloxy, Hydroxy, Cyano oder N-(C₁-C₄-Alkyl)₂ ist,
ein monocyclisches C₅-C₇-Heteroaryl, welches mindestens ein-, zwei- oder dreifach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein-, zwei- oder dreifach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CONH(C₁-C₄-Alkyl) oder CO-N(C₁-C₄-Alkyl)₂ substituiert sein kann oder
ein C₃-C₆-Cycloalkyl, welches gegebenenfalls ein-, zwei- oder dreifach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
- R⁵: ein Wasserstoff, Halogen, Amino, -S(O)ₚ-C₁-C₄-Alkyl, wobei p 0 - 2 bedeutet,
eine C₁-C₄-Acyl-, NH-CO-NH₂-, NH-CO-C₁-C₄-Alkyl-, -O-CO-NH(C₁-C₄-Alkyl)-, -O-CO-N(C₁-C₄-Alkyl)₂ oder C₁-C₄-Alkylgruppe, die gegebenenfalls ein-, zwei- oder dreifach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein-, zwei- oder dreifach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(_{C1}-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein-, zwei- oder dreifach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄- Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r für 0 - 2 steht, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein-, zwei- oder dreifach, gleich oder verschieden mit C₁-C₄-Alkyl substituiert sein kann,
unter der Voraussetzung, dass R² Cyano bedeutet oder wenn R¹ und/oder R² gleich oder verschieden ein C₁-C₄-Akylrest ist, wobei mindestens einer der Reste mindestens einfach substituiert ist,
ein monocyclisches C₅-C₇-Heteroaryl, welches mindestens ein-, zwei- oder dreifach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein-, zwei- oder dreifach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CONH(C₁-C₄-Alkyl) oder CO-N(C₁-C₄-Alkyl)₂ substituiert sein kann oder
ein C₃-C₆-Cycloalkyl, welches gegebenenfalls ein-, zwei- oder dreifach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
- R⁴ und R⁵: entweder in ortho-, meta- oder meta,para-Stellung zueinander stehen und gemeinsam -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂- bedeuten, wobei m für 1 - 3 steht,
- Y: eine -(CH₂)₂-Gruppe,
bedeuten, sowie deren Isomere, Diastereomere, Enantiomere und Salze bzw. Cyclodextrinclathrate.

Ebenfalls bevorzugt sind solche Verbindungen der allgemeinen Formel (I),
wobei
- A: einen Phenyl-, Naphthyl- oder C₅-C₁₂-Heteroarylrest, der gegebenenfalls ein- oder zweifach mit R⁴ und/oder R⁵ substituiert sein kann,
- R¹: ein C₁-C₄-Alkylrest,
- R²: ein Wasserstoff, Fluor, Chlor, Brom, Methyl, Ethyl oder Trifluormethyl,
- R³: ein Wasserstoff, Methyl, Ethyl, Trifluormethyl, Cyano, Chlor oder Brom,
- R⁴: ein Wasserstoff, Halogen, Amino,
ein C₁-C₄-Acyl- oder NH-CO-C₁-C₄-Alkylrest,
ein C₁-C₄-Alkyl, welches gegebenenfalls ein- oder zwei-, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls einfach mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-Phenyl, welches gegebenenfalls ein- oder zweifach mit Hydroxy, Cyano, COOH oder CO-NH₂ substituiert sein kann,
ein CH₂O-Phenyl, welches gegebenenfalls ein- oder zweifach mit Hydroxy, Cyano, COOH oder CO-NH₂ substituiert sein kann,
ein O-C₅-C₇-Heteroaryl, welches gegebenenfalls ein- oder zweifach mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein Phenyl, welches gegebenenfalls ein- oder zweifach, gleich oder verschieden mit Halogen, mit C₁-C₄-Alkyl, C₁-C₄-Acyl, C₁-C₄-Alkoxy, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CON(C₁-C₄-Alkyl)₂, NH-CO(C₁-C₄-Alkyl), CH₂-NH-CO(C₁-C₄-Alkyl) substituiert sein kann oder zwei benachbarte Positionen durch -OCH₂-O- oder -O-C(CH₃)₂-O- substituiert sein können,
- R⁵: ein Wasserstoff, Halogen, Amino, ein C₁-C₄-Acyl- oder NH-CO-C₁-C₄-Alkylrest,
ein C₁-C₄-Alkyl, welches gegebenenfalls ein- oder zwei-, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls einfach mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-Phenyl, welches gegebenenfalls ein- oder zweifach mit Hydroxy, Cyano, COOH oder CO-NH₂ substituiert sein kann,
ein CH₂O-Phenyl, welches gegebenenfalls ein- oder zweifach mit Hydroxy, Cyano, COOH oder CO-NH₂ substituiert sein kann,
ein O-C₅-C₇-Heteroaryl, welches gegebenenfalls ein- oder zweifach mit Hydroxy, Cyano, COOH oder CO-NH₂ substituiert sein kann,
ein Hydroxy, Cyano, O-CO-(C₁-C₄-Alkyl), CO-NH(C₅-C₁₂-
Heteroaryl), NH-(C₁-C₄-Alkyl), N-(C₁-C₄-Alkyl)₂,
ein Phenyl, welches gegebenenfalls ein- oder zweifach, gleich oder verschieden mit Halogen, mit C₁-C₄-Alkyl, C₁-C₄-Acyl, C₁-C₄-Alkoxy, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, NH-CO(C₁-C₄-Alkyl), CH₂-NH-CO(C₁-C₄-Alkyl) substituiert sein kann oder zwei benachbarte Positionen durch -O-CH₂-O- oder -O-C(CH₃)₂-O- substituiert sein können,
- R⁴ und R⁵: entweder in ortho-, meta- oder meta,para-Stellung zueinander stehen und gemeinsam die Bedeutung haben -CH₂-CO-NH-, -NH-CO-CH₂-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
- Y: eine -(CH₂)₂-Gruppe steht,
bedeuten, sowie deren Isomere, Diastereomere, Enantiomere und Salze bzw. Cyclodextrinclathrate.

Die folgenden Verbindungen entsprechend der vorliegenden Erfindung sind ganz besonders bevorzugt:
1. 3,4-Dimethoxy-N-[2-(2-methyl-1H-indol-3-yl)-ethyl]-benzamid
2. 5-Fluor-1H-indol-2-carbonsäure [2-(7-chlor-2-methyl-1H-indol-3-yl)-ethyl]-amid
3. 5-Fluor-1H-indol-2-carbonsäure [2-(7-chlor-4-fluor-2-methyl-1H-indol-3-yl)-ethyl]-amid
4. Biphenyl-3,4'-dicarbonsäure 4'-{[2-(7-chlor-4-fluor-2-methyl-1H-indol-3-yl)-ethyl]-amid} 3-methylamid
5. N-[2-(7-Chlor-2-methyl-1H-indol-3-yl)-ethyl]-6-(3-methylcarbamoyl-phenyl)-nicotinamid
6. 3'-Hydroxy-biphenyl-4-carbonsäure [2-(7-chlor-2-methyl-1H-indol-3-yl)-ethyl]-amid
7. N-[2-(7-Chlor-4-fluor-2-methyl-1H-indol-3-yl)-ethyl]-3,4-dimethoxy-benzamid
8. Biphenyl-3,4'-dicarbonsäure 3-methylamid 4'-{[2-(2-methyl-1H-indol-3-yl)-ethyl]-amid}
9. 5-Fluor-1H-indol-2-carbonsäure [2-(2-methyl-1H-indol-3-yl)-ethyl]-amid
10. 5-Chlor-1H-indol-2-carbonsäure [2-(2-methyl-1H-indol-3-yl)-ethyl]-amid
11. N-[2-(2,4-Dimethyl-1H-indol-3-yl)-ethyl]-3,4-dimethoxy-benzamid
12. N-[2-(7-Chlor-2,4-dimethyl-1H-indol-3-yl)-ethyl]-3,4-dimethoxy-benzamid
13. N-[2-(7-Brom-2,4-dimethyl-1H-indol-3-yl)-ethyl]-3,4-dimethoxy-benzamid
14. 3H-Benzotriazol-5-carbonsäure [2-(7-brom-2,4-dimethyl-1H-indol-3-yl)-ethyl]-amid
15. 1 H-Indol-2-carbonsäure [2-(7-brom-2,4-dimethyl-1H-indol-3-yl)-ethyl]-amid
16. Chinoxalin-6-carbonsäure [2-(7-brom-2,4-dimethyl-1H-indol-3-yl)-ethyl]-amid
17. 3H-Benzotriazol-5-carbonsäure [2-(7-chlor-2,4-dimethyl-1H-indol-3-yl)-ethyl]-amid
18. Chinoxalin-6-carbonsäure [2-(7-chlor-2,4-dimethyl-1H-indol-3-yl)-ethyl]-amid
19. 1H-Indol-2-carbonsäure [2-(7-chlor-2,4-dimethyl-1H-indol-3-yl)-ethyl]-amid
20. N-[2-(7-Brom-2-methyl-1H-indol-3-yl)-ethyl]-3,4-dimethoxy-benzamid
21. Biphenyl-3,4'-dicarbonsäure 4'-{[2-(7-brom-2-methyl-1H-indol-3-yl)-ethyl]-amid} 3-methylamid
22. 5-Fluor-1H-indol-2-carbonsäure [2-(7-brom-2-methyl-1H-indol-3-yl)-ethyl]-amid
23. N-[2-(7-Chlor-2-trifluormethyl-1H-indol-3-yl)-ethyl]-3,4-dimethoxy-benzamid
24. 5-Fluor-1H-indol-2-carbonsäure [2-(7-chlor-2-trifluormethyl-1H-indol-3-yl)-ethyl]-amid
25. 5-Chlor-1H-indol-2-carbonsäure [2-(7-chlor-2-trifluormethyl-1H-indol-3-yl)-ethyl]-amid
26. Biphenyl-3,4'-dicarbonsäure 4'-{[2-(7-chlor-2-trifluormethyl-1H-indol-3-yl)-ethyl]-amid} 3-methylamid
27. 5-Trifluormethoxy-1H-indol-2-carbonsäure [2-(7-chlor-2-trifluormethyl-1H-indol-3-yl)-ethyl]-amid
28. Benzofuran-2-carbonsäure [2-(7-chlor-2-trifluormethyl-1H-indol-3-yl)-ethyl]-amid
29. Benzo[b]thiophen-2-carbonsäure [2-(7-chlor-2-trifluormethyl-1H-indol-3-yl)-ethyl]-amid
30. 5-Chlor-1H-indol-2-carbonsäure [2-(7-brom-2-methyl-1H-indol-3-yl)-ethyl]-amid
31. 5-Chlor-1H-indol-2-carbonsäure [2-(7-chlor-2-methyl-1H-indol-3-yl)-ethyl]-amid
32. N-[2-(2-tert-Butyl-1H-indol-3-yl)-ethyl]-3,4-dimethoxy-benzamid
33. 5-Trifluormethoxy-1H-indol-2-carbonsäure [2-(2-tert-butyl-1H-indol-3-yl)-ethyl]-amid
34. 5-Chlor-1H-indol-2-carbonsäure [2-(2-tert-butyl-1H-indol-3-yl)-ethyl]-amid
35. 5-Trifluormethyl-1H-indol-2-carbonsäure [2-(2-tert-butyl-1H-indol-3-yl)-ethyl]-amid
36. 5-Brom-1H-indol-2-carbonsäure [2-(2-tert-butyl-1H-indol-3-yl)-ethyl]-amid
37. 5-Fluor-1H-indol-2-carbonsäure [2-(2-tert-butyl-1H-indol-3-yl)-ethyl]-amid
38. 2-Brom-N-[2-(2,7-dimethyl-1H-indol-3-yl)-ethyl]-4,5-dimethoxy-benzamid
39. 1H-Indole-6-carbonsäure [2-(2,7-dimethyl-1H-indol-3-yl)-ethyl]-amid
40. Chinolin-5-carbonsäure [2-(2,7-dimethyl-1H-indol-3-yl)-ethyl]-amid
41. 5-Phenyl-1H-pyrazol-4-carbonsäure [2-(2,7-dimethyl-1H-indol-3-yl)-ethyl]-amid
42. Pyridazin-4-carbonsäure [2-(2,7-dimethyl-1H-indol-3-yl)-ethyl]-amid
43. 5-Phenyl-2H-pyrazol-3-carbonsäure [2-(2,7-dimethyl-1H-indol-3-yl)-ethyl]-amid
44. Pyrimidin-5-carbonsäure [2-(2,7-dimethyl-1H-indol-3-yl)-ethyl]-amid
45. Pyrazolo[1,5-a]pyridin-2-carbonsäure [2-(2,7-dimethyl-1H-indol-3-yl)-ethyl]-amid
46. Benzo[b]thiophen-5-carbonsäure [2-(2,7-dimethyl-1H-indol-3-yl)-ethyl]-amid
47. Chinoxalin-2-carbonsäure [2-(2,7-dimethyl-1H-indol-3-yl)-ethyl]-amid
48. N-[2-(2,7-Dimethyl-1 H-indol-3-yl)-ethyl]-3-fluor-2-methoxy-benzamid
49. 3-Chlor-N-[2-(2,7-dimethyl-1H-indol-3-yl)-ethyl]-2-methyl-benzamid
50. 3-Chlor-N-[2-(2,7-dimethyl-1H-indol-3-yl)-ethyl]-2-fluor-benzamid
51. N-[2-(2,7-Dimethyl-1H-indol-3-yl)-ethyl]-4-phenoxy-benzamid
52. Thiazol-4-carbonsäure [2-(2,7-dimethyl-1H-indol-3-yl)-ethyl]-amid
53. 2-Chlor-N-[2-(2,7-dimethyl-1H-indol-3-yl)-ethyl]-3-methyl-benzamid
54. Chinolin-4-carbonsäure [2-(2,7-dimethyl-1H-indol-3-yl)-ethyl]-amid
55. N-[2-(2,7-Dimethyl-1H-indol-3-yl)-ethyl]-3-hydroxy-benzamid
56. 4-Hydroxy-2-phenyl-2H-pyrazol-3-carbonsäure [2-(2,7-dimethyl-1H-indol-3-yl)-ethyl]-amid
57. Benzo[b]thiophen-3-carbonsäure [2-(2,7-dimethyl-1H-indol-3-yl)-ethyl]-amid
58. 1 H-Indol-2-carbonsäure [2-(2,7-dimethyl-1H-indol-3-yl)-ethyl]-amid
59. Chinolin-2-carbonsäure [2-(2,7-dimethyl-1H-indol-3-yl)-ethyl]-amid
60. Furan-2-carbonsäure [2-(2,7-dimethyl-1H-indol-3-yl)-ethyl]-amid
61. 2-Chlor-N-[2-(2,7-dimethyl-1H-indol-3-yl)-ethyl]-nicotinamid
62. Chinolin-3-carbonsäure [2-(2,7-dimethyl-1H-indol-3-yl)-ethyl]-amid
63. 5-Brom-N-[2-(2,7-dimethyl-1H-indol-3-yl)-ethyl]-nicotinamid
64. 1 H-Indol-3-carbonsäure [2-(2,7-dimethyl-1H-indol-3-yl)-ethyl]-amid
65. 4-Benzyloxy-N-[2-(2,7-dimethyl-1H-indol-3-yl)-ethyl]-benzamid
66. 3-Brom-thiophen-2-carbonsäure [2-(2,7-dimethyl-1H-indol-3-yl)-ethyl]-amid
67. 2-Methyl-furan-3-carbonsäure [2-(2,7-dimethyl-1H-indol-3-yl)-ethyl]-amid
68. 4-Oxo-4,5,6,7-tetrahydro-benzo[b]thiophen-2-carbonsäure [2-(2,7-dimethyl-1H-indol-3-yl)-ethyl]-amid
69. 2-Benzo[1,2,5]thiadiazol-4-yl-N-[2-(2,7-dimethyl-1H-indol-3-yl)-ethyl]-acetamid
70. 1H-Imidazol-4-carbonsäure [2-(2,7-dimethyl-1H-indol-3-yl)-ethyl]-amid
71. 4'-Brom-biphenyl-2-carbonsäure [2-(2,7-dimethyl-1H-indol-3-yl)-ethyl]-amid
72. N-[2-(2,7-Dimethyl-1H-indol-3-yl)-ethyl]-2-fluor-6-iod-benzamid
73. 3'-[2-(2,7-Dimethyl-1H-indol-3-yl)-ethylcarbamoyl]-biphenyl-2-carbonsäure methylester
74. 2,3-Dihydro-benzofuran-7-carbonsäure [2-(2,7-dimethyl-1H-indol-3-yl)-ethyl]-amid
75. N-[2-(2,7-Dimethyl-1H-indol-3-yl)-ethyl]-3-fluor-2-methyl-benzamid
76. N-[2-(2,7-Dimethyl-1H-indol-3-yl)-ethyl]-2,5-dimethyl-benzamid
77. 5-Acetyl-thiophen-2-carbonsäure [2-(2,7-dimethyl-1H-indol-3-yl)-ethyl]-amid
78. N-[2-(2,7-Dimethyl-1H-indol-3-yl)-ethyl]-2-fluor-3-methoxy-benzamid
79. Chinolin-8-carbonsäure [2-(2,7-dimethyl-1H-indol-3-yl)-ethyl]-amid
80. N-[2-(2,7-Dimethyl-1H-indol-3-yl)-ethyl]-4-methylsulfanyl-benzamid
81. 2-Phenyl-2H-pyrazol-3-carbonsäure [2-(2,7-dimethyl-1H-indol-3-yl)-ethyl]-amid
82. 6-Phenyl-pyrimidin-4-carbonsäure [2-(2,7-dimethyl-1H-indol-3-yl)-ethyl]-amid
83. 1-Methyl-1H-indol-3-carbonsäure [2-(2,7-dimethyl-1H-indol-3-yl)-ethyl]-amid
84. N-[2-(2,7-Dimethyl-1H-indol-3-yl)-ethyl]-2-phenoxymethyl-benzamid
85. 2,5-Dimethyl-2H-pyrazol-3-carbonsäure [2-(2,7-dimethyl-1H-indol-3-yl)-ethyl]-amid
86. 2,3-Dihydro-benzofuran-5-carbonsäure [2-(2,7-dimethyl-1H-indol-3-yl)-ethyl]-amid
87. 5-Methoxy-1H-indol-2-carbonsäure [2-(2,7-dimethyl-1H-indol-3-yl)-ethyl]-amid
88. 5-Methoxy-1H-indol-2-carbonsäure [2-(2-methyl-7-trifluormethyl-1H-indol-3-yl)-ethyl]-amid
89. 1H-Indol-2-carbonsäure [2-(2-methyl-7-trifluormethyl-1H-indol-3-yl)-ethyl]-amid
90. 5-Fluor-1H-indol-2-carbonsäure [2-(2-methyl-7-trifluormethyl-1H-indol-3-yl)-ethyl]-amid
91. 5-Methyl-1H-indol-2-carbonsäure [2-(2-methyl-7-trifluormethyl-1H-indol-3-yl)-ethyl]-amid
92. 6-Methoxy-1H-indol-2-carbonsäure [2-(2-methyl-7-trifluormethyl-1H-indol-3-yl)-ethyl]-amid
93. 4-Methyl-1H-indol-2-carbonsäure [2-(2-methyl-7-trifluormethyl-1H-indol-3-yl)-ethyl]-amid
94. 4-Methoxy-1H-indol-2-carbonsäure [2-(2-methyl-7-trifluormethyl-1H-indol-3-yl)-ethyl]-amid
95. 4-Fluor-1H-indol-2-carbonsäure [2-(2-methyl-7-trifluormethyl-1H-indol-3-yl)-ethyl]-amid
96. 6-Fluor-1H-indol-2-carbonsäure [2-(2-methyl-7-trifluormethyl-1H-indol-3-yl)-ethyl]-amid
97. 6-Methyl-1H-indol-2-carbonsäure [2-(2-methyl-7-trifluormethyl-1H-indol-3-yl)-ethyl]-amid
98. 7-Methyl-1H-indol-2-carbonsäure [2-(2-methyl-7-trifluormethyl-1H-indol-3-yl)-ethyl]-amid
99. N-[2-(7-Cyano-2,4-dimethyl-1H-indol-3-yl)-ethyl]-3,4-dimethoxy-benzamid
100. N-[2-(7-Chlor-2-methyl-1H-indol-3-yl)-ethyl]-3,4-dimethoxy-benzamid
101. Biphenyl-4,4'-dicarbonsäure 4'-{[2-(7-chlor-2-methyl-1H-indol-3-yl)-ethyl]-amid} 4-methylamid
102. Biphenyl-3,4'-dicarbonsäure 4'-{[2-(7-chlor-2-methyl-1H-indol-3-yl)-ethyl]-amid} 3-methylamid
103. N-[2-(7-Brom-2,4-dimethyl-1H-indol-3-yl)-ethyl]-3,4-dimethoxy-benzamid
104. N-[2-(4,7-Dichlor-2-methyl-1H-indol-3-yl)-ethyl]-3,4-dimethoxy-benzamid
105. Biphenyl-3,4'-dicarbonsäure 4'-{[2-(4,7-dichlor-2-methyl-1H-indol-3-yl)-ethyl]-amid} 3-methylamid

Gegenstand der vorliegenden Erfindung ist die Verwendung der erfindungsgemäßen Verbindungen für die Herstellung von Arzneimitteln, die mindestens eine der Verbindungen gemäß Formel I enthalten.

Ebenfalls Gegenstand der vorliegenden Erfindung sind Arzneimittel, die die erfindungsgemäßen Verbindungen enthalten mit geeigneten Formulierungs- und Trägerstoffen.

Im Vergleich zu bekannten Prostaglandin E₂-Liganden zeichnen sich die neuen EP₂-Agonisten und Antagonisten durch größere Selektivität und Stabilität aus.

Gegenstand der vorliegenden Erfindung sind Arzneimittel zur Behandlung und Prophylaxe von Erkrankungen, zu denen Fertilitätsstörungen, infektiöse Erkrankungen, Krebs, virale Infektionen, Herz-Kreislauf-Erkrankungen, erhöhter Augeninnendruck, Glaukoma, Erkrankungen des Knochenapparates, angiogenetische Erkrankungen, Störungen der Uteruskontraktion, Schmerz, neuroinflammatorische Erkrankungen, immunomodulatorische Infektionen und nephrologische Erkrankungen zählen.

Unter Fertilitätsstörungen sind die Erkrankungen zu verstehen, die dazu führen, dass keine Ovulation erfolgt, dass es nicht zur Nidation einer befruchteten Eizelle kommt und keine Dezidualisierung stattfindet, unter infektiösen Erkrankungen sind durch unizelluläre Parasiten hervorgerufene Erkrankungen, unter Krebs solide Tumoren und Leukämie, unter viralen Infektionen z. B. Cytomegalus-Infektionen, Hepatitis, Hepatitis B und C und HIV Erkrankungen, unter immunmodulatorischen Infektionen z.B. Vogelgrippe, unter Herz-Kreislauf-Erkrankungen ischämische Reperfusionserkrankung, Stenosen, Arteriosklerosen und Restenosen, unter angiogenetischen Erkrankungen z.B. Endometriose und Fibrose, unter erhöhtem Augeninnendruck Glaukom, unter Störungen der Uteruskontraktion z.B. Menstruationsbeschwerden, unter Erkrankungen des Knochenapparates Osteoporose, unter neuroinflammatorischen Erkrankungen Multiple Sklerose, Morbus Alzheimer, Morbus Parkinson, Morbus Crohn, Colitis Ulcerosa, Schmerz und unter nephrologischen Erkrankungen polyzystische Nierenerkrankung, Glomerulonephritis, zu verstehen.

Ebenfalls Gegenstand der vorliegenden Erfindung sind Arzneimittel zur Behandlung und Prophylaxe der oben aufgeführten Erkrankungen, die mindestens eine Verbindung gemäß der allgemeinen Formel I enthalten, sowie Arzneimittel mit geeigneten Formulierungs- und Trägerstoffen.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln, in halbfester Form, zum Beispiel als Salben, Cremes, Gele, Suppositiorien, Emulsionen oder in flüssiger Form, zum Beispiel als Lösungen, Suspensionen oder Emulsionen vorliegen.

Gegebenenfalls enthalten sie Hilfsstoffe, die beispielsweise als Füllstoffe, Bindemeittel, Sprengmittel, Gleitmittel, Lösungsmittel, Lösungsvermittler, Geschmackskorrigentien, Farbstoff, Emulgatoren, fungieren sollen. Hilfsstoffarten im Sinne der Erfindung sind beispielsweise Saccharide (Mono-, Di-, Tri-, Oligo-, und/oder Polysaccharide), Fette, Wachse, Öle, Kohlenwasserstoffe, anionische, nichtionische, kationische natürliche, synthetische oder halbsynthestische Tenside. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer. Diese pharmazeutischen Präparate sind ebenfalls Gegenstand der vorliegenden Erfindung.

Zur Inhalation werden zweckmäßigerweise Aerosollösungen hergestellt.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist. Für die orale Anwendung derartiger Verbindungen sind ebenfalls auch Clathrate geeignet, beispielsweise seien genannt die Clathrate mit alpha-, beta-, gamma-Cyclodextrin oder auch beta-hydroxypropyl-Cyclodextrin.

Für die parenterale Verabreichung werden sterile, injizierbare, wässrige oder ölige Lösungen benutzt. Besonders geeignet sind Injektionslösungen oder Suspensionen, insbesondere wässrige Lösungen der aktiven Verbindungen in polyethoxyliertem Rizinusöl.

Für die vaginale Applikation sind z. B. Zäpfchen, Tampons oder ein intrauterines System geeignet und üblich.

Für die intraartikuläre Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Ärosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01 % - 20 % betragen, um eine ausreichende pharmakologische Wirkung zu erzielen.

Die Dosierung der Wirkstoffe kann je nach Verabfolgungsweg, Alter und Gewicht des Patienten, Art und Schwere der zu behandelnden Erkrankung und ähnlichen Faktoren variieren. Die Behandlung kann durch Einzeldosierungen oder durch eine Vielzahl von Dosierungen über einen längeren Zeitraum erfolgen. Die tägliche Dosis beträgt 0,5 - 1000 mg, vorzugsweise 50 - 200 mg, wobei die Dosis als einmal zu verabreichende Einzeldosis oder unterteilt in 2 oder mehrere Tagesdosen gegeben werden kann.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Die oben beschrieben Formulierungen und Darreichungsformen sind ebenfalls Gegenstand der vorliegenden Erfindung.

Die Administration der erfindungsgemäßen Verbindungen kann durch jede konventionelle Methode erfolgen, einschließlich oraler und parenteraler, z.B. durch subcutane oder intramuskuläre Injektionen. Die enteralen, parenteralen, vaginalen und oralen Applikationen sind ebenfalls Gegenstand der vorliegenden Erfindung.

Die erfindungsgemäßen Verbindungen der allgemeinen Formel I binden an den EP₂ Rezeptor und haben agonistische oder antagonistische Wirkung. Es kann durch einen Agonismustest (siehe Beispiel 1.2.1. der biologischen Beispiele) oder durch einen Antagonismustest (siehe Beispiel 1.2.2. der biologischen Beispiele) bestimmt werden, ob eine agonistische oder antagonistische Wirkung vorl iegt.

Unter Antagonisten sind solche Moleküle zu verstehen, die an ihre entsprechenden Rezeptoren binden und welche die Initiierung des/der mit dem Rezeptor gekoppelten Signaltransduktionswege/s durch den oder die natürlich vorkommenden Liganden inhibieren. Üblicherweise kompetitieren die Antagonisten mit dem natürlich vorkommenden Liganden des Rezeptors um die Bindung an den Rezeptor. Aber auch andere Modifikationen des Rezeptors durch Moleküle, die verhindern, dass die mit dem Rezeptor gekoppelten Signaltransduktionswege durch den oder die natürlich vorkommenden Liganden aktiviert werden, sind möglich (z.B. nicht-kompetitive, sterische Modifikationen des Rezeptors).

Rezeptorantagonisten binden typischerweise selektiv an ihren bestimmten Rezeptor und nicht an andere Rezeptoren. Sie weisen normalerweise eine höhere Bindungsaffinität als der natürliche Ligand auf. Obwohl Antagonisten, die eine höhere Affinität zum Rezeptor haben als der natürliche Ligand, bevorzugt sind, können ebenfalls Antagonisten mit einer geringeren Affinität eingesetzt werden.
Bevorzugterweise binden die Antagonisten reversibel an ihre korrespondierenden Rezeptoren.

Der EP₂ Rezeptor Antagonist hat eine bevorzugte Affinität für den EP₂ Rezeptor gegenüber jedem anderen EP-Rezeptor. Der Antagonismus wird in Gegenwart des natürlichen Agonisten gemessen (PGE₂).

Unter Agonisten sind solche Moleküle zu verstehen, die an ihre entsprechenden Rezeptoren binden und üblicherweise mit dem natürlich vorkommenden Liganden des Rezeptors um die Bindung an den Rezeptor kompetitieren und welche die Initiierung des mit dem Rezeptor gekoppelten Signaltransduktionsweges stimulieren. Agonisten können auch die Bindung des natürlichen Liganden unterstützen.

Rezeptoragonisten binden typischerweise selektiv an ihren bestimmten Rezeptor und nicht an andere Rezeptoren. Sie haben normalerweise eine höhere Bindungsaffinität als der natürliche Ligand. Obwohl Agonisten, die eine höhere Affinität zum Rezeptor haben als der natürliche Ligand, bevorzugt sind, können ebenfalls Agonisten mit einer geringeren Affinität eingesetzt werden. Bevorzugterweise binden die Agonisten reversibel an ihre korrespondierenden Rezeptoren.

Der EP₂ Rezeptor Agonist hat eine bevorzugte Affinität für den EP₂ Rezeptor gegenüber jedem anderen EP-Rezeptor.

Agonisten werden über die Initiierung der entspechenden Rezeptor vermittelten Signaltransduktion und/oder physiologischen Wirkung getestet.
Als Liganden werden die Verbindungen oder niedermolekulare Substanzen bezeichnet, die an einen Rezeptor binden. Ihre Bindung ist üblicherweise reversibel. Durch die Bindung eines Liganden an den entsprechenden Rezeptor wird der mit dem Rezeptor gekoppelte Signaltransduktionsweg aktiviert oder inaktiviert. Auf diese Art und Weise vermittelt der Ligand seine intrazelluläre Wirkung. Unter Liganden sind Agonisten und Antagonisten eines Rezeptors zu verstehen.

Die Substanz gemäß Beispiel 7 zeigt im zellulären Agonismustest keine Inhibition, im Antagonismustest jedoch eine gute Wirksamkeit (IC₅₀ = 0.047 x 10 E-6 M).
Ebenfalls Gegenstand der vorliegenden Erfindung ist die Verwendung der erfindungsgemäßen Substanzen als EP₂-Rezeptor Antagonisten für die Behandlung von Erkrankungen, die verursacht werden durch Störungen in der Signaltransduktionskette, an der der EP₂-Rezeptor beteiligt ist, wie beispielsweise Schmerz und Fertilitätsstörungen, und die ebenfalls geeignet sind für die Fertilitätskontrolle.

Im prä-ovulatorischen antralen Follikel ist die Eizelle von Kumulus Zellen umgeben, die einen dichten Zellkranz um die Eizelle bilden. Nach dem Peak des Lutenisierenden Hormons (LH-Peak) wird eine Reihe von Prozessen aktiviert, die eine starke morphologische Veränderung dieses Zellkranzes aus Kumulus Zellen zur Folge hat. Hierbei bilden die Kumulus Zellen eine extrazelluläre Matrix, die zur sogenannten Kumulus Expansion führt (Vanderhyden et al. Dev Biol. 1990 Aug;140(2):307-317). Diese Kumulus Expansion ist ein wichtiger Bestandteil des ovulatorischen Prozesses und der nachfolgenden Möglichkeit zur Fertilisation.

Bei der Kumulus Expansion sind Prostaglandine und hier das Prostaglandin E₂, dessen Synthese durch den LH-Peak induziert wird, von entscheidender Bedeutung. Prostanoid EP₂ Knock-out Mäuse (Hizaki et al.. Proc Natl Acad Sci U S A. 1999 Aug 31;96(18):10501-6.) zeigen eine deutlich verminderte Kumulus Expansion und starke Subfertilität, was die Bedeutung des Prostanoid EP₂-Rezeptors für diesen Prozess demonstriert.

Die erfindungsgemäßen Substanzen haben inhibitorische Wirkungen in Kumulus Expansionstests.

Gegenstand der vorliegenden Erfindung ist die Verwendung der erfindungsgemäßen Substanzen zur Fertilitätskontrolle.

Während der EP₂-Rezeptor Antagonist AH 6809 die Expansion des Kumulus erst bei einer Konzentration von 100 - 200 µM um nur ca. 30 % unterdrückt, kann in Anwesenheit von der Substanz gemäß Beispiel 8 eine ca. 60 %ige Unterdrückung der Kumulus Expansion bereits bei einer 20 - 40-fach geringeren Konzentration (5 µM) erreicht werden. Bei diesen Versuchen kompetitieren die Testsubstanzen mit dem natürlichen EP₂-Rezeptor Agonisten PGE₂.

Gegenstand der vorliegenden Erfindung ist die Verwendung der erfindungsgemäßen Substanzen für die Hemmung der Kumulus Expansion und dadurch der Ovulation und Fertilisation zur Kontrazeption.

Prostaglandine spielen eine wichtige Rolle bei der Angiogenese (Sales, Jabbour, 2003, Reproduction 126, 559 - 567; Kuwano et al., 2004, FASEB J. 18, 300-310; Kamiyama et al., 2006, Oncogene 25, 7019-7028; Chang et al. 2005, Prostaglandins & other Lipid Mediators 76, 48-58).

Endometriose ist eine chronische Erkrankung, die verursacht wird durch Störungen der Blutgefäße. Circa 10 % der Frauen leiden regelmäßig an starken Blutungen während der Menstruation, verursacht durch Änderungen der Blutgefäße des Endometriums. Zusätzlich wurden strukturelle Unterschiede in den Blutgefäßen beobachtet, wie zum Beispiel die unvollständige Ausbildung der glatten Muskelzellschicht (Abberton et al., 1999, Hum. Reprod. 14, 1072-1079). Da der Blutverlust während der Menstruation zum Teil durch die Konstriktion der Blutgefäße geregelt wird, ist es naheliegend, dass die Defekte der glatten Muskulatur wesentlich zur Blutung beitragen.

Gegenstand der vorliegenden Erfindung ist die Verwendung der Substanzen der allgemeinen Formel I für die Behandlung von Endometriose.

Prostaglandine spielen eine wichtige Rolle bei der Uteruskontraktion, zu starke Kontraktionen sind verantwortlich für Menstruationsbeschwerden (Sales, Jabbour, 2003, Reproduction 126, 559 - 567, Jabbour, Molecular and Cellular Endocrinology 252 (2006) 191-200). Neuere Untersuchungen weisen darauf hin dass bei starken Blutungen während der Menstruation der EP₂-Rezeptor beteiligt ist (Smith et al. Human Reproduction 2007; 22(5): 1450-1456)

Gegenstand der vorliegenden Erfindung ist die Verwendung der Substanzen der allgemeinen Formel I für die Behandlung von Menstruationsbeschwerden.

Zunehmende Forschungsergebnisse belegen auch die Bedeutung der EP-Rezeptoren, und insbesondere auch des EP₂-Rezeptoren bei einer Vielzahl von Krebsarten (z. B. Brustkrebs, Kolonkarzinom, Lungenkrebs, Prostatakrebs, Leukämie, Hautkrebs, Ösophaguskrebs), was auf zukünftige Möglichkeiten des Einsatzes von Modulatoren (Antagonisten oder Agonisten) des EP₂-Rezeptors für die Therapie und Vorbeugung (prophylaktisch und/oder adjuvant) von Krebs schließen lässt (Fulton et al. Cancer Res 2006; 66(20): 9794-7; Pan et al. 2008; The Journal of Biological Chemistry 283(17): 11155-11163; Subbaramaiah et al. 2008; The Journal of Biological Chemistry 283(6): 3433-3444; Castellone et al. Science VOL 310 2005, 1504-1510; Chang et al. Cancer Res 2005; 65(11): 4496-9); Hull et al. Mol Cancer Ther 2004;3(8):1031-9; Richards et al. J Clin Endocrinol Metab 88: 2810-2816, 2003; Sinha et al. 2007, Cancer Res; 67(9):4507-13; Wang et al. 2004, Seminars in Oncology, Vol 31, No 1, Suppl 3: pp 64-73 ; Yu et al. 2008; JPET Published on June 26, 2008 as DOI:10.1124/jpet.108.141275).

Gegenstand der vorliegenden Erfindung ist die Verwendung der Substanzen der allgemeinen Formel I für die Behandlung und Vorbeugung von Krebserkrankungen.

Prostaglandine spielen auch eine wichtige Rolle bei den Prozessen, die dem Knochenschwund entgegen wirken. Gegenstand der vorliegenden Erfindung ist daher die Verwendung der erfindungsgemäßen Substanzen für die Behandlung von Knochenschwund.

Reinold et al. (J. Clin. Invest. 115, 673-679 (2005)) beschreibt PGE₂ Rezeptoren vom EP₂-Subtyp als die Schlüssel-Signalelemente bei der inflammatorischen Hyperalgesie. Mäuse, die diesen Rezeptor nicht mehr tragen (EP₂^{-/-}), empfinden keinen spinalen inflammatorischen Schmerz. Es gibt Hinweise, dass eine inflammatorische, gesteigerte Schmerzempfindlichkeit behandelt werden kann, indem gezielt EP₂-Rezeptoren moduliert werden.

Gegenstand der vorliegenden Erfindung ist die Verwendung der erfindungsgemäßen Substanzen für die Behandlung von inflammatorischer Hyperalgesie.

Prostaglandine und speziell der EP₂-Rezeptor stehen auch im Zusammenhang mit der β-Amyloid Genese bei Alzheimerschen Erkrankung (Hoshino et al. 2007 J Biol Chem.; 282(45):32676-3288).

Gegenstand der vorliegenden Erfindung ist die Verwendung der erfindungsgemäßen Substanzen für die Vorbeugung und Behandlung der Alzheimerschen Erkrankung.

Über den EP₂-Rezeptor vermittelte Effekte von PGE₂ stehen ebenfalls in Zusammenhang mit der Parkinsonschen Erkrankung (Jin et al. 2007; Journal of Neuroinflammation 1186/1742-2094-4-2)

Gegenstand der vorliegenden Erfindung ist die Verwendung der erfindungsgemäßen Substanzen für die Vorbeugung und Behandlung von Parkinsonschen Erkrankung.

Der EP₂-Rezeptor spielt wegen seiner immunmodulatorische Effekte eine Rolle bei entzündlichen Darmerkrankungen (Morbus Crohn, Colitis Ulcerosa) (Sheibanie et al. 2007; The Journal of Immunology, 178: 8138-8147.)

Gegenstand der vorliegenden Erfindung ist die Verwendung der erfindungsgemäßen Substanzen für die Vorbeugung und Behandlung von entzündlichen Darmerkrankungen wie beispielsweise Morbus Crohn, Colitis Ulcerosa.

Neuere Forschungen zeigen, dass der EP₂-Rezeptor an der Entstehung polyzystischer Nieren beteiligt ist. EP₂-Rezeptor Antagonisten können ein Ansatz zur Prävention und Therapie dieser Erkrankung sein (Elberg et al. 2007, Am J Physiol Renal Physiol 293: F1622-F1632.)

Gegenstand der vorliegenden Erfindung ist die Verwendung der erfindungsgemäßen Substanzen für die Vorbeugung und Behandlung von polyzystischen Nierenerkrankungen.

Der EP₂- Rezeptor wird ebenfalls in Zusammenhang mit atherosklerotischen Enstehungsprozessen gebracht (Lie et al. 2006, Circ Res. ;98:642-650).

Gegenstand der vorliegenden Erfindung ist die Verwendung der erfindungsgemäßen Substanzen für die Vorbeugung und Behandlung von Atheriosklerose.

Serezani et al. (Am Respir Cell Mol Biol Vol 37. pp 562-570, 2007) beschreibt, dass über die Aktivierung des EP₂-Rezeptors durch PGE₂ Makrophagen des Atmungstraktes in ihrer Fähigkeit beeinträchtigt werden, Bakterien zu zerstören. Bakterieninfektionen führen zu einer vermehrten Produktion von Prostaglandinen, unter anderem PGE₂, das über diesen Mechanismus die körpereigene Abwehr gegen Bakterien schwächt. Wie in dieser Publikation gezeigt, kann durch eine Inaktivierung des EP₂ Rezeptors (und des EP₄-Rezeptors) diese Fähigkeit der Bakterienbekämpfung wieder hergestellt werden. Weitere relevante Publikationen, die diese Zusammenhänge darlegen sind:
Sadikot et al. Eur. J. Immunol. 2007. 37: 1001-1009 und Aronoff et al. The Journal of Immunology, 2004, 173: 559-565.
Gegenstand der vorliegenden Erfindung ist die Verwendung der erfindungsgemäßen Substanzen für die Behandlung von Infektionskrankheiten der Lunge.
Der natürliche Ligand (Agonist) des EP₂ Rezeptors ist das PGE₂ dessen Synthese über Cyclooxygenases (COX)-Enzyme (COX-1, COX-2) vermittelt wird. Diese Enzyme sind in den erwähnten Krankheitsbildern, Indikationen und deren Entstehung meist über eine verstärkte Expression und Aktivität beteiligt. Deshalb ist bei allen erwähnten Anwendungsmöglichkeiten eine Kombination eines COX-Inhibitors (COX-2 und/oder COX-1) möglich, mit dem Ziel
   a) eine höhere und effektivere pharmakologische Wirksamkeit als mit einer Substanzklasse zu erreichen und
   b) eine niedrigere Dosierung einer der beiden oder beider Substanzklassen zu ermöglichen, was zu einer Reduzierung möglicher Nebenwirkungen und einer beseren Vertäglichkeit führt.

Gegenstand der vorliegenden Erfindung sind daher auch Arzneimittel enthaltend eine Verbindung der allgemeinen Formel (I) in Kombination mit einem COX-Inhibitor zur Behandlung von Erkrankungen. Als COX-Inhibitoren seien beispielsweise die nicht selektiven COX-Inhbitoren wie Aspirin, Naproxen, Indomethacin, Ibuprofen genannt oder die selektiven COX-Inhibitoren Meloxicam, Celecoxib (4-[5-(4-methylphenyl)-3-(trifluoromethyl)-1H-pyrazol-1-yl]benzenesulfonamide) Parecoxib (N-[4-(5-methyl-3-phenyl-4-isoxazolyl)phenyl]sulfonylpropionamide), Rofecoxib (4-(4-mesylphenyl)-3-phenylfuran-2(5H)-one), Valdecoxib (4-[5-methyl-3-phenyl-4-isoxazoyl)benzenesulfonamide), NS-398 (N-methyl-2-cyclohexanoxy-4-nitrobenzenesulfonamide), Lumiracoxib [2-(2'-chloro-6'-fluorophenyl)-amino-5-methylbenzeneacetic acid, Ceracoxib und Etoricoxib.

Außerdem betrifft die Erfindung ein Verfahren zur Herstellung der erfindungsgemäßen Verbindungen der allgemeinen Formel I, das dadurch gekennzeichnet ist, dass eine Verbindung der Formel II, 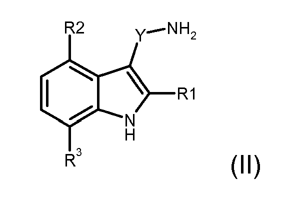 worin R¹, R², R³ und Y die oben angegebenen Bedeutungen haben, mit einem Carbonsäurederivat der allgemeinen Formel III, 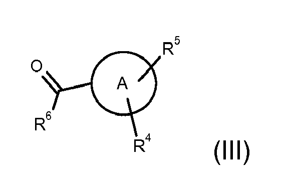 worin A, R⁴ und R⁵ die oben angegebenen Bedeutungen aufweisen und R⁶ eine Hydroxygruppe, ein Chlor- oder Bromatom oder ein C₁-C₆-Akylrest sein kann, bevorzugt ist Wasserstoff, Chlor, der Methyl- oder Ethyl-Rest, nach dem Fachmann bekannten Methoden umgesetzt wurde und gegebenenfalls benötigte Schutzgruppen anschließend abgespalten wurden.

Für den Fall, dass R⁶ eine Hydroxygruppe bedeutet, kann die Umsetzung zunächst durch Aktivierung der Säurefunktion erfolgen, hierbei wird beispielsweise zunächst die Carbonsäure der Formel III in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amins erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen - 30°C und + 60°C, vorzugsweise bei 0°C bis 30°C.

Eine weitere Möglichkeit bestand darin, die Carbonsäure durch Reagenzien wie beispielsweise HOBt (N-Hydroxybenzotriazol) oder HATU (o-(7-Azabenzotriazol-1-yl)-N,N,N',N'-te-tramethyluronium hexafluorophosphat) zu aktivieren. Die Umsetzung der Säure erfolgte z.B. mit HATU in einem inerten Lösungsmittel wie beispielsweise DMF in Gegenwart des entsprechenden Amins der allgemeinen Formel III und einem tertiären Amin wie beispielsweise Ethyldiisopropylamin bei Temperaturen zwischen - 50°C und + 60°C, vorzugsweise bei 0°C bis 30°C, bzw. alternativ zwischen 80°C und 140°C in der Mikrowelle.

Eine weitere Möglichkeit besteht darin die Säurefunktion in den Verbindungen der allgemeinen Funktion II zunächst mittels beispielsweise Thionylchlorid, Phosphoroxychlorid, Phosphorpentachlorid oder auch Oxalylchlorid in das entsprechende Säurechlorid zu überführen und dann die Umsetzung zu den Verbindungen der allgemeinen Formel I beispielsweise in Pyridin oder einem inerten Lösungsmittel wie beispielsweise DMF in Gegenwart des entsprechenden Amins der allgemeinen Formel III oder IV und einem tertiären Amin wie beispielsweise Ethyldiisopropylamin bei Temperaturen zwischen - 50°C und + 60°C, vorzugsweise bei 0°C bis 30°C, durchzuführen.

Für den Fall, dass R⁶ C₁-C₆-Alkyl bedeutet, kann beispielsweise auch eine direkte Amidolyse des Esters mit dem entsprechenden Amin eventuell unter zu Hilfenahme von Aluminiumtrialkyl-Reagenzien, vorzugsweise Aluminiumtrimethyl, durchgeführt werden.

Für den Fall, dass R⁶ ein Chlor- oder Bromatom bedeutet, kann beispielsweise die Umsetzung beispielsweise in Pyridin oder einem inerten Lösungsmittel wie beispielsweise DMF in Gegenwart des entsprechenden Amins der allgemeinen Formel II und einem tertiären Amin wie beispielsweise Ethyldiisopropylamin bei Temperaturen zwischen - 50°C und + 60°C, vorzugsweise bei 0°C bis 30°C, durchgeführt werden.

Gegebenenfalls können die Verbindungen der allgemeinen Formel (I) mit R² oder R³ = CN auch ausgehend von den entsprechenden Halogeniden, bevorzugt ist Brom bzw. Chlor, durch eine Cu- oder Pd-katalysierte (z.B. Pd(AOc)₂) Cyanid-Einführung mit Zn(CN)₂ oder auch K₃[Fe(CN)₆] in einem inerten Lösungsmittel wie Dimethylacetamid, Dimethylforamid oder N-Methylpyrolidon bei Temperaturen zwischen 60°C und dem Siedepunkt des jeweiligen Lösungsmittels hergestellt werden.

Gegebenenfalls können die Verbindungen der allgemeinen Formel (I) mit R⁴ bzw. R⁵ = Aryl bzw. Heteroaryl, die gegebenenfalls mit den zuvor angegebenen Resten substituiert sein können, ausgehend von einem entsprechenden Halogenid, bevorzugt ist Brom bzw. Chlor durch eine Pd-katalysierte (z.B. Pd(AOc)₂, Pd(PPh₃)₄, Pd₂(dba)₃, PdCl₂(dppf)) Reaktion in Gegenwart einer Base wie beispielsweise Natriumcarbonat, Cäsiumcarbonat, Kaliumphosphat oder Ethyldiisoproylamin mit einer entsprechenden Aryl- bzw. Heteroaryl-Boronsäure bzw. Boronsäurederivat in einem Lösungsmittel wie beispielsweise Toluol, Dioxan, Dimethylacetamid, Dimethylforamid oder N-Methylpyrolidon bei Temperaturen zwischen 60°C und dem Siedepunkt des jeweiligen Lösungsmittels hergestellt werden.

Die als Ausgangsmaterialien dienenden Verbindungen der allgemeinen Formel II sind entweder bekannt oder können beispielsweise hergestellt werden, indem in an sich bekannter Weise die bekannten Hydrazine IV, gegebenenfalls hergestellt werden aus den entsprechenden bekannten Anilinen durch Nitrosierung gefolgt von einer Reduktion, 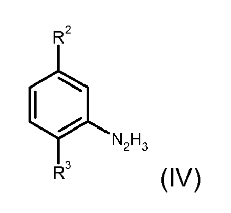 worin R² und R³ die oben angegebene Bedeutung aufweist,
a) mit einem Keton der allgemeinen Formel V, worin R¹ und Y die oben angegebene Bedeutung aufweist und n = 2 und 3 ist, in einer Fischer-Indol-Zyklisierung umgesetzt wird 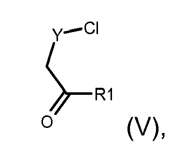 oder
b) mit einem Enolether der allgemeinen Formel VI, worin R¹ und Y die oben angegebene Bedeutung aufweist und n = 2 und 3 ist, in einer Fischer-Indol-Zyklisierung umgesetzt wird (Org. Lett. 2004, 79ff), 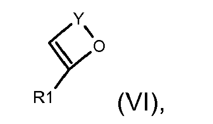 und der anschließend erhaltene Alkohol durch die dem Fachmann bekannten Methoden durch Überführung in eine Abgangsgruppe wie Tosylat, Mesylat, Trifluormesylat, Chlorid, Bromid oder Iodid und anschließende Umsetzung mit z. B. Natriumazid gefolgt von einer Hydrolyse mittels Triphenylphosphin/Wasser in Tetrahydrofuran in die Aminofunktion überführt wird,
   oder
c) mit einem Ketoester der allgemeinen Formel VII, für den Fall von Y mit n = 1 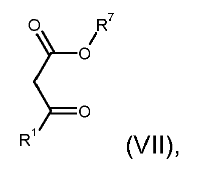
worin R¹ die oben angegebene Bedeutung hat und R⁷ ein C₁-C₆-Akylrest ist, in einer Fischer-Indol-Zyklisierung umgesetzt wird und anschließend der erhaltene Ester mit den dem Fachmann bekannten Methoden wie beispielsweise Diisobutylalumiumhydrid in einem inerten Lösungsmittel bei Temperaturen zwischen - 50 und 25°C, vorzugsweise zwischen - 30 und 0°C, zum entsprechenden Alkohol reduziert wird, der wiederum durch Überführung in eine Abgangsgruppe wie Tosylat, Mesylat, Trifluormesylat, Chlorid, Bromid oder Iodid und anschließende Umsetzung mit z.B. Natriumazid, gefolgt von einer Hydrolyse mittels Triphenylphosphin/Wasser in Tetrahydrofuran in die Aminofunktion überführt wird.

Eine weitere Alternative zur Darstellung der Verbindungen der allgemeinen Formel II wäre zunächst die Umsetzung von bekannten Indolen VIII, worin R¹ bis R³ die oben angegebene Bedeutung aufweist,oder nach dem Fachmann bekannten Indolsynthesen (Chem. Rev. 2006, 2875 bzw. J. Chem. Soc., Perkin Trans 1, 2000, 1045) aufzubauenden Indolen VIII 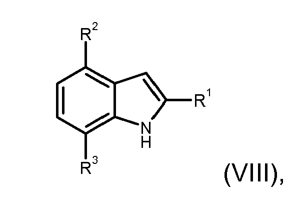 worin R¹ bis R³ die oben angegebene Bedeutung aufweist, mit Formaldehyd / Dimethylamin in Gegenwart einer Base wie beispielsweise Kaliumcarbonat in einem inerten Lösungsmittel wie beispielsweise Dioxan bei Temperaturen zwischen 0°C und dem Siedepunkt des jeweiligen Lösungsmittels, vorzugsweise zwischen 60°C und 80°C zu den Verbindungen der allgemeinen Formel IX 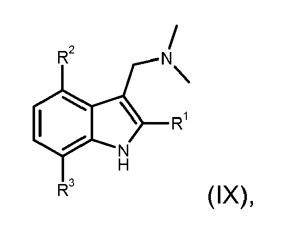 worin R¹ bis R³ die oben angegebene Bedeutung aufweist. Die Verbindungen der allgemeinen Formel IX werden dann durch Reaktion von Natrium- oder Kaliumcyanid in einem Lösungsmittelgemisch wie vorzugsweise DMF / Wasser unter Rückfluß in das um ein C-Atom verlängerte Nitril überführt, welches anschließend durch eine Reduktion mit Lithiumaluminiumhydrid in einem inerten Lösungsmittel wie beispielsweise Diethylether oder Tetrahydrofuran unter Rückfluß oder alternativ mittels Natriumborhydrid / Kobaltdiacetat in Ethanol oder Methanol vorzugsweise bei Temparaturen zwischen 10°C und 40°C die Verbindungen der allgemeinen Formel II ergeben.

Sollten für Umsetzungen Schutzgruppen nötig sein, so können diese auf Vorstufen oder für den benötigten Schritt nach dem den Fachmann bekannten Methoden (Protective groups in organic synthesis, T. W. Greene und P. G. M. Wuts, John Wiley & Sons, 1999) eingeführt werden und gegebenenfalls anschließend oder auf einer späteren Stufe der Synthese auch wieder gespalten werden.

### Herstellung der erfindungsgemäßen Verbindungen

Die nachfolgenden Beispiele erläutern die Herstellung der erfindungsgemäßen Verbindungen der allgemeinen Formel (I), ohne den Umfang der beanspruchten Verbindungen auf diese Beispiele zu beschränken.
Die erfindungsgemäßen Verbindungen der allgemeinen Formel (I) lassen sich wie nachstehend beschrieben herstellen.

### Beispiel 1: N-[2-(7-Chlor-2-methyl-1H-indol-3-yl)-ethyl]-3,4-dimethoxy-benzamid

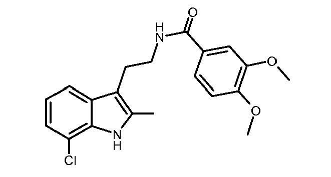

Zu einer Lösung von 100 mg 2-(7-Chlor-2-methyl-1H-indol-3-yl)-ethylamin Hydrochlorid in 6 ml Tetrahydrofuran (alternativ auch Dimethylformamid) wurden 0.14 ml Triethylamin zugegeben und 10 Minuten bei 25°C gerührt. Anschließend wurden bei dieser Temperatur 123 mg 3,4-Dimethoxybenzoesäurechlorid hinzugegeben und für weitere 15 Stunden bei 25° C gerührt. Anschließend wurde die Reaktionslösung auf Eiswasser gegeben und zweimal extrahiert mit Ethylacetat. Die vereinigten organischen Phasen wurden zweimal mit Wasser gewaschen, über Natriumsulfat getrocknet und nach Filtration im Vakuum eingeengt. Der so erhaltene Rückstand wird durch Mitteldruck-Chromatographie an Kieselgel mit Hexan/0 - 100 % Ethylacetat gereinigt. Ausbeute: 103 mg der Titelverbindung.
NMR (300 MHz, DMSO-d6): δ = 2.30 (3H), 2.84 (2H), 3.35 (2H), 3.75 (3H), 3.76 (3H), 6.90 (1H), 6.97 (1H), 7.01 (1H), 7.36-7.45 (3H), 8.41 (1H), 11.00 (1H).

### Beispiel 2: Biphenyl-4,4'-dicarbonsäure 4'-{[2-(7-chlor-2-methyl-1H-indol-3-yl)-ethyl]-amid} 4-methylamid

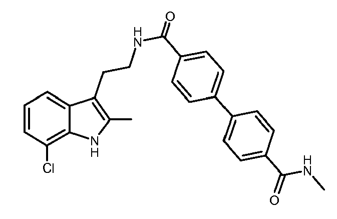

Zu einer Lösung von 156 mg Biphenyl-4,4'-dicarbonsäure-4-methylamid in 1.7 ml Dimethylsulfoxid (alternativ auch Dimethylformamid) wurden 256 mg N-[(Dimethylamino)-1*H*-1,2,3-triazolo[4,5-*b*]pyridin-1-ylmethylen]-*N-*Methylmethanaminiumhexafluorphosphat-N-oxid (HATU) gegeben und 100 mg 2-(7-Chlor-2-methyl-1 H-indol-3-yl)-ethylamin Hydrochlorid. Bei 0°C wurden dann 0.12 ml Ethyldiisopropylamin zugetropft und 20 Stunden bei 25°C gerührt. Die Mischung wurde durch HPLC (Säule: XBridge C18 5µ 100x30 mm, Laufmittel: 99% eines Gemisches aus Wasser mit 0.1% Ameisensäure / 1% Acetonitril bis zu 1% eines Gemisches aus Wasser mit 0.1% Ameisensäure / 99% Acetonitril, Detektion per MS ESI (+)) gereinigt. Ausbeute: 38 mg der Titelverbindung.
NMR (300 MHz, DMSO-d6): δ = 2.35 (3H), 2.82 (3H), 2.92 (2H), 3.44 (2H), 6.96 (1H), 7.06 (1H), 7.47 (1H), 7.84 (4H), 7.95 (4H), 8.53 (1H), 8.67 (1H), 11.05 (1H).

### Beispiel 3: Biphenyl-3,4'-dicarbonsäure 4'-{[2-(7-chlor-2-methyl-1H-indol-3-yl)-ethyl]-amid} 3-methylamid

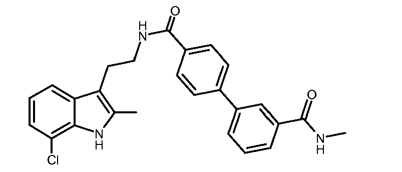

In Analogie zu Beispiel 2 wurden aus 100 mg 2-(7-Chlor-2-methyl-1 H-indol-3-yl)-ethylamin Hydrochlorid und 183 mg Biphenyl-3,4'-dicarbonsäure 3-methylamid 48 mg der Titelverbindung erhalten.
NMR (300 MHz, DMSO-d6): δ = 2.36 (3H), 2.83 (3H), 2.92 (2H), 3.45 (2H), 6.96 (1H), 7.06 (1H), 7.48 (1H), 7.58 (1H), 7.81-7.99 (6H), 8.18 (1H), 8.59 (1H), 8.67 (1H), 11.05 (1H).

### Beispiel 4: N-[2-(7-Brom-2,4-dimethyl-1H-indol-3-yl)-ethyl]-3,4-dimethoxy-benzamid

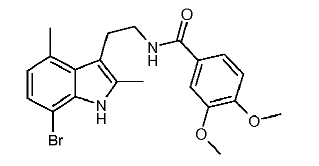

In Analogie zu Beispiel 1 wurden aus 100 mg 2-(7-Brom-2,4-dimethyl-1H-indol-3-yl)-ethylamin Hydrochlorid und 90 mg 3,4-Dimethoxybenzoesäurechlorid 37 mg der Titelverbindung erhalten.
NMR (300 MHz, DMSO-d6): δ = 2.30 (3H), 2.61 (3H), 2.94 (2H), 3.31 (2H), 3.76 (3H), 3.77 (3H), 6.60 (1H), 6.98 (1H), 7.01 (1H), 7.40 (1H), 7.44 (1H), 8.49 (1H), 10.81 (1H).

Das Ausgangsmaterial für die obige Titelverbindung wurde wie folgt hergestellt:
4a) 2-(7-Brom-2,4-dimethyl-1H-indol-3-yl)-ethylamin 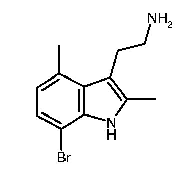 5.8g 2-Brom-5-methylphenylhydrazin Hydrochlorid wurden in 78 ml eines Gemisches aus Ethanol und Wasser im Verhältnis 14 : 1 bei 120°C gelöst. Anschließend wurden in der Siedehitze 2.8 ml 5-Chlor-2-pentanon gelöst in 2 ml Ethanol hinzugegeben und bei dieser Temperatur für 16 Stunden gerührt. Nach dem Abkühlen wurde im Vakuum eingeengt und mit 100ml Wasser und Natronlauge versetzt (pH ca. 10). Dann wurde mit Ether/Essigester 1:1 dreimal extrahiert, die vereinigten organischen Phasen mit Wasser gewaschen und über Natriumsulfat getrocket. Nach Filtration wurde im Vakuum eingeengt und der so erhaltene Rückstand durch Säulenchromatographie an Kieselgel mit Methylenchlorid/0 - 20 % Methanol/0.5 % Triethylamin gereinigt Ausbeute: 2.45 g der Titelverbindung.
   NMR (300 MHz, DMSO-d6): δ = 2.30 (3H), 2.51 (3H), 2.62 (2H), 2.75 (2H), 6.57 (1H), 6.98 (1H), 10.75 (1H).
4b) 2-Brom-5-methyl-phenylhydrazin Hydrochlorid 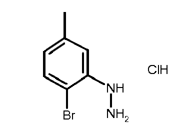 Zu einer Lösung von 5.0 g 2-Brom-5-methyl-Anilin in 17 ml Salzsäure (37 %ig) wurden bei 0°C tropfenweise, innerhalb von 30 Minuten eine Lösung von 1.9 g Natriumnitrit in 9.6 ml Wasser zugegeben. Anschließend wurde bei 0°C eine Lösung aus 16.6 g Zinnchlorid in 15 ml Salzsäure (37 %ig) zugetropft und tropft für weitere 1.5 Stunden bei dieser Temperatur. gerührt. Nach Zugabe von 60 ml Natronlauge (50 %ig) und 30 ml Eiswasser (pH >10) wurde dreimal mit je 500 ml Ether extrahiert. Die vereinigten organischen Phasen wurden mit halbgesättigter Natriumchlorid-Lösung gewaschen, getrocknet über Natriumsulfat. Das Filtrat wurde mit 4.0 M Salzsäure in 1,4 Dioxan-Lösung angesäuert und der erhaltene Niederschlag dann abfiltiert und getrocknet. Ausbeute: 5.85 g der Titelverbindung.
   NMR (300 MHz, DMSO-d6): δ = 2.22 (3H), 6.70 (1H), 6.93 (1H), 7.39 (1H), 7.78 (1H), 10.29 (2H).

### Beispiel 5: N-[2-(4,7-Dichlor-2-methyl-1H-indol-3-yl)-ethyl]-3,4-dimethoxy-benzamid

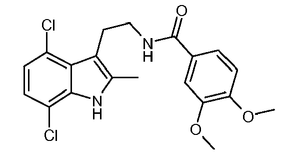

In Analogie zu Beispiel 1 wurden aus 50 mg 2-(4,7-Dichlor-2-methyl-1 H-indol-3-yl)-ethylamin Hydrochlorid und 36 mg 3,4-Dimethoxybenzoesäurechlorid 36 mg der Titelverbindung erhalten.
NMR (300 MHz, DMSO-d6): δ = 2.27 (3H), 3.04 (2H), 3.41 (2H), 3.75 (3H), 3.76 (3H), 6.94 (1H), 6.96 (1H), 7.01 (1H), 7.38 (1H), 7.41 (1H), 8.39 (1H), 11.37 (1H).

Benötigte Startmaterialien zu Verbindungen in der folgenden Tabelle:
A) 2-(7-Chlor-4-fluor-2-methyl-1 H-indol-3-yl)-ethylamin 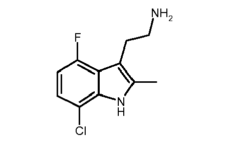 In Analogie zu Beispiel 4a) wurden aus 6 g 2-Chlor-4-Fluor-phenylhydrazin Hydrochlorid 1.8 g 2-(7-Chlor-4-fluor-2-methyl-1 H-indol-3-yl)-ethylamin erhalten. NMR (300 MHz, DMSO-d6): δ = 2.32 (3H), 2.91 (4H), 6.72 (1H), 6.99 (1H), 11.45 (1H).
B) 2-(7-Chlor-2,4-dimethyl-1H-indol-3-yl)-ethylamin 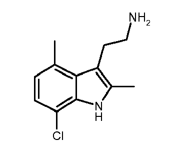
   B1) In Analogie zu Beispiel 4b) wurden aus 5.0 g 2-Chlor-5-methyl-phenylamin 6.5 g (2-Chlor-5-methyl-phenyl)-hydrazin Hydrochlorid erhalten.
   B2) In Analogie zu Beispiel 4a) wurden aus 6.5 g des vorstehend hergestellten Hydrazins 2.75 g 2-(7-Chlor-2,4-dimethyl-1 H-indol-3-yl)-ethylamin erhalten. NMR (300 MHz, DMSO-d6): δ = 2.30 (3H), 2.64 (2H), 2.77 (2H), 6.61 (1H), 6.84 (1H), 10.90 (1H).
C) 2-(7-Brom-2-methyl-1 H-indol-3-yl)-ethylamin 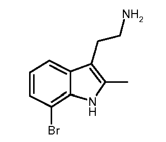 In Analogie zu Beispiel 4a) wurden aus 6.5 g 2-Brom-phenylhydrazin Hydrochlorid 7.2 g 2-(7-Brom-2-methyl-1 H-indol-3-yl)-ethylamin erhalten.
   NMR (300 MHz, DMSO-d6): δ = 2.34 (3H), 2.98 (4H), 6.87 (1H), 7.17 (1H), 7.46 (1H), 11.01 (1H).
D) 2-(7-Chlor-2-trifluormethyl-1H-indol-3-yl)-ethylamin 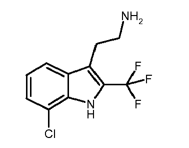
   D1) (2-Amino-3-chlor-phenyl)-methanol
      Zu einer Lösung von 10 g Lithiumaluminiumhydrid in 1I THF wurden vorsichtig bei 10°C portionsweise 30 g 2-Amino-3-Chlor-benzoesäure hinzugegeben und anschließend 2 Stunden unter Rückfluß erhitzt. Nach dem Abkühlen wurden tropfenweise 10 ml Wasser gefolgt von einer Lösung von 3.3 g Natriumhydroxid in 10 ml Wasser hinzugegeben und die Mischung 30 Minuten unter Rückfluß erhitzt. Nach dem Abkühlen und Filtration wurde im Vakuum eingeengt und der so erhaltene Rückstand durch Säulenchromatographie an Kieselgel mit Chloroform/Methanol 19:1 gereinigt. Ausbeute: 19.1 g (2-Amino-3-chlor-phenyl)-methanol.
      NMR (300 MHz, DMSO-d6): δ = 4.53 (2H), 5.08 (2H), 5.20 (1 H), 6.58 (1 H), 7.13 (1H), 7.17 (1H).
   D2) N-(2-Chlor-6-hydroxymethyl-phenyl)-2,2,2-trifluor-acetamid
      Zu einer Lösung von 18.8 g des vorstehend hergestellten Amins in 400 ml Methylenchlorid wurden bei 0°C tropfenweise 50.1 g Trifluoressigsäureanhydrid gegeben und anschließend 16 Stunden bei 24°C gerührt. Dann wurde die organische Phase mit 15%iger Kaliumcarbonat-Lösung gewaschen, über Natriumsulfat getrocknet und nach Filtration im Vakuum eingeengt. Der Rückstand wurde mit heißem Hexan extrahiert. Nach dem Einengen im Vakuum wurden ohne weitere Reinigung 10.5 g N-(2-Chlor-6-hydroxymethyl-phenyl)-2,2,2-trifluor-acetamid erhalten.
      NMR (300 MHz, DMSO-d6): δ = 5.46 (2H), 7.54 (1H), 7.58 (1H), 7.75 (1H), 11.40 (1H).
   D3) 7-Chlor-2-trifluormethyl-1 H-indol
      Eine Mischung von 10.5 g des vorstehend hergestellten Amids und 15.6 g Triphenylphosphin Hydrobromid (PPh₃ x HBr) in 300 ml Acetonitril wurde für 17 Stunden unter Rückfluß erhitzt. Nach dem Abkühlen wurde im Vakuum eingeengt und der Rückstand mit 200 ml Ether gewaschen. Nach dem Trocknen an der Luft wurden 23.9 g Phosphoniumsalz erhalten, welches in 600 ml DMF gelöst wurde und anschließemd für 20 Stunden unter Rückfluß erhitzt wurde. Nach dem Abkühlen wurde im Vakuum eingeengt und der so erhaltene Rückstand durch Säulenchromatographie an Kieselgel mit Hexan gereinigt. Ausbeute: 4.2 g 7-Chlor-2-trifluormethyl-1H-indol.
      NMR (300 MHz, DMSO-d6): δ = 7.53 (1H), 7.62 (1H), 7.81 (1H), 7.62 (1H), 12.58 (1H).
   D4) (7-Chlor-2-trifluormethyl-1 H-indol-3-ylmethyl)-dimethyl-amin
      Zu einer Lösung von 1.98 g Kaliumcarbonat in 40 ml Essigsäure wurden bei -10°C 2.33 g Dimethylamin Hydrochlorid in 40 ml Dioxan gegeben, gefolgt von 1.74 ml 40% Formaldehyd-Lösung und 4.2 g des zuvor hergestellten Indols in 40 ml Dioxan. Anschließend wurde diese Mischung bei 25°C für 2 Stunden gerührt und dann für weitere 5 Stunden auf 80°C erwärmt. Dann wurde die Reaktionsmischung nach dem Abkühlen im Vakuum eingeengt und zu 15%iger Kaliumcarbonat-Lösung gegeben. Nach dreimaliger Extraktion mit jeweils 100 ml Essigester wurden die vereinigten organischen Phasen über Natriumsulfat getrocknet. Nach Filtration wurde im Vakuum eingeengt und der so erhaltene Rückstand mittels Säulenchromatographie an Kieselgel mit Hexan / Essigester 19:1 gereinigt. Ausbeute: 4.46 g der Titelverbindung als Feststoff.
      NMR (300 MHz, DMSO-d6): δ = 2.15 (6H), 3.61 (2H), 7.11 (1H), 7.36 (1H), 7.76 (1H), 12.32 (1H).
   D5) (7-Chlor-2-trifluormethyl-1H-indol-3-yl)-acetonitril
      Zu einer Lösung von 4.4 g des zuvor hergestellten Amins in 50 ml DMF wurde eine Lösung von 10.35 g Kaliumcyanid in 50 ml Wasser gegeben und diese Mischung 2 Stunden unter Rückfluß erhitzt. Nach dem Abkühlen wurde im Vakuum eingeengt und der so erhaltene Rückstand durch Säulenchromatographie an Kieselgel mit Hexan / Essigester 9:1 gereinigt. Ausbeute: 2.37 g der Titelverbindung als Feststoff.
      NMR (300 MHz, DMSO-d6): δ = 4.28 (2H), 7.22 (1H), 7.44 (1H), 7.86 (1H), 12.76 (1 H).
   D6) 2-(7-Chlor-2-trifluormethyl-1 H-indol-3-yl)-ethylamin
      Zu einer Lösung von 0.5 g des vorstehend hergestellten Nitrils in einem Gemisch aus 7 ml THF und 2 ml Wasser wurde unter Rühren bei 25°C eine Lösung von 250 mg Kobaltdiacetat-tetrahydrat in 5 ml Wasser gegeben, gefolgt von 720 mg Natriumborhydrid und anschließend bei dieser Temperatur 30 Minuten gerührt. Dann wurde die Mischung im Vakuum eingeengt und der so erhaltene Rückstand durch Säulenchromatographie an Kieselgel mit Chloroform / Methanol / aq. Ammoniak im Verhältnis 100:10:1 gereinigt. Ausbeute: 200 mg der Titelverbindung als Feststoff.
      NMR (300 MHz, DMSO-d6): δ = 2.70 (2H), 2.88 (2H), 5.14 (2H), 7.08 (1H), 7.31 (1H), 7.66 (1H).
E) 2-(2-tert-Butyl-1H-indol-3-yl)-ethylamin 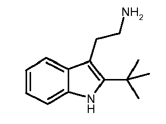
   E1) (2-tert-Butyl-1H-indol-3-ylmethyl)-dimethyl-amin
      In Analogie zu der in D4) hergestellten Verbindung wurden aus 1.0 g 2-tert-Butyl-1 H-indol 1.21 g (2-tert-Butyl-1 H-indol-3-ylmethyl)-dimethyl-amin als weißen Feststoff erhalten.
      NMR (300 MHz, DMSO-d6): δ = 1.46 (9H), 3.52 (2H), 6.95 (2H), 7.27 (1H), 7.50 (1H), 10.56 (1H).
   E2) (2-tert-Butyl-1H-indol-3-yl)-acetonitril
      In Analogie zu der in D5) hergestellten Verbindung wurden aus 6.21 g des zuvor hergestellten Amins (E1) 4.7 g (2-tert-Butyl-1 H-indol-3-yl)-acetonitril als weißen Feststoff erhalten.
      NMR (300 MHz, DMSO-d6): δ = 1.47 (9H), 4.10 (2H), 7.03 (2H), 7.34 (1H), 7.53 (1H), 10.78 (1H).
   E3) 2-(2-tert-Butyl-1H-indol-3-yl)-ethylamin
      Zu einer Mischung von 1.0 g Lithiumaluminiumhydrid in 400 ml Ether wurden vorsichtig 1.1 g des vorstehend hergestellten Nitrils (E2) gegeben und 48 Stunden unter Rückfluß erhitzt. Nach dem Abkühlen wurden vorsichtig nacheinander 1 ml Wasser gefolgt von einer Lösung von 0.3 g Natriumhydroxid in 1 ml Wasser hinzugegeben und diese Mischung wurde nochmals unter Rückfluß erhitzt. Nach dem Abkühlen wurde abfiltriert und das Filtrat im Vakuum eingeengt. Ausbeute (ohne weitere Reinigung): 0.76 g 2-(2-tert-Butyl-1 H-indol-3-yl)-ethylamin als weißen Feststoff.
      NMR (300 MHz, DMSO-d6): δ = 1.44 (9H), 2.72 (2H), 2.88 (2H), 6.89 (1H), 6.95 (1H), 7.27 (1H), 7.41 (1H), 10.36 (1H).

In Analogie zu Beispiel 1 bzw. 2 werden die folgenden Beispiele hergestellt und per HPLC gereinigt:

### HPLC-Methode:

Gerät: analytisches 4-Kanal MUX-System mit CTC Pal Injektor, Waters 1525 Pumpen, Waters 2488 UV-Detektor und Waters ZQ 2000 single quad MS Detektor.
Säule X-Bridge RP C18 4.6x50 3.5µm; Detektionswellenlänge 214 nm; Flussrate 2 ml/min; Eluenten A: 0.1% TFA in Wasser, B 0.1% TFA in Acetonitril; Gradient jeweils bezogen auf B: 1% auf 99% (5') auf 99% (1') auf 1% (0.25') auf 1% (1.75')

| Bei- spiel | Struktur | Name | Theoretische Masse m/z [M]⁺ | Gefundene Masse m/z [M+H]⁺ | Retentionszeit [min.] |
|---|---|---|---|---|---|
| 6 | 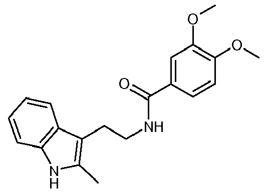 | 3,4-Dimethoxy-N-[2-(2-methyl-1H-indol-3-yl)-ethyl]-benzamid | 338 | 339 | 1,09 |
| 7 | 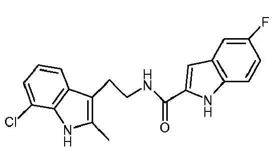 | 5-Fluor-1H-indol-2-carbonsäure [2-(7-chlor-2-methyl-1H-indol-3-yl)-ethyl]-amid | 369 | 370 | 1,25 |
| 8 | 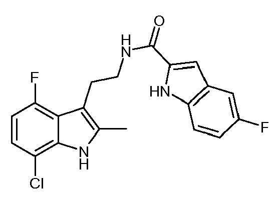 | 5-Fluor-1H-indol-2-carbonsäure [2-(7-chlor-4-fluor-2-methyl-1H-indol-3-yl)-ethyl]-amid | 387 | 388 | 1,34 |
| 9 | 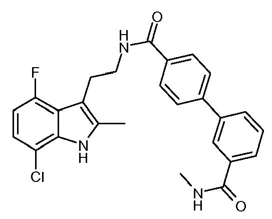 | Biphenyl-3,4'-dicarbonsäure 4'-{[2-(7-chlor-4-fluor-2-methyl-1H-indol-3-yl)-ethyl]-amid} 3-methylamid | 463 | 464 | 1,24 |
| 10 | 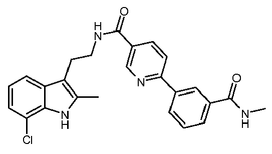 | N-[2-(7-Chlor-2-methyl-1 H-indol-3-yl)-ethyl]-6-(3-methylcarbamoyl-phenyl)-nicotinamid | 446 | 447 | 1,15 |
| 11 | 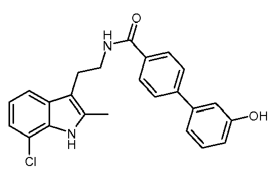 | 3'-Hydroxy-biphenyl-4-carbonsäure [2-(7-chlor-2-methyl-1H-indol-3-yl)-ethyl]-amid | 404 | 405 | 1,27 |
| 12 | 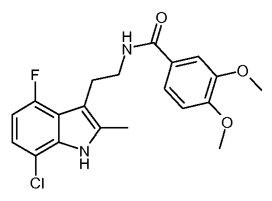 | N-[2-(7-Chlor-4-fluor-2-methyl-1H-indol-3-yl)-ethyl]-3,4-dimethoxy-benzamid | 390 | 391 | 1,25 |
| 13 | 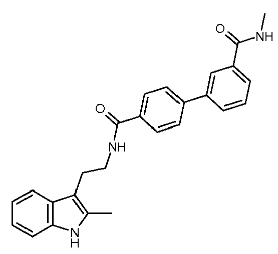 | Biphenyl-3,4'-dicarbonsäure 3-methylamid 4'-{[2-(2-methyl-1H-indol-3-yl)-ethyl]-amid} | 411 | 412 | 1,13 |
| 14 | 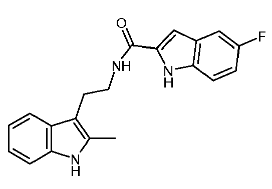 | 5-Fluor-1H-indol-2-carbonsäure [2-(2-methyl-1H-indol-3-yl)-ethyl]-amid | 335 | 336 | 1,24 |
| 15 | 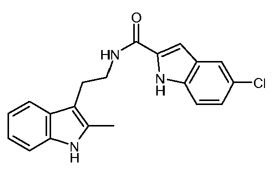 | 5-Chlor-1H-indol-2-carbonsäure [2-(2-methyl-1H-indol-3-yl)-ethyl]-amid | 351 | 352 | 1,31 |
| 16 | 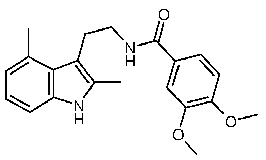 | N-[2-(2,4-Dimethyl-1 H-indol-3-yl)-ethyl]-3,4-dimethoxy-benzamid | 352 | 353 | 1,15 |
| 17 | 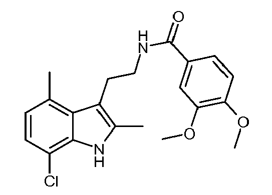 | N-[2-(7-Chlor-2,4-dimethyl-1H-indol-3-yl)-ethyl]-3,4-dimethoxy-benzamid | 386 | 387 | 1,23 |
| 18 | 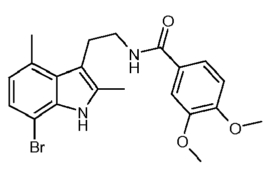 | N-[2-(7-Brom-2,4-dimethyl-1H-indol-3-yl)-ethyl]-3,4-dimethoxy-benzamid | 432 | 433 | 1,27 |
| 19 | 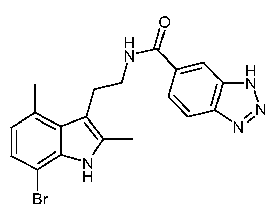 | 3H-Benzotriazol-5-carbonsäure [2-(7-brom-2,4-dimethyl-1H-indol-3-yl)-ethyl]-amid | 413 | 414 | 1,03 |
| 20 | 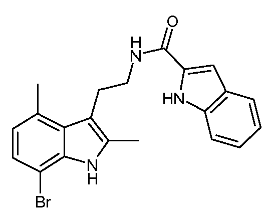 | 1H-Indol-2-carbonsäure [2-(7-brom-2,4-dimethyl-1H-indol-3-yl)-ethyl]-amid | 411 | 412 | 1,3 |
| 21 | 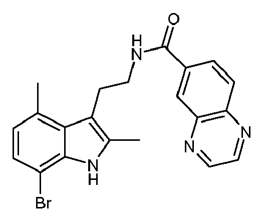 | Chinoxalin-6-carbonsäure [2-(7-brom-2,4-dimethyl-1H-indol-3-yl)-ethyl]-amid | 424 | 425 | 1,13 |
| 22 | 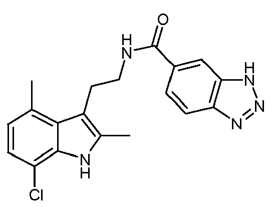 | 3H-Benzotriazol-5-carbonsäure [2-(7-chlor-2,4-dimethyl-1H-indol-3-yl)-ethyl]-amid | 367 | 368 | 1,07 |
| 23 | 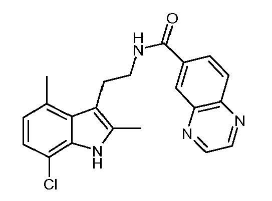 | Chinoxalin-6-carbonsäure [2-(7-chlor-2,4-dimethyl-1H-indol-3-yl)-ethyl]-amid | 378 | 379 | 1,19 |
| 24 | 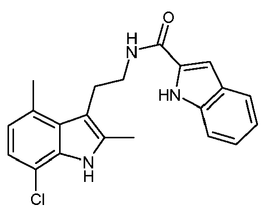 | 1H-Indol-2-carbonsäure [2-(7-chlor-2,4-dimethyl-1H-indol-3-yl)-ethyl]-amid | 365 | 366 | 1,36 |
| 25 | 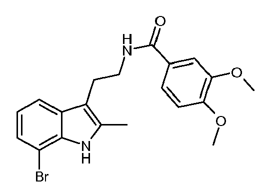 | N-[2-(7-Brom-2-methyl-1H-indol-3-yl)-ethyl]-3,4-dimethoxy-benzamid | 418 | 419 | 1,24 |
| 26 | 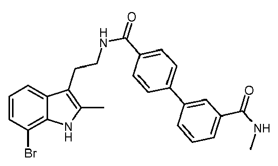 | Biphenyl-3,4'-dicarbonsäure 4'-{[2-(7-brom-2-methyl-1H-indol-3-yl)-ethyl]-amid} 3-methylamid | 491 | 492 | 1,26 |
| 27 | 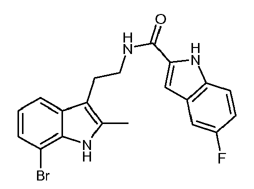 | 5-Fluor-1H-indol-2-carbonsäure [2-(7-brom-2-methyl-1H-indol-3-yl)-ethyl]-amid | 415 | 416 | 1,36 |
| 28 | 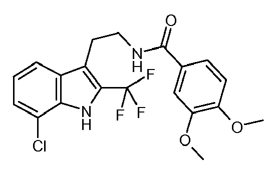 | N-[2-(7-Chlor-2-trifluormethyl-1H-indol-3-yl)-ethyl]-3,4-dimethoxy-benzamid | 426 | 427 | 1,24 |
| 29 | 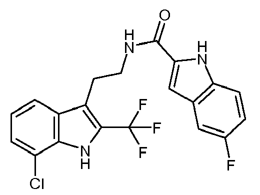 | 5-Fluor-1H-indol-2-carbonsäure [2-(7-chlor-2-trifluormethyl-1H-indol-3-yl)-ethyl]-amid | 423 | 424 | 1,37 |
| 30 | 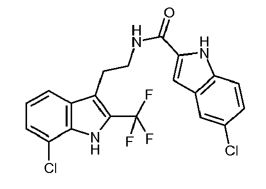 | 5-Chlor-1H-indol-2-carbonsäure [2-(7-chlor-2-trifluormethyl-1H-indol-3-yl)-ethyl]-amid | 439 | 438 [M-H]⁻ | 1,43 |
| 31 | 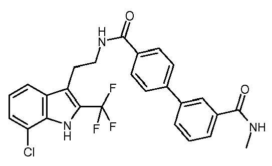 | Biphenyl-3,4'-dicarbonsäure 4'-{[2-(7-chlor-2-trifluormethyl-1H-indol-3-yl)-ethyl]-amid} 3-methylamid | 499 | 498 | 1,26 |
| 32 | 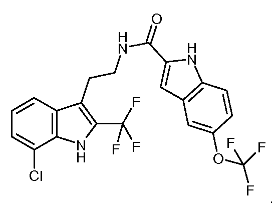 | 5-Trifluormethoxy-1H-indol-2-carbonsäure [2-(7-chlor-2-trifluormethyl-1H-indol-3-yl)-ethyl]-amid | 489 | 488 [M-H]⁻ | 1,48 |
| 33 | 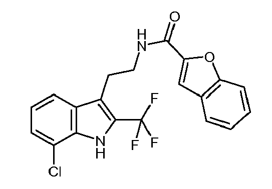 | Benzofuran-2-carbonsäure [2-(7-chlor-2-trifluormethyl-1H-indol-3-yl)-ethyl]-amid | 406 | 405 [M-H]⁻ | 1,39 |
| 34 | 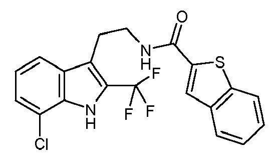 | Benzo[b]thiophen-2-carbonsäure [2-(7-chlor-2-trifluormethyl-1H-indol-3-yl)-ethyl]-amid | 422 | 421 [M-H]⁻ | 1,43 |
| 35 | 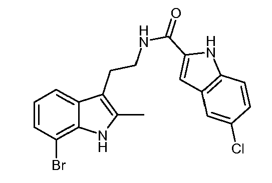 | 5-Chlor-1H-indol-2-carbonsäure [2-(7-brom-2-methyl-1H-indol-3-yl)-ethyl]-amid | 430 | 431 | 1,43 |
| 36 | 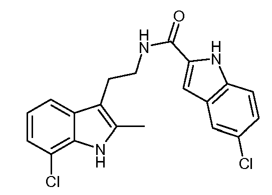 | 5-Chlor-1H-indol-2-carbonsäure [2-(7-chlor-2-methyl-1H-indol-3-yl)-ethyl]-amid | 385 | 386 | 1,39 |
| 37 | 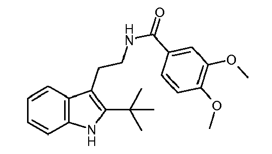 | N-[2-(2-tert-Butyl-1H-indol-3-yl)-ethyl]-3,4-dimethoxy-benzamid | 380 | 381 | 1,26 |
| 38 | 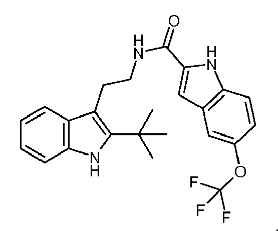 | 5-Trifluormethoxy-1H-indol-2-carbonsäure [2-(2-tert-butyl-1H-indol-3-yl)-ethyl]-amid | 443 | 444 | 1,51 |
| 39 | 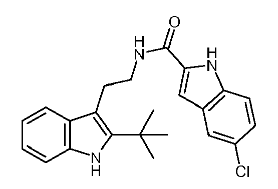 | 5-Chlor-1H-indol-2-carbonsäure [2-(2-tert-butyl-1H-indol-3-yl)-ethyl]-amid | 393 | 394 | 1,46 |
| 40 | 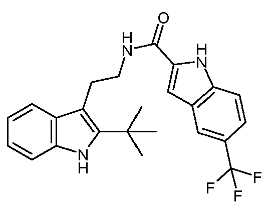 | 5-Trifluormethyl-1H-indol-2-carbonsäure [2-(2-tert-butyl-1H-indol-3-yl)-ethyl]-amid | 427 | 428 | 1,49 |
| 41 | 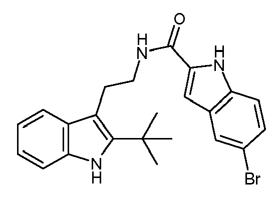 | 5-Brom-1H-indol-2-carbonsäure [2-(2-tert-butyl-1H-indol-3-yl)-ethyl]-amid | 439 | 440 | 1,48 |
| 42 | 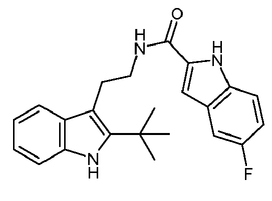 | 5-Fluor-1H-indol-2-carbonsäure [2-(2-tert-butyl-1H-indol-3-yl)-ethyl]-amid | 377 | 378 | 1,39 |
| 43 | 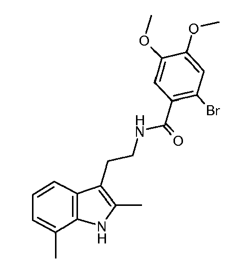 | 2-Brom-N-[2-(2,7-dimethyl-1H-indol-3-yl)-ethyl]-4,5-dimethoxy-benzamid | 431 | 432 | 9,1 |
| 44 | 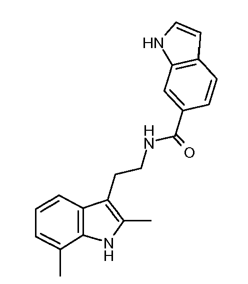 | 1H-Indole-6-carbonsäure [2-(2,7-dimethyl-1H-indol-3-yl)-ethyl]-amid | 331 | 332 | 8,88 |
| 45 | 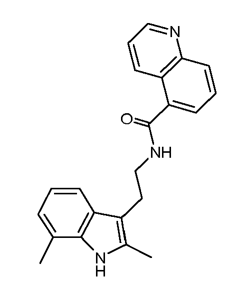 | Chinolin-5-carbonsäure [2-(2,7-dimethyl-1H-indol-3-yl)-ethyl]-amid | 343 | 344 | 6,57 |
| 46 | 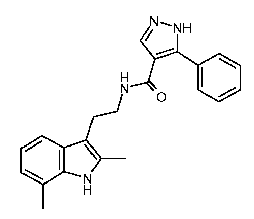 | 5-Phenyl-1H-pyrazol-4-carbonsäure [2-(2,7-dimethyl-1H-indol-3-yl)-ethyl]-amid | 358 | 359 | 8,26 |
| 47 | 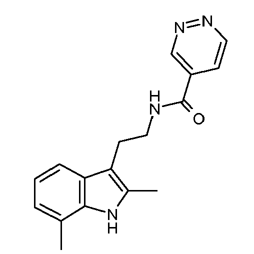 | Pyridazin-4-carbonsäure [2-(2,7-dimethyl-1H-indol-3-yl)-ethyl]-amid | 294 | 295 | 7,22 |
| 48 | 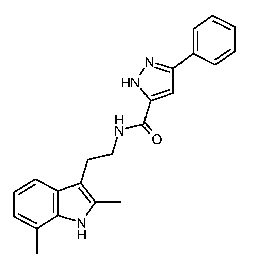 | 5-Phenyl-2H-pyrazol-3-carbonsäure [2-(2,7-dimethyl-1H-indol-3-yl)-ethyl]-amid | 358 | 359 | 9,15 |
| 49 | 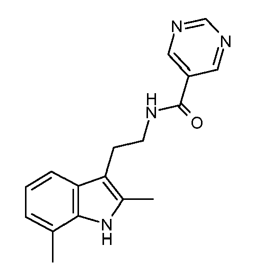 | Pyrimidin-5-carbonsäure [2-(2,7-dimethyl-1H-indol-3-yl)-ethyl]-amid | 294 | 295 | 7,48 |
| 50 | 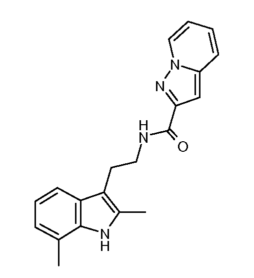 | Pyrazolo[1,5-a]pyridin-2-carbonsäure [2-(2,7-dimethyl-1H-indol-3-yl)-ethyl]-amid | 332 | 333 | 9,07 |
| 51 | 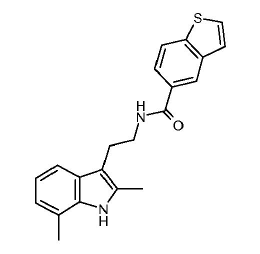 | Benzo[b]thiophen-5-carbonsäure [2-(2,7-dimethyl-1H-indol-3-yl)-ethyl]-amid | 348 | 349 | 9,85 |
| 52 | 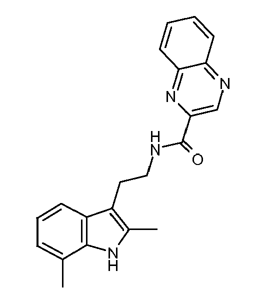 | Chinoxalin-2-carbonsäure [2-(2,7-dimethyl-1H-indol-3-yl)-ethyl]-amid | 344 | 345 | 9,86 |
| 53 | 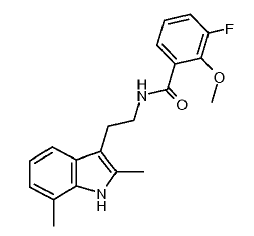 | N-[2-(2,7-Dimethyl-1H-indol-3-yl)-ethyl]-3-fluor-2-methoxy-benzamid | 340 | 341 | 9,86 |
| 54 | 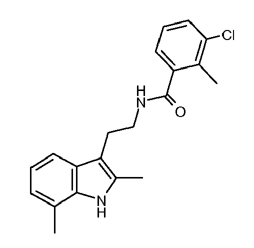 | 3-Chlor-N-[2-(2,7-dimethyl-1H-indol-3-yl)-ethyl]-2-methyl-benzamid | 340 | 341 | 9,98 |
| 55 | 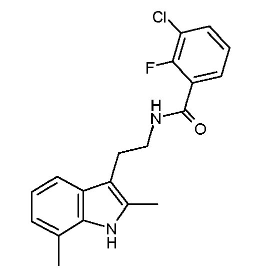 | 3-Chlor-N-[2-(2,7-dimethyl-1H-indol-3-yl)-ethyl]-2-fluor-benzamid | 344 | 345 | 9,97 |
| 56 | 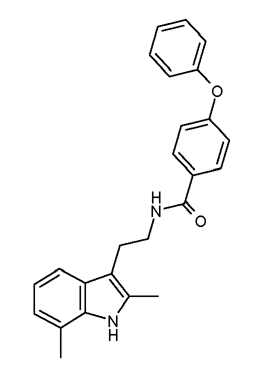 | N-[2-(2,7-Dimethyl-1H-indol-3-yl)-ethyl]-4-phenoxy-benzamid | 384 | 385 | 10,53 |
| 57 | 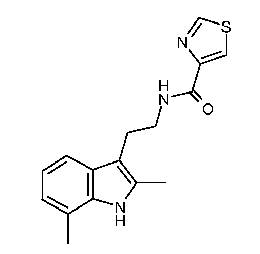 | Thiazol-4-carbonsäure [2-(2,7-dimethyl-1H-indol-3-yl)-ethyl]-amid | 299 | 300 | 8,6 |
| 58 | 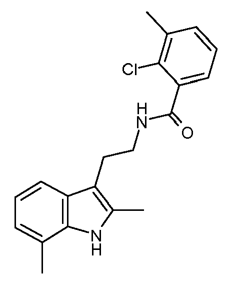 | 2-Chlor-N-[2-(2,7-dimethyl-1H-indol-3-yl)-ethyl]-3-methyl-benzamid | 340 | 341 | 9,7 |
| 59 | 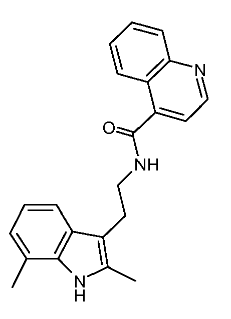 | Chinolin-4-carbonsäure [2-(2,7-dimethyl-1H-indol-3-yl)-ethyl]-amid | 343 | 344 | 7,08 |
| 60 | 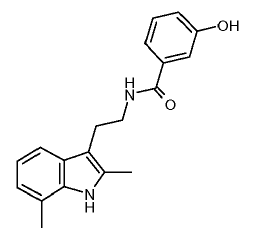 | N-[2-(2,7-Dimethyl-1H-indol-3-yl)-ethyl]-3-hydroxy-benzamid | 308 | 309 | 8,11 |
| 61 | 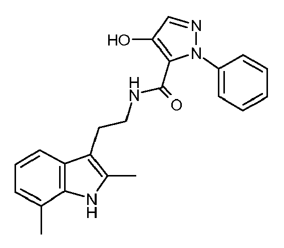 | 4-Hydroxy-2-phenyl-2H-pyrazol-3-carbonsäure [2-(2,7-dimethyl-1H-indol-3-yl)-ethyl]-amid | 374 | 375 | 8,35 |
| 62 | 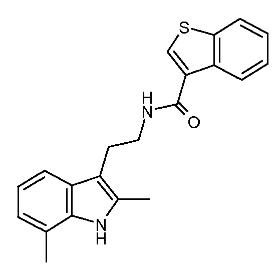 | Benzo[b]thiophen-3-carbonsäure [2-(2,7-dimethyl-1H-indol-3-yl)-ethyl]-amid | 348 | 349 | 10,08 |
| 63 | 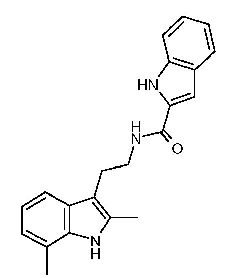 | 1H-Indol-2-carbonsäure [2-(2,7-dimethyl-1H-indol-3-yl)-ethyl]-amid | 331 | 332 | 9,55 |
| 64 | 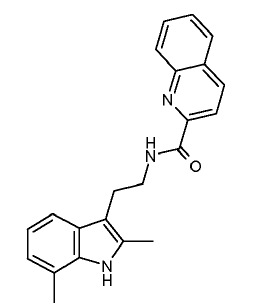 | Chinolin-2-carbonsäure [2-(2,7-dimethyl-1H-indol-3-yl)-ethyl]-amid | 343 | 344 | 10,43 |
| 65 | 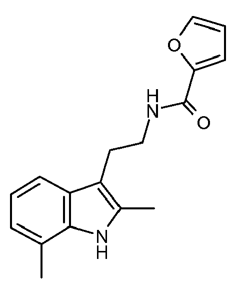 | Furan-2-carbonsäure [2-(2,7-dimethyl-1H-indol-3-yl)-ethyl]-amid | 282 | 283 | 8,55 |
| 66 | 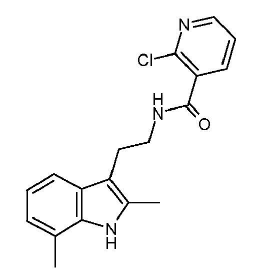 | 2-Chlor-N-[2-(2,7-dimethyl-1H-indol-3-yl)-ethyl]-nicotinamid | 327 | 328 | 8,28 |
| 67 | 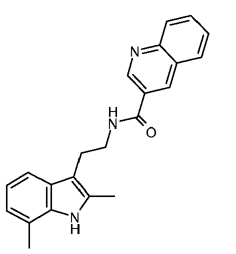 | Chinolin-3-carbonsäure [2-(2,7-dimethyl-1H-indol-3-yl)-ethyl]-amid | 343 | 344 | 7,78 |
| 68 | 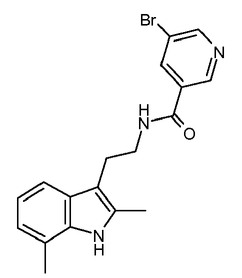 | 5-Brom-N-[2-(2,7-dimethyl-1H-indol-3-yl)-ethyl]-nicotinamid | 372 | 373 | 9,05 |
| 69 | 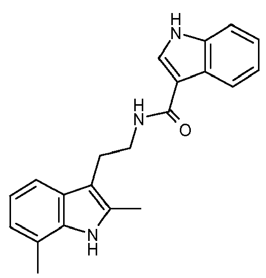 | 1H-Indol-3-carbonsäure [2-(2,7-dimethyl-1H-indol-3-yl)-ethyl]-amid | 331 | 332 | 8,73 |
| 70 | 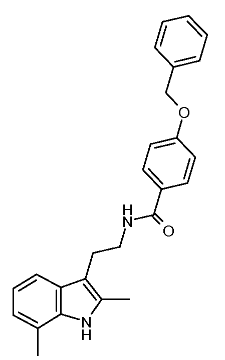 | 4-Benzyloxy-N-[2-(2,7-dimethyl-1H-indol-3-yl)-ethyl]-benzamid | 398 | 399 | 10,36 |
| 71 | 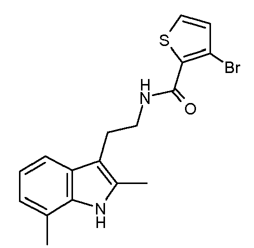 | 3-Brom-thiophen-2-carbonsäure [2-(2,7-dimethyl-1H-indol-3-yl)-ethyl]-amid | 377 | 378 | 9,93 |
| 72 | 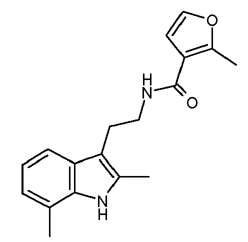 | 2-Methyl-furan-3-carbonsäure [2-(2,7-dimethyl-1H-indol-3-yl)-ethyl]-amid | 296 | 297 | 9,06 |
| 73 | 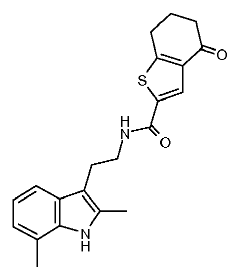 | 4-Oxo-4,5,6,7-tetrahydro-benzo[b]thiophen-2-carbonsäure [2-(2,7-dimethyl-1H-indol-3-yl)-ethyl]-amid | 366 | 367 | 9,03 |
| 74 | 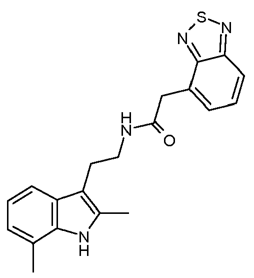 | 2-Benzo[1,2,5]thiadiaz ol-4-yl-N-[2-(2,7-dimethyl-1H-indol-3-yl)-ethyl]-acetamid | 364 | 365 | 9,1 |
| 75 | 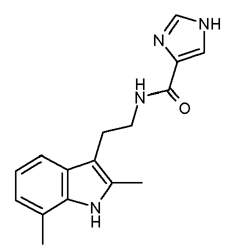 | 1H-Imidazol-4-carbonsäure [2-(2,7-dimethyl-1H-indol-3-yl)-ethyl]-amid | 282 | 283 | 6,1 |
| 76 | 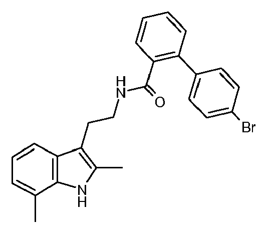 | 4'-Brom-biphenyl-2-carbonsäure [2-(2,7-dimethyl-1H-indol-3-yl)-ethyl]-amid | 447 | 448 | 10,64 |
| 77 | 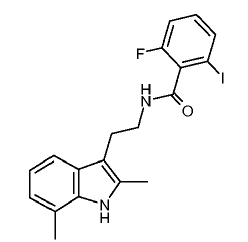 | N-[2-(2,7-Dimethyl-1H-indol-3-yl)-ethyl]-2-fluor-6-iod-benzamid | 436 | 437 | 9,52 |
| 78 | 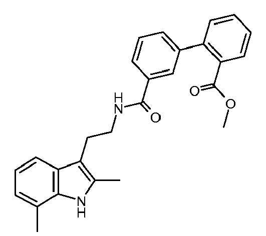 | 3'-[2-(2,7-Dimethyl-1H-indol-3-yl)-ethylcarbamoyl]-biphenyl-2-carbonsäure methylester | 426 | 427 | 10,08 |
| 79 | 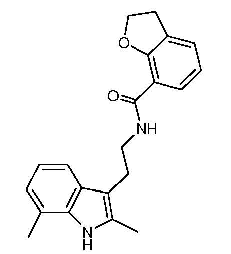 | 2,3-Dihydro-benzofuran-7-carbonsäure [2-(2,7-dimethyl-1H-indol-3-yl)-ethyl]-amid | 334 | 335 | 9,57 |
| 80 | 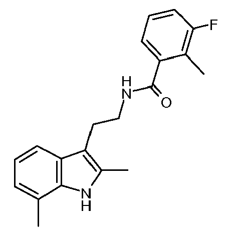 | N-[2-(2,7-Dimethyl-1H-indol-3-yl)-ethyl]-3-fluor-2-methyl-benzamid | 324 | 325 | 9,52 |
| 81 | 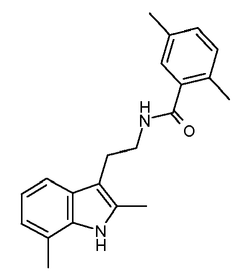 | N-[2-(2,7-Dimethyl-1H-indol-3-yl)-ethyl]-2,5-dimethyl-benzamid | 320 | 321 | 9,73 |
| 82 | 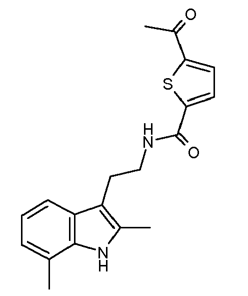 | 5-Acetyl-thiophen-2-carbonsäure [2-(2,7-dimethyl-1H-indol-3-yl)-ethyl]-amid | 340 | 341 | 8,86 |
| 83 | 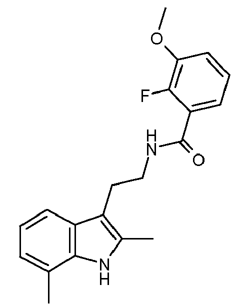 | N-[2-(2,7-Dimethyl-1H-indol-3-yl)-ethyl]-2-fluor-3-methoxy-benzamid | 340 | 341 | 9,33 |
| 84 | 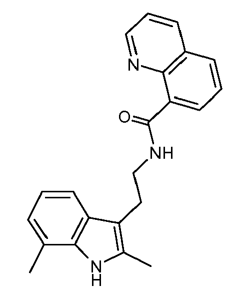 | Chinolin-8-carbonsäure [2-(2,7-dimethyl-1H-indol-3-yl)-ethyl]-amid | 343 | 344 | 8,53 |
| 85 | 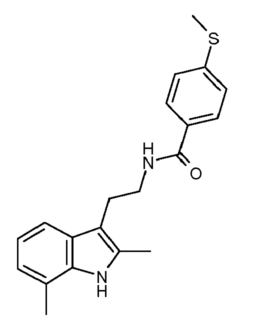 | N-[2-(2,7-Dimethyl-1H-indol-3-yl)-ethyl]-4-methylsulfanyl-benzamid | 338 | 339 | 9,63 |
| 86 | 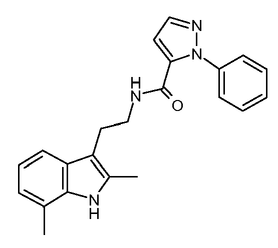 | 2-Phenyl-2H-pyrazol-3-carbonsäure [2-(2,7-dimethyl-1H-indol-3-yl)-ethyl]-amid | 358 | 359 | 9,1 |
| 87 | 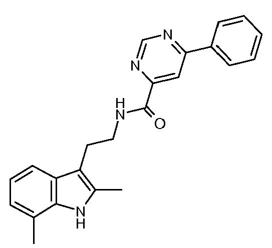 | 6-Phenyl-pyrimidin-4-carbonsäure [2-(2,7-dimethyl-1H-indol-3-yl)-ethyl]-amid | 370 | 371 | 10,66 |
| 88 | 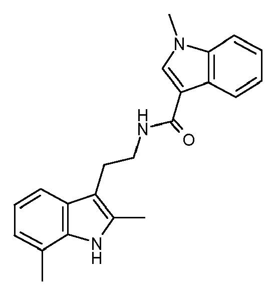 | 1-Methyl-1H-indol-3-carbonsäure [2-(2,7-dimethyl-1H-indol-3-yl)-ethyl]-amid | 345 | 346 | 9,27 |
| 89 | 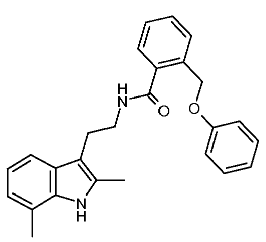 | N-[2-(2,7-Dimethyl-1H-indol-3-yl)-ethyl]-2-phenoxymethyl-benzamid | 398 | 399 | 10,42 |
| 90 | 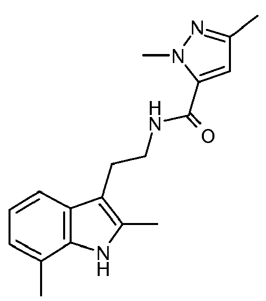 | 2,5-Dimethyl-2H-pyrazol-3-carbonsäure [2-(2,7-dimethyl-1H-indol-3-yl)-ethyl]-amid | 310 | 311 | 8,48 |
| 91 | 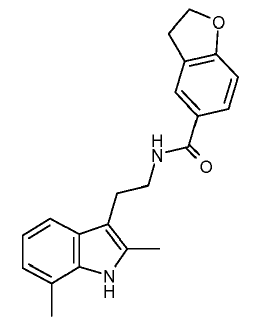 | 2,3-Dihydro-benzofuran-5-carbonsäure [2-(2,7-dimethyl-1H-indol-3-yl)-ethyl]-amid | 334 | 335 | 9,05 |
| 92 | 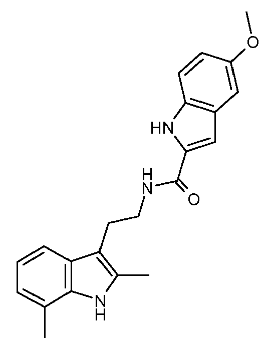 | 5-Methoxy-1H-indol-2-carbonsäure [2-(2,7-dimethyl-1H-indol-3-yl)-ethyl]-amid | 361 | 362 | 9,12 |
| 93 | 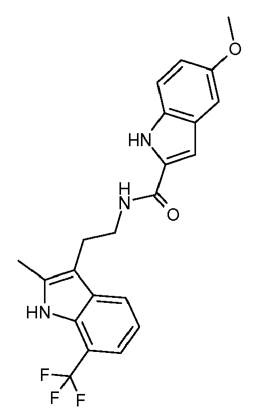 | 5-Methoxy-1H-indol-2-carbonsäure [2-(2-methyl-7-trifluormethyl-1H-indol-3-yl)-ethyl]-amid | 415 | 416 | 9,58 |
| 94 | 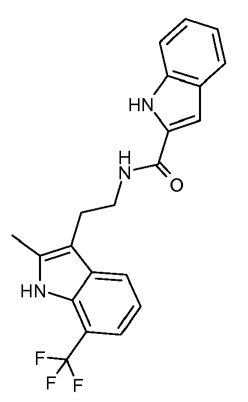 | 1H-Indol-2-carbonsäure [2-(2-methyl-7-trifluormethyl-1H-indol-3-yl)-ethyl]-amid | 385 | 386 | 9,78 |
| 95 | 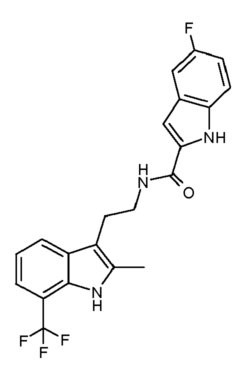 | 5-Fluor-1H-indol-2-carbonsäure [2-(2-methyl-7-trifluormethyl-1H-indol-3-yl)-ethyl]-amid | 403 | 404 | 9,97 |
| 96 | 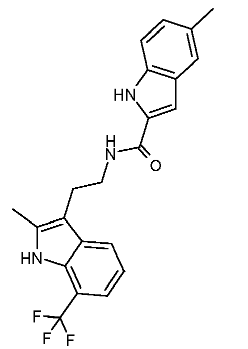 | 5-Methyl-1H-indol-2-carbonsäure [2-(2-methyl-7-trifluormethyl-1H-indol-3-yl)-ethyl]-amid | 399 | 400 | 10,07 |
| 97 | 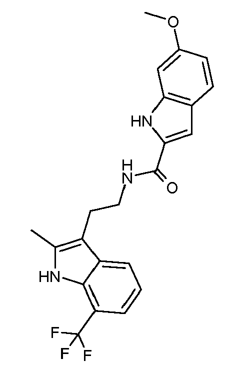 | 6-Methoxy-1H-indol-2-carbonsäure [2-(2-methyl-7-trifluormethyl-1H-indol-3-yl)-ethyl]-amid | 415 | 416 | 9,66 |
| 98 | 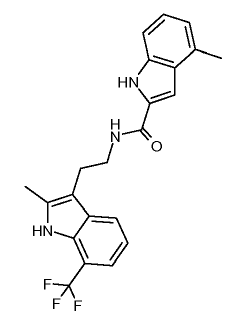 | 4-Methyl-1H-indol-2-carbonsäure [2-(2-methyl-7-trifluormethyl-1H-indol-3-yl)-ethyl]-amid | 399 | 400 | 10,17 |
| 99 | 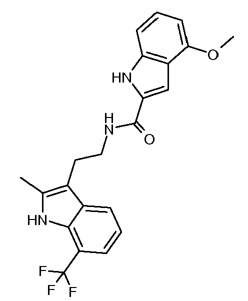 | 4-Methoxy-1H-indol-2-carbonsäure [2-(2-methyl-7-trifluormethyl-1H-indol-3-yl)-ethyl]-amid | 415 | 416 | 9,64 |
| 100 | 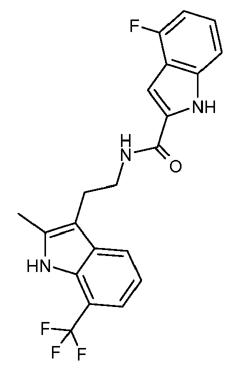 | 4-Fluor-1H-indol-2-carbonsäure [2-(2-methyl-7-trifluormethyl-1H-indol-3-yl)-ethyl]-amid | 403 | 404 | 9,98 |
| 101 | 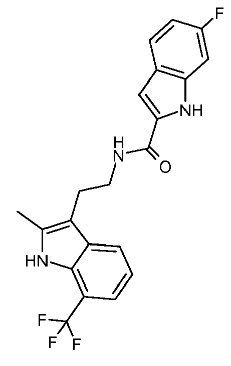 | 6-Fluor-1H-indol-2-carbonsäure [2-(2-methyl-7-trifluormethyl-1H-indol-3-yl)-ethyl]-amid | 403 | 404 | 9,89 |
| 102 | 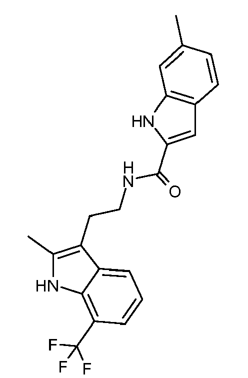 | 6-Methyl-1H-indol-2-carbonsäure [2-(2-methyl-7-trifluormethyl-1H-indol-3-yl)-ethyl]-amid | 399 | 400 | 10,05 |
| 103 | 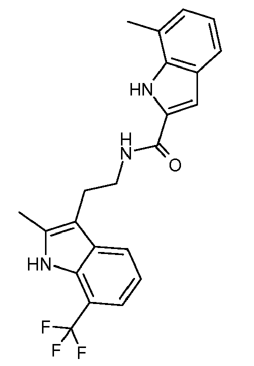 | 7-Methyl-1H-indol-2-carbonsäure [2-(2-methyl-7-trifluormethyl-1H-indol-3-yl)-ethyl]-amid | 399 | 400 | 9,61 |
| 104 | 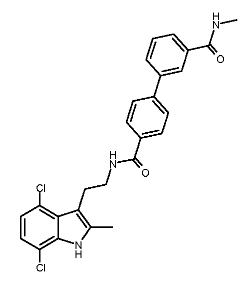 | Biphenyl-3,4'-dicarbonsäure 4'-{[2-(4,7-dichlor-2-methyl-1H-indol-3-yl)-ethyl]-amid} 3-methylamid | 480 | 481 | 3.99 |

### Biologische Beispiele:

### 1. Nachweis des Antagonismus des humanen Prostaglandin E₂ (Subtyp EP₂) Rezeptorsignals

### 1. 1 Nachweisprinzip

Die Bindung von PGE₂ an den EP₂-Subtyp des humanen PGE₂-Rezeptors induziert die Aktivierung membranständiger Adenylatzyklasen und führt zur Bildung von cAMP. In Gegenwart des Phosphodiesteraseinhibitors IBMX wird das durch diese Stimulation akkumulierte und mittels Zell-Lyse freigesetzte cAMP in einem kompetitiven Nachweisverfahren eingesetzt. In diesem Test konkurriert das cAMP im Lysat mit cAMP-XL665 um die Bindung eines Eu-Kryptat markierten Anti-cAMP Antikörpers.
Dabei entsteht in Abwesenheit von zellulärem cAMP ein maximales Signal, welches auf der Kopplung dieses Antikörpers an das cAMP-XL665 Molekül beruht. Dadurch entsteht nach Anregung bei 337 nm ein auf FRET (fluorescence resonance energy transfer) basierendes, langanhaltendes Emissionssignal bei 665 nm (und bei 620 nm). Beide Signale werden in einem geeigneten Messgerät zeitlich versetzt, d.h. nach Abklingen der Hintergrundsfluoreszenz gemessen. Jegliche Erhöhung des durch Prostglandin-E₂-Gabe bedingten niedrigen FRET-Signals (gemessen als Well-Ratio-Veränderung = Emission₆₆₅ₙₘ/Emission₆₂₀ₙₘ * 10000) zeigt die Wirkung von Antagonisten.

### 1.2. Nachweisverfahren

### 1.2.1. Test auf Antagonismus (Angaben pro Vertiefung einer 384-Loch-Platte):

Die in einer Testplatte und 30 % DMSO vorgelegten Substanzlösungen (0.75 µl) wurden in 16 µl einer KRSB+IBMX-Stimulationslösung gelöst (1 X Krebs-Ringer Bicarbonate Buffer; Sigma-Aldrich # K-4002; inklusive 750 µM 3-Isobutyl-1-methylxanthine Sigma-Aldrich # I-7018), anschließend wurden 15 µl davon in eine medienfreie Zellkulturplatte überführt, die kurz zuvor mit KRSB gewaschen worden war.
Nach einer 30-minütigen Vorinkubation bei Raumtemperatur (RT) wurden 5 µl einer 4xPGE₂ Lösung (11 nM) zugegeben und in Gegenwart des Agonisten für weitere 60 min bei RT inkubiert (Volumen: ∼ 20 µl), bevor die Reaktion anschließend durch Zugabe von 5 µl Lysispuffer gestoppt wurde und für weitere 20 min bei RT inkubiert wurde (Volumen: ∼ 25 µl). Das Zell-Lysat wurde anschließend in eine Messplatte überführt und entsprechend den Herstellerangaben (cyclic AMP kit Cisbio International # 62AMPPEC) gemessen.

### 1.2.2. Test auf Agonismus (Angaben pro Vertiefung einer 384-Loch-Platte):

Die in einer in einer Testplatte und 30 % DMSO vorgelegten Substanzlösungen (0.75 µl) wurden in 16 µl einer KRSB+IBMX-Stimulationslösung gelöst (1 X Krebs-Ringer Bicarbonate Buffer; Sigma-Aldrich # K-4002; inklusive 750 µM 3-Isobutyl-1-methylxanthine Sigma-Aldrich # I-7018), anschließend wurden 15 µl davon in eine medienfreie Zellkulturplatte überführt, die kurz zuvor mit KRSB gewaschen worden war.
Nach einer 60-minütigen Inkubation bei Raumtemperatur (RT; Volumen: ∼ 15 µl) wurde die Reaktion anschließend durch Zugabe von 5 µl Lysisbuffer gestoppt und für weitere 20 min bei RT inkubiert (Volumen: ∼ 20 µl). Das Zell-Lysat wurde anschließend in eine Messplatte überführt und entsprechend den Herstellerangaben (cyclic AMP kit Cisbio International # 62AMPPEC) gemessen.

### 2. Der EP₂-Subtyp des PGE₂-Rezeptors und die prä-ovulatorische Kumulus Expansion

### 2.1. Hintergrund:

Im prä-ovulatorischen antralen Follikel ist die Eizelle von Kumulus Zellen umgeben, die einen dichten Zellkranz um die Eizelle bilden. Nach dem LH-Peak (Lutenisierendes Hormon) wird eine Reihe von Prozessen aktiviert, die eine starke morphologische Veränderung dieses Zellkranzes aus Kumulus Zellen zur Folge hat. Hierbei bilden die Kumulus Zellen eine extrazelluläre Matrix, die zur sogenannten Kumulus Expansion führt (Vanderhyden et al. Dev Biol. 1990 Aug;140(2):307-317). Diese Kumulus Expansion ist ein wichtiger Bestandteil des ovulatorischen Prozesses und der nachfolgenden Möglichkeit zur Fertilisation. Bei der Kumulus Expansion sind Prostaglandine und hier das Prostaglandin E₂, dessen Synthese durch den LH-Peak induziert wird, von entscheidender Bedeutung. Prostanoid EP₂ Knock-out Mäuse (Hizaki et al. Proc Natl Acad Sci U S A. 1999 Aug 31 ;96(18):10501-6.) zeigen eine deutlich verminderte Kumulus Expansion und starke Subfertilität, was die Bedeutung des Prostanoid EP₂-Rezeptors für diesen Prozess demonstriert.

### 2.2. Kumulus Expansions Test in vitro

In immaturen weiblichen Mäusen (Stamm : CD1 (ICR) von Charles River) wurde in einem Alter von 14 - 18 Tagen die Follikulogenese durch eine einmalige Gabe (intraperitoneal) von 10 I.E. PMSG (Pregnant Mare Serum Gonadotropine; Sigma G-4877, Lot 68H0909) induziert. 47 - 50 Stunden nach der Injektion wurden die Ovarien entnommen und die Kumulus-Eizell Komplexe entnommen. Der Kumulus Komplex ist in diesem Stadium noch nicht expandiert.
Die Kumulus-Eizell Komplexe wurden nun für 20-24 Stunden mit Prostaglandin E₂ (PGE₂) (0,3 µM), Vehikel Kontrolle (Ethanol) oder Testsubstanzen inkubiert. Medium: alpha- MEM Medium mit 0,1 mM IBMX, Pyruvate (0,23 mM) Glutamine (2 mM), Pen/Strep 100 IU/ml Pen. und 100 µg/ml Strep.) und HSA (8 mg/ml)). Danach wurde die Kumulus Expansion durch die Einteilung in vier Stadien (nach Vanderhyden et al. Dev Biol. 1990 Aug;140(2):307-317) festgestellt.

**Tabelle 1: Beispiel für die biologische Wirksamkeit der erfindungsgemäßen Verbindungen (gemessen mittels cAMP Antagonismustest):**

| Substanz gemäß Beispiel | Antagonismus [IC₅₀, µM] |
|---|---|
| 1 | 0.32 |
| 5 | 1.9 |
| 7 | 0.047 |
| 8 | 0.12 |
| 17 | 0.72 |
| 30 | 0.67 |

## Patentansprüche

1. Verbindungen der allgemeinen Formel I, wobei 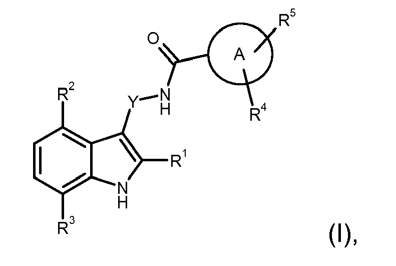
A ein C₆-C₁₂-Aryl- oder C₅-C₁₂-Heteroarylrest, der gegebenenfalls ein- oder mehrfach mit R⁴ und/oder R⁵ substituiert sein kann,
R¹ ein C₁-C₆-Alkylrest, der gegebenenfalls substituiert sein kann,
R² ein Wasserstoff, Halogen, Cyano, ein -S(O)_{q}-CH₃, wobei q für 0 - 2 steht, ein C₁-C₄-Alkoxyrest oder C₁-C₆-Alkyl, wobei dieser Rest beliebig substituiert sein kann,
R³ ein Wasserstoff, ein C₁-C₆-Alkylrest, Cyano, Chlor oder Brom ,
R⁴ ein Wasserstoff, Halogen, Amino, eine -S(O)ₚ-C₁-C₆-Alkylgruppe, wobei p 0 - 2 bedeutet,
ein C₁-C₆-Acyl-, NH-CO-NH₂, -O-CO-NH(C₁-C₆-Alkyl), -O-CO-N(C₁-C₆-Alkyl)₂ oder NH-CO-C₁-C₆-Alkylrest,
ein C₁-C₆-Alkyl, welches gegebenenfalls ein- oder mehrfach, gleich oder verschieden mit C₁-C₆-Acyl, C₁-C₆-Alkoxy, Hydroxy, Cyano, CO₂-(C₁-C₆-Alkyl), N-(C₁-C₆-Alkyl)₂, C₅-C₁₂-Heteroaryl, COOH,
CO-NH₂, CO-NH(C₁-C₆-Alkyl) oder mit CO-N(C₁-C₆-Alkyl)₂ substituiert sein kann,
ein C₁-C₆-Alkoxy, welches gegebenenfalls ein- oder mehrfach, gleich oder verschieden mit Hydroxy, Cyano, CO₂-(C₁-C₆-Alkyl), N-(C₁-C₆-Alkyl)₂, NH-C₃-C₆-Cycloalkyl, COOH, CO-NH₂, CO-NH(C₁-C₆-Alkyl) oder mit CO-N(C₁-C₆-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₆-Alkyl), CO-NH(C₅-C₁₂-Heteroaryl), NH-(C₁-C₆-Alkyl), N-(C₁-C₆-Alkyl)₂,
ein C₆-C₁₂-Aryl, welches gegebenenfalls ein- oder mehrfach, gleich oder verschieden mit Halogen, mit C₁-C₆-Alkyl, C₃-C₆-Cycloalkyl, C₁-C₆-Acyl, C₁-C₆-Alkoxy, C₆-C₁₂-Aryl, C₅-C₁₂-Heteroaryl, Hydroxy, CH₂-OH, Cyano, CH₂-CN, Amino, CO₂-(C₁-C₆-Alkyl), N-(C₁-C₆-Alkyl)₂, NHSO₂CH₃, SO₂NH₂, SO₂NH(C₁-C₆-Alkyl), SO₂N(C₁-C₆-Alkyl)₂, COOH, CO-NH₂, CO-NH(C₁-C₆-Alkyl), CO-N(C₁-C₆-Alkyl)₂, CO-NH(C₅-C₁₂-Heteroaryl), NH-CO(C₁-C₆-Alkyl), CH₂-NH-CO(C₁-C₆-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₆-Alkyl substituiert sein kann,
unter der Voraussetzung, dass R² Cyano bdeutet oder R¹ und/oder R² gleich oder verschieden einen C₁-C₆-Akylrest bedeuten, wobei mindestens einer der Reste mindestens einfach substituiert ist oder
unter der Voraussetzung, dass R⁵ ein -S(O)ₚ-C₁-C₆-Alkyl bedeutet, wobei p für 0 - 2 steht, ein C₁-C₆-Acyl-, -O-CO-NH(C₁-C₆-Alkyl), -O-CO-N(C₁-C₆-Alkyl)₂, C₆-C₁₂-Aryloxy, C₅-C₁₆-Heteroaryloxy, Hydroxy, Cyano oder N-(C₁-C₆-Alkyl)₂ ist,
ein monocyclisches C₅-C₇-Heteroaryl, welches mindestens ein- oder mehrfach, gleich oder verschieden mit Halogen, CF₃, C₁-C₆-Acyl, C₁-C₆-Alkoxy, Hydroxy, CH₂-OH, Cyano, CO₂-(C₁-C₆-Alkyl), N-(C₁-C₆-Alkyl)₂, COOH, CO-NH₂, CO-NH(C₁-C₆-Alkyl) oder CO-N(C₁-C₆-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₆-Alkyl, C₁-C₆-Acyl, C₁-C₆-Alkoxy, Hydroxy, Cyano, CO₂-(C₁-C₆-Alkyl), N-(C₁-C₆-Alkyl)₂, COOH, CO-NH₂, CONH(C₁-C₆-Alkyl) oder CO-N(C₁-C₆-Alkyl)₂ substituiert sein kann oder
ein C₃-C₆-Cycloalkyl, welches gegebenenfalls ein- oder mehrfach, gleich oder verschieden mit Halogen, mit C₁-C₆-Alkyl, Hydroxy, Cyano, CO₂-(C₁-C₆-Alkyl), C₁-C₆-Acyl, N-(C₁-C₆-Alkyl)₂, COOH, CO-NH₂, CO-NH(C₁-C₆-Alkyl), CO-N(C₁-C₆-Alkyl)₂ oder C₁-C₆-Alkoxy substituiert sein kann,
R⁵ ein Wasserstoff, Halogen, Amino, -S(O)ₚ-C₁-C₆-Alkyl, wobei p 0 - 2 bedeutet,
eine C₁-C₆-Acyl-, NH-CO-NH₂-, NH-CO-C₁-C₆-Alkyl-, -O-CO-NH(C₁-C₆-Alkyl)-, -O-CO-N(C₁-C₆-Alkyl)₂ oder C₁-C₆-Alkylgruppe, die gegebenenfalls ein- oder mehrfach, gleich oder verschieden mit C₁-C₆-Acyl, C₁-C₆-Alkoxy, Hydroxy, Cyano, CO₂-(C₁-C₆-Alkyl), N-(C₁-C₆-Alkyl)₂, C₅-C₁₂-Heteroaryl, COOH, CO-NH₂, CO-NH(C₁-C₆-Alkyl) oder mit CO-N(C₁-C₆-Alkyl)₂ substituiert sein kann,
ein C₁-C₆-Alkoxy, welches gegebenenfalls ein- oder mehrfach, gleich oder verschieden mit Hydroxy, Cyano, CO₂-(C₁-C₆-Alkyl), N-(C₁-C₆-Alkyl)₂, NH-C₃-C₆-Cycloalkyl, COOH, CO-NH₂, CO-NH(C₁-C₆-Alkyl) oder mit CO-N(C₁-C₆-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₆-Alkyl), CO-NH(C₅-C₁₂-Heteroaryl), NH-(C₁-C₆-Alkyl), N-(C₁-C₆-Alkyl)₂,
ein C₆-C₁₂-Aryl, welches gegebenenfalls ein- oder mehrfach, gleich oder verschieden mit Halogen, mit C₁-C₆-Alkyl, C₃-C₆-Cycloalkyl, C₁-C₆-Acyl, C₁-C₆-Alkoxy, C₆-C₁₂-Aryl, C₅-C₁₂-Heteroaryl, Hydroxy, CH₂-OH, Cyano, CH₂-CN, Amino, CO₂-(C₁-C₆-Alkyl), N-(C₁-C₆-Alkyl)₂, NHSO₂CH₃, SO₂NH₂, SO₂NH(C₁-C₆-Alkyl), SO₂N(C₁-C₆-Alkyl)₂, COOH, CO-NH₂, CO-NH(C₁-C₆-Alkyl), CO-N(C₁-C₆-Alkyl)₂, CO-NH(C₅-C₁₂-Heteroaryl), NH-CO(C₁-C₆-Alkyl), CH₂-NH-CO(C₁- C₆-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₆-Alkyl substituiert sein kann, wenn R² gleich Cyano ist oder
unter der Voraussetzung, dass R¹ und/oder R² gleich oder verschieden ein C₁-C₆-Akylrest bedeuten, wobei mindestens einer der Reste mindestens einfach substituiert ist,
ein monocyclisches C₅-C₇-Heteroaryl, welches mindestens ein- oder mehrfach, gleich oder verschieden mit Halogen, CF₃, C₁-C₆-Acyl, C₁-C₆-Alkoxy, Hydroxy, CH₂-OH, Cyano, CO₂-(C₁-C₆-Alkyl), N-(C₁-C₆-Alkyl)₂, COOH, CO-NH₂, CO-NH(C₁-C₆-Alkyl) oder CON(C₁-C₆-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₆-Alkyl, C₁-C₆-Acyl, C₁-C₆-Alkoxy, Hydroxy, Cyano, CO₂-(C₁-C₆-Alkyl), N-(C₁-C₆-Alkyl)₂, COOH, CO-NH₂, CONH(C₁-C₆-Alkyl) oder CO-N(C₁-C₆-Alkyl)₂ substituiert sein kann oder
ein C₃-C₆-Cycloalkyl, welches gegebenenfalls ein- oder mehrfach, gleich oder verschieden mit Halogen, mit C₁-C₆-Alkyl, Hydroxy, Cyano, CO₂-(C₁-C₆-Alkyl), C₁-C₆-Acyl, N-(C₁-C₆-Alkyl)₂, COOH, CO-NH₂, CO-NH(C₁-C₆-Alkyl), CO-N(C₁-C₆-Alkyl)₂ oder C₁-C₆-Alkoxy substituiert sein kann,
R⁴ und R⁵ entweder in ortho-, meta- oder meta,para-Stellung zueinander stehen und gemeinsam die Bedeutung haben -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
Y eine -(CH₂)ₙ-Gruppe, wobei n für 1 - 3 steht,
bedeuten, sowie deren Isomere, Diastereomere, Enantiomere und Salze bzw. Cyclodextrinclathrate, wobei die folgenden Verbindungen ausgeschlossen sind:
4-Chlor-1,3-dimethyl-N-[2-(2-methyl-1H-indol-3-yl)ethyl]-1H-pyrazol-5-carbonsäureamid
N-[2-(2-Methyl-1H-indol-3-yl)ethyl]-6-chinoxalincarbonsäureamid
3,5-Dimethyl-N-[2-(2-methyl-1H-indol-3-yl)ethyl]-4-isoxazolcarbonsäureamid
5,7-Dimethyl-N-[2-(2-methyl-1H-indol-3-yl)ethyl]-pyrazolo[1,5-a]pyrimidin-2-carbonsäureamid
N-[2-(2-Methyl-1H-indol-3-yl)ethyl]-2-methylbenzamid
N-[2-(2-Methyl-1H-indol-3-yl)ethyl]-2-(difluoromethyl)benzamid
3-Iod-1-methyl-N-[2-(2-methyl-1H-indol-3-yl)ethyl]-1H-pyrol-2-carbonsäureamid
1-Methyl-N-[2-(2-methyl-1H-indol-3-yl)ethyl]-4-(trifluormethyl)-1H-pyrol-3-carbonsäureamid
2-lod-N-[2-(2-methyl-1H-indol-3-yl)ethyl]-3-thiophencarbonsäureamid
3-(Difluormethyl)-1-methyl-N-[2-(2-methyl-1H-indol-3-yl)ethyl]-1H-pyrazol-4-carbonsäureamid
3-(Trifluormethyl)-1-methyl-N-[2-(2-methyl-1H-indol-3-yl)ethyl]-1H-pyrazol-4-carbonsäureamid
5-Fluor-1,3-dimethyl-N-[2-(2-methyl-1H-indol-3-yl)ethyl]-1H-pyrazol-4-carbonsäureamid
4-(Difluormethyl)-2-methyl-N-[2-(2-methyl-1H-indol-3-yl)ethyl]-5-thiazolcarbonsäureamid
4-(Trifluormethyl)-2-methyl-N-[2-(2-methyl-1H-indol-3-yl)ethyl]-5-thiazolcarbonsäureamid
2-lod-N-[2-(2-methyl-1H-indol-3-yl)ethyl]-3-furancarbonsäureamid
2,5-Dimethyl-N-[2-(2-methyl-1H-indol-3-yl)ethyl]-3-furancarbonsäureamid
5-Methyl-N-[2-(2-methyl-1H-indol-3-yl)ethyl]-2-(trifluormethyl)-3-furancarbonsäureamid
2-Methyl-N-[2-(2-methyl-1H-indol-3-yl)ethyl]-3-furancarbonsäureamid
3-Methyl-N-[2-(2-methyl-1H-indol-3-yl)ethyl]-2-thiophencarbonsäureamid
3-lod-N-[2-(2-methyl-1H-indol-3-yl)ethyl]-2-thiophencarbonsäureamid
3-Brom-N-[2-(2-methyl-1H-indol-3-yl)ethyl]-2-thiophencarbonsäureamid
4-Methoxy-N-[2-(2-methyl-1H-indol-3-yl)ethyl]-benzamid
N-[2-(7-Chlor-2-methyl-1H-indol-3-yl)ethyl]-2-methyl-7-benzoxazolcarbonsäureamid
4-(1,1-Dimethylethyl)-N-[2-(2-methyl-1H-indol-3-yl)ethyl]-benzamid
N-[2-(2,7-Dimethyl-1H-indol-3-yl)ethyl]-3,4-dimethoxy-benzamid
2-Ethoxy-N-[2-(2-methyl-1H-indol-3-yl)ethyl]-benzamid
2-(1-Methylethoxy)-N-[2-(2-methyl-1H-indol-3-yl)ethyl]-benzamid
2-Chlor-4,5-difluor-N-[2-(2-methyl-1H-indol-3-yl)ethyl]-benzamid
4-Brom-N-[2-(4-fluoro-2,7-dimethyl-1H-indol-3-yl)ethyl]-benzamid
2,6-Difluor-N-[2-(2-methyl-1H-indol-3-yl)ethyl]-benzamid
3,4-Difluor-N-[2-(2-methyl-1H-indol-3-yl)ethyl]-benzamid
N-[2-(2-Methyl-1H-indol-3-yl)ethyl]-4-(trifluormethyl)-benzamid
N-[2-(2-Methyl-1H-indol-3-yl)ethyl]-4-methyl-benzamid
2,4-Dichlor-N-[2-(2-methyl-1H-indol-3-yl)ethyl]-benzamid
2-Brom-N-[2-(2-Methyl-1H-indol-3-yl)ethyl]-benzamid
4-Fluor-N-[2-(2-methyl-1H-indol-3-yl)ethyl]-benzamid
N-[2-(4-Fluor-2,7-dimethyl-1H-indol-3-yl)ethyl]-2-methyl-benzamid
N-[2-(4-Fluor-2,7-dimethyl-1H-indol-3-yl)ethyl]-2-methoxy-benzamid
3,4-Dimethoxy-N-[2-(4-Fluor-2,7-dimethyl-1H-indol-3-yl)ethyl]-benzamid
4-[[[2-(4-Fluor-2,7-dimethyl-1H-indol-3-yl)ethyl]amino]carbonyl]-benzoesäure methyl ester
N-[2-(4-Fluor-2,7-dimethyl-1H-indol-3-yl)ethyl]-1-methyl-1H-pyrazol-5-carbonsäureamid
N-[2-(7-Ethyl-2-methyl-1H-indol-3-yl)ethyl]-2-methoxy-benzamid
4-Chlor-N-[2-(2-methyl-1H-indol-3-yl)ethyl]-benzamid
2,6-Dichlor-N-[2-(2-methyl-1H-indol-3-yl)ethyl]-benzamid
5-Chlor-2-methoxy-N-[2-(2-methyl-1H-indol-3-yl)ethyl]-benzamid
3,4-Dichlor-N-[2-(2-methyl-1H-indol-3-yl)ethyl]-benzamid
2-Fluor-N-[2-(2-methyl-1H-indol-3-yl)ethyl]-benzamid
4-Chlor-1-methyl-N-[2-(2-methyl-1H-indol-3-yl)ethyl]-1H-pyrazol-5-carbonsäureamid
4-[[[2-(2-Methyl-1H-indol-3-yl)ethyl]amino]carbonyl]-benzoesäure methylester
N-[2-(2-Methyl-1H-indol-3-yl)ethyl]-2-thiophencarbonsäureamid
3,4,5-Trimethoxy-N-[2-(2-methyl-1H-indol-3-yl)ethyl]-benzamid
3-Methoxy-N-[2-(2-methyl-1H-indol-3-yl)ethyl]-benzamid
N-[2-(2-methyl-1H-indol-3-yl)ethyl]-4-pyridincarbonsäureamid
N-[2-(2-methyl-1H-indol-3-yl)ethyl]-3-pyridincarbonsäureamid

2. Verbindungen gemäß Anspruch 1, wobei
A ein C₆-C₁₂-Aryl- oder C₅-C₁₂-Heteroarylrest, der gegebenenfalls ein- oder mehrfach mit R⁴ und/oder R⁵ substituiert sein kann,
R¹ ein C₁-C₄-Alkylrest, der gegebenenfalls substituiert sein kann,
R² ein Wasserstoff, Halogen, ein C₁-C₄-Alkoxyrest oder C₁-C₆-Alkyl, wobei dieser Rest beliebig substituiert sein kann,
R³ ein Wasserstoff, ein C₁-C₆-Alkylrest, Cyano, Chlor oder Brom und die Reste R⁴, R⁵, R⁶, R⁷ und Y die in Anspruch 1 angegebenen Bedeutung haben,
bedeuten, sowie deren Isomere, Diastereomere, Enantiomere und Salze bzw. Cyclodextrinclathrate.

3. Verbindungen gemäß Anspruch 1-2, wobei
A ein C₆-C₁₂-Aryl- oder C₅-C₁₂- oder Heteroarylrest, der gegebenenfalls ein- oder mehrfach mit R⁴ und/oder R⁵ substituiert sein kann,
R¹ ein C₁-C₄-Alkylrest,
R² ein Wasserstoff, Halogen oder C₁-C₄-Alkyl, wobei dieser Rest beliebig substituiert sein kann,
R³ ein Wasserstoff, ein C₁-C₄-Alkylrest, Cyano, Chlor oder Brom,
R⁴ ein Wasserstoff, Halogen, Amino, eine -S(O)ₚ-C₁-C₄-Alkylgruppe, wobei p 0 - 2 bedeutet,
ein C₁-C₄-Acyl-, NH-CO-NH₂, -O-CO-NH(C₁-C₄-Alkyl), -O-CO-N(C₁-C₄-Alkyl)₂ oder NH-CO-C₁-C₄-Alkylrest,
ein C₁-C₄-Alkyl, welches gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann, ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann,
unter der Voraussetzung, dass R² Cyano oder R¹ und/oder R² gleich oder verschieden einen C₁-C₄-Akylrest bedeuten, wobei mindestens einer der Reste mindestens einfach substituiert ist oder
unter der Voraussetzung, dass R⁵ ein -S(O)ₚ-C₁-C₄-Alkyl bedeutet, wobei p für 0 - 2 steht, ein C₁-C₆-Acyl-, -O-CO-NH(C₁-C₄-Alkyl), -O-CO-N(C₁-C₄-Alkyl)₂, C₆-C₁₂-Aryloxy, C₅-C₁₆-Heteroaryloxy, Hydroxy, Cyano oder N-(C₁-C₄-Alkyl)₂ bedeutet,
ein monocyclisches C₅-C₇-Heteroaryl, welches mindestens ein- oder mehrfach, gleich oder verschieden mit Halogen, CF₃, C₁-C₄- Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann, ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
R⁵ ein Wasserstoff, Halogen, Amino, -S(O)ₚ-C₁-C₄-Alkyl, wobei p 0 - 2 bedeutet,
eine C₁-C₄-Acyl-, NH-CO-NH₂-, NH-CO-C₁-C₄-Alkyl-, -O-CO-NH(C₁-C₄-Alkyl)-, -O-CO-N(C₁-C₄-Alkyl)₂ oder C₁-C₄-Alkylgruppe, die gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder ein -S(O)ᵣ-CH₃, wobei r für 0 - 2 steht, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann, unter derVoraussetzung, dass R² Cyano bedeutet oder wenn R¹ und/oder R² gleich oder verschieden ein C₁-C₄-Akylrest ist, wobei mindestens einer der Reste mindestens einfach substituiert ist,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
R⁴ und R⁵ entweder in ortho-, meta- oder meta,para-Stellung zueinander stehen und gemeinsam die Bedeutung haben -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
Y eine -(CH₂)ₙ-Gruppe, wobei n für 1 - 3 steht,
bedeuten, sowie deren Isomere, Diastereomere, Enantiomere und Salze bzw. Cyclodextrinclathrate.

4. Verbindungen gemäß Anspruch 1-3, wobei
A einen Phenyl-, Naphthyl- oder Heteroarylrest, der gegebenenfalls ein- oder zweifach mit R⁴ und/oder R⁵ substituiert sein kann,
R¹ ein C₁-C₄-Alkylrest,
R² ein Wasserstoff, Halogen oder C₁-C₄-Alkyl, wobei dieser Rest beliebig substituiert sein kann,
R³ ein Wasserstoff, ein C₁-C₄-Alkylrest, Cyano, Chlor oder Brom,
R⁴ ein Wasserstoff, Halogen, Amino, eine -S(O)ₚ-C₁-C₄-Alkylgruppe, wobei p 0 - 2 bedeutet,
ein C₁-C₄-Acyl-, NH-CO-NH₂, -O-CO-NH(C₁-C₄-Alkyl), -O-CO-N(C₁-C₄-Alkyl)₂ oder NH-CO-C₁-C₄-Alkylrest,
ein C₁-C₄-Alkyl, welches gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann, ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann, ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann,
unter der Voraussetzung, dass R² Cyano oder R¹ und/oder R² gleich oder verschieden einen C₁-C₄-Akylrest bedeuten, wobei mindestens einer der Reste mindestens einfach substituiert ist oder
unter der Voraussetzung, dass R⁵ ein -S(O)ₚ-C₁-C₄-Alkyl bedeutet, wobei p für 0 - 2 steht, ein C₁-C₆-Acyl-, -O-CO-NH(C₁-C₄-Alkyl), -O-CO-N(C₁-C₄-Alkyl)₂, C₆-C₁₂-Aryloxy, C₅-C₁₆-Heteroaryloxy, Hydroxy, Cyano oder N-(C₁-C₄-Alkyl)₂ bedeutet,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
R⁵ ein Wasserstoff, Halogen, Amino, -S(O)ₚ-C₁-C₄-Alkyl, wobei p 0 - 2 bedeutet,
eine C₁-C₄-Acyl-, NH-CO-NH₂-, NH-CO-C₁-C₄-Alkyl-, -O-CO-NH(C₁-C₄-Alkyl)-, -O-CO-N(C₁-C₄-Alkyl)₂ oder C₁-C₄-Alkylgruppe, die gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder ein -S(O)ᵣ-CH₃, wobei r für 0 - 2 steht, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann, unter derVoraussetzung, dass R² Cyano bedeutet oder wenn R¹ und/oder R² gleich oder verschieden ein C₁-C₄-Akylrest ist, wobei mindestens einer der Reste mindestens einfach substituiert ist,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
R⁴ und R⁵ entweder in ortho-, meta- oder meta,para-Stellung zueinander stehen und gemeinsam die Bedeutung haben -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
Y eine -(CH₂)₂-Gruppe,
bedeuten, sowie deren Isomere, Diastereomere, Enantiomere und Salze bzw. Cyclodextrinclathrate,

5. Verbindungen gemäß Anspruch 1-4, wobei,
A einen Phenyl-, Naphthyl- oder C₅-C₁₂-Heteroarylrest, der gegebenenfalls ein- oder zweifach mit R⁴ und/oder R⁵ substituiert sein kann,
R¹ ein C₁-C₄-Alkylrest,
R² ein Wasserstoff, Fluor, Chlor, Brom oder C₁-C₄-Alkyl,
R³ ein Wasserstoff, Methyl, Ethyl, Trifluormethyl, Cyano, Chlor oder Brom,
R⁴ ein Wasserstoff, Halogen, Amino, eine -S(O)ₚ-C₁-C₄-Alkylgruppe, wobei p 0 - 2 bedeutet,
ein C₁-C₄-Acyl-, NH-CO-NH₂, -O-CO-NH(C₁-C₄-Alkyl), -O-CO-N(C₁-C₄-Alkyl)₂ oder NH-CO-C₁-C₄-Alkylrest,
ein C₁-C₄-Alkyl, welches gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann, ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂, ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann,
unter der Voraussetzung, dass R² Cyano oder R¹ und/oder R² gleich oder verschieden einen C₁-C₄-Akylrest bedeuten, wobei mindestens einer der Reste mindestens einfach substituiert ist oder
unter der Voraussetzung, dass R⁵ ein -S(O)ₚ-C₁-C₄-Alkyl bedeutet, wobei p für 0 - 2 steht, ein C₁-C₆-Acyl-, -O-CO-NH(C₁-C₄-Alkyl), -O-CO-N(C₁-C₄-Alkyl)₂, C₆-C₁₂-Aryloxy, C₅-C₁₆-Heteroaryloxy, Hydroxy, Cyano oder N-(C₁-C₄-Alkyl)₂ bedeutet,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy,
Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
R⁵ ein Wasserstoff, Halogen, Amino, -S(O)ₚ-C₁-C₄-Alkyl, wobei p 0 - 2 bedeutet,
eine C₁-C₄-Acyl-, NH-CO-NH₂-, NH-CO-C₁-C₄-Alkyl-, -O-CO-NH(C₁-C₄-Alkyl)-, -O-CO-N(C₁-C₄-Alkyl)₂ oder C₁-C₄-Alkylgruppe, die gegebenenfalls ein- oder mehrfach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein- oder mehrfach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein- oder mehrfach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder ein -S(O)ᵣ-CH₃, wobei r für 0 - 2 steht, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein- oder mehrfach, gleich oder verschieden mit C₁-C₄-Alkyl substituiert sein kann, unter derVoraussetzung, dass R² Cyano bedeutet oder wenn R¹ und/oder R² gleich oder verschieden ein C₁-C₄-Akylrest ist, wobei mindestens einer der Reste mindestens einfach substituiert ist,
ein monocyclisches C₅-C₇-Heteroaryl, welches mindestens ein- oder mehrfach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein- oder mehrfach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann oder
ein C₃-C₆-Cycloalkyl, welches gegebenenfalls ein- oder mehrfach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
R⁴ und R⁵ entweder in ortho-, meta- oder meta,para-Stellung zueinander stehen und gemeinsam die Bedeutung haben -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
Y eine -(CH₂)₂-Gruppe,
bedeuten, sowie deren Isomere, Diastereomere, Enantiomere und Salze bzw. Cyclodextrinclathrate.

6. Verbindungen gemäß Anspruch 1-5, wobei
A einen Phenyl-, Naphthyl- oder C₅-C₁₂-Heteroarylrest, der gegebenenfalls ein- oder zweifach mit R⁴ und/oder R⁵ substituiert sein kann,
R¹ ein C₁-C₄-Alkylrest,
R² ein Wasserstoff, Fluor, Chlor, Brom oder C₁-C₄-Alkyl,
R³ ein Wasserstoff, Methyl, Ethyl, Trifluormethyl, Cyano, Chlor oder Brom,
R⁴ ein Wasserstoff, Halogen, Amino, eine -S(O)ₚ-C₁-C₄-Alkylgruppe, wobei p 0 - 2 bedeuten,
ein C₁-C₄-Acyl-, NH-CO-NH₂, -O-CO-NH(C₁-C₄-Alkyl), -O-CO-N(C₁-C₄-Alkyl)₂ oder NH-CO-C₁-C₄-Alkylrest,
ein C₁-C₄-Alkyl, welches gegebenenfalls ein-, zwei- oder dreifach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein-, zwei- oder dreifach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein-, zwei- oder dreifach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r 0 - 2 bedeutet, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein-, zwei- oder dreifach, gleich oder verschieden mit C₁-C₄-Alkyl substituiert sein kann,
unter der Voraussetzung, dass R² Cyano ist oder wenn R¹ und/oder R² gleich oder verschieden einen C₁-C₄-Akylrest bedeuten, wobei mindestens einer der Reste mindestens einfach substituiert ist oder
unter der Voraussetzung, dass R⁵ ein -S(O)ₚ-C₁-C₄-Alkyl bedeutet, wobei p für 0 - 2 steht, ein C₁-C₆-Acyl-, -O-CO-NH(C₁-C₄-Alkyl), -O-CO-N(C₁-C₄-Alkyl)₂, C₆-C₁₂-Aryloxy, C₅-C₁₆-Heteroaryloxy, Hydroxy, Cyano oder N-(C₁-C₄-Alkyl)₂ ist,
ein monocyclisches C₅-C₇-Heteroaryl, welches mindestens ein-, zwei- oder dreifach, gleich oder verschieden mit Halogen, CF₃, C₁-C₄-Acyl, C₁-C₄-Alkoxy, Hydroxy, CH₂-OH, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein-, zwei- oder dreifach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann oder
ein C₃-C₆-Cycloalkyl, welches gegebenenfalls ein-, zwei- oder dreifach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
R⁵ ein Wasserstoff, Halogen, Amino, -S(O)ₚ-C₁-C₄-Alkyl, wobei p 0 - 2 bedeutet,
eine C₁-C₄-Acyl-, NH-CO-NH₂-, NH-CO-C₁-C₄-Alkyl-, -O-CO-NH(C₁-C₄-Alkyl)-, -O-CO-N(C₁-C₄-Alkyl)₂ oder C₁-C₄-Alkylgruppe, die gegebenenfalls ein-, zwei- oder dreifach, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls ein-, zwei- oder dreifach, gleich oder verschieden mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-C₆-C₁₂-Aryl, welches gegebenenfalls mit Hydroxy, Cyano, COOH oder CO-NH₂ substituiert sein kann,
ein CH₂O-C₆-C₁₂-Aryl, welches gegebenenfalls mit Hydroxy, Cyano, COOH oder CO-NH₂ substituiert sein kann,
ein O-C₅-C₁₆-Heteroaryl, welches gegebenenfalls mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein C₆-C₁₂-Aryl, welches gegebenenfalls ein-, zwei- oder dreifach, gleich oder verschieden mit Halogen, mit C₁-C₄-Alkyl, C₃-C₆-Cycloalkyl, C₁-C₄-Acyl, C₁-C₄-Alkoxy, C₆-C₁₂-Aryl, C₅-C₁₂-Heteroaryl, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, CO-NH(C₅-C₁₂-Heteroaryl), NH-CO(C₁-C₄-Alkyl), CH₂-NH-CO(C₁-C₄-Alkyl), NH-CO(C₅-C₁₂-Heteroaryl), CH₂-NH-CO(C₅-C₁₂-Heteroaryl), Styryl, oder mit -S(O)ᵣ-CH₃, wobei r für 0 - 2 steht, substituiert sein kann oder zwei benachbarte Positionen durch -O-CH₂-O- oder -O-C(CH₃)₂-O- substituiert sein können,
ein monocyclisches C₅-C₇-Heteroaryl, welches gegebenenfalls ein-, zwei- oder dreifach, gleich oder verschieden mit C₁-C₄-Alkyl substituiert sein kann,
unter der Voraussetzung, dass R² Cyano bedeutet oder wenn R¹ und/oder R² gleich oder verschieden ein C₁-C₄-Akylrest ist, wobei mindestens einer der Reste mindestens einfach substituiert ist,
ein monocyclisches C₅-C₇-Heteroaryl, welches mindestens ein-, zwei- oder dreifach, gleich oder verschieden mit Halogen, CF₃, C₁-C₄-Acyl, C₁-C₄-Alkoxy, Hydroxy, CH₂-OH, Cyano, CO₂-(C₁-C₄- Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann,
ein bi- oder tricyclisches C₈-C₁₂-Heteroaryl, welches gegebenenfalls ein-, zwei- oder dreifach, gleich oder verschieden mit Halogen, mit C₁-C₄-Alkyl, C₁-C₄-Acyl, C₁-C₄-Alkoxy, Hydroxy, Cyano, CO₂-(C₁-C₄-Alkyl), N-(C₁-C₄-Alkyl)₂, COOH, CO-NH₂, CO-NH(C₁-C₄-Alkyl) oder CO-N(C₁-C₄-Alkyl)₂ substituiert sein kann oder
ein C₃-C₆-Cycloalkyl, welches gegebenenfalls ein-, zwei- oder dreifach, gleich oder verschieden mit Halogen, mit C₁-C₄-Alkyl, Hydroxy, Cyano, CO₂-(C₁-C₄-Alkyl), C₁-C₄-Acyl, N-(C₁-C₄-Alkyl)₂, COOH, CO-NH₂, CO-NH(C₁-C₄-Alkyl), CO-N(C₁-C₄-Alkyl)₂ oder C₁-C₄-Alkoxy substituiert sein kann,
R⁴ und R⁵ entweder in ortho-, meta- oder meta,para-Stellung zueinander stehen und gemeinsam die Bedeutung haben -O-CO-S-, -S-CO-O-, CH₂-CO-O-, O-CO-CH₂-, -CH₂-CO-NH-, -NH-CO-CH₂-, -O-CO-NH-, -NH-CO-O-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
Y eine -(CH₂)₂-Gruppe,
bedeuten, sowie deren Isomere, Diastereomere, Enantiomere und Salze bzw. Cyclodextrinclathrate.

7. Verbindunden gemäß der Ansprüche 1-6, wobei,
A einen Phenyl-, Naphthyl- oder C₅-C₁₂-Heteroarylrest, der gegebenenfalls ein- oder zweifach mit R⁴ und/oder R⁵ substituiert sein kann,
R¹ ein C₁-C₄-Alkylrest,
R² ein Wasserstoff, Fluor, Chlor, Brom, Methyl, Ethyl oder Trifluormethyl,
R³ ein Wasserstoff, Methyl, Ethyl, Trifluormethyl, Cyano, Chlor oder Brom,
R⁴ ein Wasserstoff, Halogen, Amino,
ein C₁-C₄-Acyl- oder NH-CO-C₁-C₄-Alkylrest,
ein C₁-C₄-Alkyl, welches gegebenenfalls ein- oder zwei-, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls einfach mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-Phenyl, welches gegebenenfalls ein- oder zweifach mit Hydroxy, Cyano, COOH oder CO-NH₂ substituiert sein kann,
ein CH₂O-Phenyl, welches gegebenenfalls ein- oder zweifach mit Hydroxy, Cyano, COOH oder CO-NH₂ substituiert sein kann,
ein O-C₅-C₇-Heteroaryl, welches gegebenenfalls ein- oder zweifach mit Hydroxy, Cyano, COOH oder CO-NH₂ substituiert sein kann, ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein Phenyl, welches gegebenenfalls ein- oder zweifach, gleich oder verschieden mit Halogen, mit C₁-C₄-Alkyl, C₁-C₄-Acyl, C₁-C₄-Alkoxy, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, NH-CO(C₁-C₄-Alkyl), CH₂-NH-CO(C₁-C₄-Alkyl) substituiert sein kann oder zwei benachbarte Positionen durch -O-CH₂-O- oder -O-C(CH₃)₂-O- substituiert sein können,
R⁵ ein Wasserstoff, Halogen, Amino,
ein C₁-C₄-Acyl- oder NH-CO-C₁-C₄-Alkylrest,
ein C₁-C₄-Alkyl, welches gegebenenfalls ein- oder zwei-, gleich oder verschieden mit C₁-C₄-Acyl, C₁-C₄-Alkoxy, Hydroxy, Cyano, CO₂-(C₁-C₄-Alkyl), N-(C₁-C₄-Alkyl)₂, C₅-C₁₂-Heteroaryl, COOH, CO-NH₂, CO-NH(C₁-C₄-Alkyl) oder mit CO-N(C₁-C₄-Alkyl)₂ substituiert sein kann,
ein C₁-C₄-Alkoxy, welches gegebenenfalls einfach mit Hydroxy, Cyano, CO₂-(C₁-C₄-Alkyl), N-(C₁-C₄-Alkyl)₂, NH-C₃-C₆-Cycloalkyl, COOH, CO-NH₂, CO-NH(C₁-C₄-Alkyl) oder mit CO-N(C₁-C₄-Alkyl)₂ substituiert sein kann,
ein O-Phenyl, welches gegebenenfalls ein- oder zweifach mit Hydroxy, Cyano, COOH oder CO-NH₂ substituiert sein kann,
ein CH₂O-Phenyl, welches gegebenenfalls ein- oder zweifach mit Hydroxy, Cyano, COOH oder CO-NH₂ substituiert sein kann,
ein O-C₅-C₇-Heteroaryl, welches gegebenenfalls ein- oder zweifach mit Hydroxy, Cyano, COOH oder CO-NH₂ substituiert sein kann,
ein Hydroxy, Cyano, O-CO-(C₁-C₄-Alkyl), CO-NH(C₅-C₁₂-Heteroaryl), NH-(C₁-C₄-Alkyl), N-(C₁-C₄-Alkyl)₂,
ein Phenyl, welches gegebenenfalls ein- oder zweifach, gleich oder verschieden mit Halogen, mit C₁-C₄-Alkyl, C₁-C₄-Acyl, C₁-C₄-Alkoxy, Hydroxy, CH₂-OH, Cyano, CH₂-CN, Amino, CO₂-(C₁-C₄-Alkyl), N-(C₁-C₄-Alkyl)₂, NHSO₂CH₃, SO₂NH₂, SO₂NH(C₁-C₄-Alkyl), SO₂N(C₁-C₄-Alkyl)₂, COOH, CO-NH₂, CO-NH(C₁-C₄-Alkyl), CO-N(C₁-C₄-Alkyl)₂, NH-CO(C₁-C₄-Alkyl), CH₂-NH-CO(C₁-C₄-Alkyl) substituiert sein kann oder zwei benachbarte Positionen durch -O-CH₂-O- oder -O-C(CH₃)₂-O- substituiert sein können,
R⁴ und R⁵ entweder in ortho-, meta- oder meta,para-Stellung zueinander stehen und gemeinsam die Bedeutung haben -CH₂-CO-NH-, -NH-CO-CH₂-, -CO-CH₂-(CH₂)ₘ-, -CH₂-(CH₂)ₘ-CO-, -O-(CH₂)ₘ-O-, -O-C-(CH₃)₂-O-, -CH₂-(CH₂)ₘ-CH₂-, wobei m für 1 - 3 steht,
Y eine -(CH₂)₂-Gruppe,
bedeuten, sowie deren Isomere, Diastereomere, Enantiomere und Salze bzw. Cyclodextrinclathrate.

8. Verbindungen gemäß den Ansprüchen 1 - 7, ausgewählt aus einer Gruppe, die die folgenden Verbindungen enthält:
1. 3,4-Dimethoxy-N-[2-(2-methyl-1H-indol-3-yl)-ethyl]-benzamid
2. 5-Fluor-1H-indol-2-carbonsäure [2-(7-chlor-2-methyl-1H-indol-3-yl)-ethyl]-amid
3. 5-Fluor-1H-indol-2-carbonsäure [2-(7-chlor-4-fluor-2-methyl-1H-indol-3-yl)-ethyl]-amid
4. Biphenyl-3,4'-dicarbonsäure 4'-{[2-(7-chlor-4-fluor-2-methyl-1H-indol-3-yl)-ethyl]-amid} 3-methylamid
5. N-[2-(7-Chlor-2-methyl-1H-indol-3-yl)-ethyl]-6-(3-methylcarbamoyl-phenyl)-nicotinamid
6. 3'-Hydroxy-biphenyl-4-carbonsäure [2-(7-chlor-2-methyl-1H-indol-3-yl)-ethyl]-amid
7. N-[2-(7-Chlor-4-fluor-2-methyl-1H-indol-3-yl)-ethyl]-3,4-dimethoxy-benzamid
8. Biphenyl-3,4'-dicarbonsäure 3-methylamid 4'-{[2-(2-methyl-1H-indol-3-yl)-ethyl]-amid}
9. 5-Fluor-1H-indol-2-carbonsäure [2-(2-methyl-1H-indol-3-yl)-ethyl]-amid
10.5-Chlor-1H-indol-2-carbonsäure [2-(2-methyl-1H-indol-3-yl)-ethyl]-amid
11. N-[2-(2,4-Dimethyl-1H-indol-3-yl)-ethyl]-3,4-dimethoxy-benzamid
12. N-[2-(7-Chlor-2,4-dimethyl-1H-indol-3-yl)-ethyl]-3,4-dimethoxy-benzamid
13. N-[2-(7-Brom-2,4-dimethyl-1H-indol-3-yl)-ethyl]-3,4-dimethoxy-benzamid
14.3H-Benzotriazol-5-carbonsäure [2-(7-brom-2,4-dimethyl-1H-indol-3-yl)-ethyl]-amid
15.1H-Indol-2-carbonsäure [2-(7-brom-2,4-dimethyl-1H-indol-3-yl)-ethyl]-amid
16. Chinoxalin-6-carbonsäure [2-(7-brom-2,4-dimethyl-1H-indol-3-yl)-ethyl]-amid
17.3H-Benzotriazol-5-carbonsäure [2-(7-chlor-2,4-dimethyl-1H-indol-3-yl)-ethyl]-amid
18. Chinoxalin-6-carbonsäure [2-(7-chlor-2,4-dimethyl-1H-indol-3-yl)-ethyl]-amid
19.1H-Indol-2-carbonsäure [2-(7-chlor-2,4-dimethyl-1H-indol-3-yl)-ethyl]-amid
20. N-[2-(7-Brom-2-methyl-1H-indol-3-yl)-ethyl]-3,4-dimethoxy-benzamid
21. Biphenyl-3,4'-dicarbonsäure 4'-{[2-(7-brom-2-methyl-1H-indol-3-yl)-ethyl]-amid} 3-methylamid
22.5-Fluor-1H-indol-2-carbonsäure [2-(7-brom-2-methyl-1H-indol-3-yl)-ethyl]-amid
23. N-[2-(7-Chlor-2-trifluormethyl-1H-indol-3-yl)-ethyl]-3,4-dimethoxy-benzamid
24.5-Fluor-1H-indol-2-carbonsäure [2-(7-chlor-2-trifluormethyl-1H-indol-3-yl)-ethyl]-amid
25.5-Chlor-1H-indol-2-carbonsäure [2-(7-chlor-2-trifluormethyl-1H-indol-3-yl)-ethyl]-amid
26. Biphenyl-3,4'-dicarbonsäure 4'-{[2-(7-chlor-2-trifluormethyl-1H-indol-3-yl)-ethyl]-amid} 3-methylamid
27.5-Trifluormethoxy-1H-indol-2-carbonsäure [2-(7-chlor-2-trifluormethyl-1H-indol-3-yl)-ethyl]-amid
28. Benzofuran-2-carbonsäure [2-(7-chlor-2-trifluormethyl-1H-indol-3-yl)-ethyl]-amid
29. Benzo[b]thiophen-2-carbonsäure [2-(7-chlor-2-trifluormethyl-1H-indol-3-yl)-ethyl]-amid
30.5-Chlor-1H-indol-2-carbonsäure [2-(7-brom-2-methyl-1H-indol-3-yl)-ethyl]-amid
31.5-Chlor-1H-indol-2-carbonsäure [2-(7-chlor-2-methyl-1H-indol-3-yl)-ethyl]-amid
32. N-[2-(2-tert-Butyl-1H-indol-3-yl)-ethyl]-3,4-dimethoxy-benzamid
33.5-Trifluormethoxy-1H-indol-2-carbonsäure [2-(2-tert-butyl-1H-indol-3-yl)-ethyl]-amid
34.5-Chlor-1H-indol-2-carbonsäure [2-(2-tert-butyl-1H-indol-3-yl)-ethyl]-amid
35.5-Trifluormethyl-1H-indol-2-carbonsäure [2-(2-tert-butyl-1H-indol-3-yl)-ethyl]-amid
36.5-Brom-1H-indol-2-carbonsäure [2-(2-tert-butyl-1H-indol-3-yl)-ethyl]-amid
37.5-Fluor-1H-indol-2-carbonsäure [2-(2-tert-butyl-1H-indol-3-yl)-ethyl]-amid
38.2-Brom-N-[2-(2,7-dimethyl-1H-indol-3-yl)-ethyl]-4,5-dimethoxy-benzamid
39.1H-Indole-6-carbonsäure [2-(2,7-dimethyl-1H-indol-3-yl)-ethyl]-amid
40.Chinolin-5-carbonsäure [2-(2,7-dimethyl-1H-indol-3-yl)-ethyl]-amid
41.5-Phenyl-1H-pyrazol-4-carbonsäure [2-(2,7-dimethyl-1H-indol-3-yl)-ethyl]-amid
42. Pyridazin-4-carbonsäure [2-(2,7-dimethyl-1H-indol-3-yl)-ethyl]-amid
43.5-Phenyl-2H-pyrazol-3-carbonsäure [2-(2,7-dimethyl-1H-indol-3-yl)-ethyl]-amid
44. Pyrimidin-5-carbonsäure [2-(2,7-dimethyl-1H-indol-3-yl)-ethyl]-amid
45. Pyrazolo[1,5-a]pyridin-2-carbonsäure [2-(2,7-dimethyl-1H-indol-3-yl)-ethyl]-amid
46. Benzo[b]thiophen-5-carbonsäure [2-(2,7-dimethyl-1H-indol-3-yl)-ethyl]-amid
47. Chinoxalin-2-carbonsäure [2-(2,7-dimethyl-1H-indol-3-yl)-ethyl]-amid
48. N-[2-(2,7-Dimethyl-1H-indol-3-yl)-ethyl]-3-fluor-2-methoxy-benzamid
49.3-Chlor-N-[2-(2,7-dimethyl-1H-indol-3-yl)-ethyl]-2-methyl-benzamid
50.3-Chlor-N-[2-(2,7-dimethyl-1H-indol-3-yl)-ethyl]-2-fluor-benzamid
51. N-[2-(2,7-Dimethyl-1H-indol-3-yl)-ethyl]-4-phenoxy-benzamid
52.Thiazol-4-carbonsäure [2-(2,7-dimethyl-1H-indol-3-yl)-ethyl]-amid
53.2-Chlor-N-[2-(2,7-dimethyl-1H-indol-3-yl)-ethyl]-3-methyl-benzamid
54.Chinolin-4-carbonsäure [2-(2,7-dimethyl-1H-indol-3-yl)-ethyl]-amid
55. N-[2-(2,7-Dimethyl-1H-indol-3-yl)-ethyl]-3-hydroxy-benzamid
56.4-Hydroxy-2-phenyl-2H-pyrazol-3-carbonsäure [2-(2,7-dimethyl-1H-indol-3-yl)-ethyl]-amid
57. Benzo[b]thiophen-3-carbonsäure [2-(2,7-dimethyl-1H-indol-3-yl)-ethyl]-amid
58.1H-Indol-2-carbonsäure [2-(2,7-dimethyl-1H-indol-3-yl)-ethyl]-amid
59.Chinolin-2-carbonsäure [2-(2,7-dimethyl-1H-indol-3-yl)-ethyl]-amid
60. Furan-2-carbonsäure [2-(2,7-dimethyl-1H-indol-3-yl)-ethyl]-amid
61.2-Chlor-N-[2-(2,7-dimethyl-1H-indol-3-yl)-ethyl]-nicotinamid
62. Chinolin-3-carbonsäure [2-(2,7-dimethyl-1H-indol-3-yl)-ethyl]-amid
63.5-Brom-N-[2-(2,7-dimethyl-1H-indol-3-yl)-ethyl]-nicotinamid
64.1H-Indol-3-carbonsäure [2-(2,7-dimethyl-1H-indol-3-yl)-ethyl]-amid
65.4-Benzyloxy-N-[2-(2,7-dimethyl-1H-indol-3-yl)-ethyl]-benzamid
66.3-Brom-thiophen-2-carbonsäure [2-(2,7-dimethyl-1H-indol-3-yl)-ethyl]-amid
67.2-Methyl-furan-3-carbonsäure [2-(2,7-dimethyl-1H-indol-3-yl)-ethyl]-amid
68.4-Oxo-4,5,6,7-tetrahydro-benzo[b]thiophen-2-carbonsäure [2-(2,7-dimethyl-1H-indol-3-yl)-ethyl]-amid
69.2-Benzo[1,2,5]thiadiazol-4-yl-N-[2-(2,7-dimethyl-1H-indol-3-yl)-ethyl]-acetamid
70.1H-Imidazol-4-carbonsäure [2-(2,7-dimethyl-1H-indol-3-yl)-ethyl]-amid
71.4'-Brom-biphenyl-2-carbonsäure [2-(2,7-dimethyl-1H-indol-3-yl)-ethyl]-amid
72. N-[2-(2,7-Dimethyl-1H-indol-3-yl)-ethyl]-2-fluor-6-iod-benzamid
73.3'-[2-(2,7-Dimethyl-1H-indol-3-yl)-ethylcarbamoyl]-biphenyl-2-carbonsäure methylester
74.2,3-Dihydro-benzofuran-7-carbonsäure [2-(2,7-dimethyl-1H-indol-3-yl)-ethyl]-amid
75. N-[2-(2,7-Dimethyl-1H-indol-3-yl)-ethyl]-3-fluor-2-methyl-benzamid
76. N-[2-(2,7-Dimethyl-1H-indol-3-yl)-ethyl]-2,5-dimethyl-benzamid
77.5-Acetyl-thiophen-2-carbonsäure [2-(2,7-dimethyl-1H-indol-3-yl)-ethyl]-amid
78. N-[2-(2,7-Dimethyl-1H-indol-3-yl)-ethyl]-2-fluor-3-methoxy-benzamid
79.Chinolin-8-carbonsäure [2-(2,7-dimethyl-1H-indol-3-yl)-ethyl]-amid
80. N-[2-(2,7-Dimethyl-1H-indol-3-yl)-ethyl]-4-methylsulfanyl-benzamid
81.2-Phenyl-2H-pyrazol-3-carbonsäure [2-(2,7-dimethyl-1H-indol-3-yl)-ethyl]-amid
82.6-Phenyl-pyrimidin-4-carbonsäure [2-(2,7-dimethyl-1H-indol-3-yl)-ethyl]-amid
83.1-Methyl-1H-indol-3-carbonsäure [2-(2,7-dimethyl-1H-indol-3-yl)-ethyl]-amid
84. N-[2-(2,7-Dimethyl-1H-indol-3-yl)-ethyl]-2-phenoxymethyl-benzamid
85.2,5-Dimethyl-2H-pyrazol-3-carbonsäure [2-(2,7-dimethyl-1H-indol-3-yl)-ethyl]-amid
86.2,3-Dihydro-benzofuran-5-carbonsäure [2-(2,7-dimethyl-1H-indol-3-yl)-ethyl]-amid
87.5-Methoxy-1H-indol-2-carbonsäure [2-(2,7-dimethyl-1H-indol-3-yl)-ethyl]-amid
88.5-Methoxy-1H-indol-2-carbonsäure [2-(2-methyl-7-trifluormethyl-1H-indol-3-yl)-ethyl]-amid
89.1H-Indol-2-carbonsäure [2-(2-methyl-7-trifluormethyl-1H-indol-3-yl)-ethyl]-amid
90.5-Fluor-1H-indol-2-carbonsäure [2-(2-methyl-7-trifluormethyl-1H-indol-3-yl)-ethyl]-amid
91.5-Methyl-1H-indol-2-carbonsäure [2-(2-methyl-7-trifluormethyl-1H-indol-3-yl)-ethyl]-amid
92.6-Methoxy-1H-indol-2-carbonsäure [2-(2-methyl-7-trifluormethyl-1H-indol-3-yl)-ethyl]-amid
93.4-Methyl-1H-indol-2-carbonsäure [2-(2-methyl-7-trifluormethyl-1H-indol-3-yl)-ethyl]-amid
94.4-Methoxy-1H-indol-2-carbonsäure [2-(2-methyl-7-trifluormethyl-1H-indol-3-yl)-ethyl]-amid
95.4-Fluor-1H-indol-2-carbonsäure [2-(2-methyl-7-trifluormethyl-1H-indol-3-yl)-ethyl]-amid
96.6-Fluor-1H-indol-2-carbonsäure [2-(2-methyl-7-trifluormethyl-1H-indol-3-yl)-ethyl]-amid
97.6-Methyl-1H-indol-2-carbonsäure [2-(2-methyl-7-trifluormethyl-1H-indol-3-yl)-ethyl]-amid
98.7-Methyl-1H-indol-2-carbonsäure [2-(2-methyl-7-trifluormethyl-1H-indol-3-yl)-ethyl]-amid
99. N-[2-(7-Cyano-2,4-dimethyl-1H-indol-3-yl)-ethyl]-3,4-dimethoxy-benzamid
100. N-[2-(7-Chlor-2-methyl-1H-indol-3-yl)-ethyl]-3,4-dimethoxy-benzamid
101. Biphenyl-4,4'-dicarbonsäure 4'-{[2-(7-chlor-2-methyl-1H-indol-3-yl)-ethyl]-amid} 4-methylamid
102. Biphenyl-3,4'-dicarbonsäure 4'-{[2-(7-chlor-2-methyl-1H-indol-3-yl)-ethyl]-amid} 3-methylamid
103. N-[2-(7-Brom-2,4-dimethyl-1H-indol-3-yl)-ethyl]-3,4-dimethoxy-benzamid
104. N-[2-(4,7-Dichlor-2-methyl-1H-indol-3-yl)-ethyl]-3,4-dimethoxy-benzamid
105. Biphenyl-3,4'-dicarbonsäure 4'-{[2-(4,7-dichlor-2-methyl-1H-indol-3-yl)-ethyl]-amid} 3-methylamid

9. Verwendung der Verbindungen gemäß Formel I für die Herstellung eines Arzneimittels.

10. Verwendung der Arzneimittel gemäß Anspruch 9, **dadurch gekennzeichnet, dass** das Arzneimittel zur Behandlung und Prophylaxe von Erkrankungen verwendet wird.

11. Verwendung der Arzneimittel gemäß Anspruch 9, zur Behandlung und Prophylaxe von Erkrankungen, die im Zusammenhang mit dem EP₂-Rezeptor stehen.

12. Verwendung der Arzneimittel gemäß Anspruch 9 zur Behandlung und Prophylaxe von Fertilitätsstörungen.

13. Verwendung der Arzneimittel gemäß Anspruch 9 zur Behandlung und Prophylaxe von Menstruationsbeschwerden, wobei es sich bei den Menstruationsbeschwerden um starke und langanhaltende Blutungen handeln kann.

14. Verwendung der Arzneimittel gemäß Anspruch 9 zur Behandlung und Prophylaxe von Endometriose.

15. Verwendung der Arzneimittel gemäß Anspruch 9 zur Behandlung und Prophylaxe von Schmerz.

16. Verwendung der Verbindungen gemäß Anspruch 1 - 8 und der Arzneimittel gemäß Anspruch 9 zur Fertilitätskontrolle/Kontrazeption.

17. Verwendung der Arzneimittel gemäß Anspruch 9 zur Behandlung und Prophylaxe von Osteoporose.

18. Verwendung der Arzneimittel gemäß Anspruch 9 zur Behandlung und Prophylaxe von Krebs.

19. Verwendung der Arzneimittel gemäß Anspruch 9 zur Behandlung und Prophylaxe der Alzheimerschen Erkrankung.

20. Verwendung der Arzneimittel gemäß Anspruch 9 zur Behandlung und Prophylaxe der Parkinsonschen Erkrankung.

21. Verwendung der Arzneimittel gemäß Anspruch 9 zur Behandlung und Prophylaxe von entzündlichen Darmerkrankungen, wobei es sich bei den entzündichen Darmerkrankungen um Morbus Crohn und Colitis Ulcerosa handeln kann.

22. Verwendung der Arzneimittel gemäß Anspruch 9 zur Behandlung und Prophylaxe von polizystischen Nierenerkrankungen.

23. Verwendung der Arzneimittel gemäß Anspruch 9 zur Behandlung und Prophylaxe von Artherosklerose.

24. Arzneimittel enthaltend eine Verbindung der allgemeinen Formel (I) in Kombination mit einem COX-Inhibitor zur Behandlung von Erkrankungen, wobei es sich bei den COX-Inhibitoren um beispielsweise um Aspirin, Naproxen, Indomethacin, Meloxicam, Celecoxib (4-[5-(4-Methylphenyl)-3-(Trifluoromethyl)-1H-Pyrazol-1-yl]Benzenesulfonamid), Ibuprofen, Parecoxib (N-[4-(5-Methyl-3-Phenyl-4-Isoxazolyl)Phenyl]Sulfonylpropionamide), Rofecoxib (4-(4-Mesylphenyl)-3-Phenylfuran-2(5H)-one), Valdecoxib (4-[5-Methyl-3-Phenyl-4-isoxazoyl)Benzenesulfonamide), NS-398 (N-Methyl-2-Cyclohexanoxy-4-Nitrobenzenesulfonamide), Lumiracoxib [2-(2'-chloro-6'-fluorophenyl)-amino-5-methylbenzeneacetic, Ceracoxib und Etoricoxib handelt.

25. Arzneimittel gemäß Anspruch 24, wobei es sich bei den Erkrankungen um Fertilitätsstörungen, Menstruationsbeschwerden" Endometriose, Schmerz, Oesteoporose, Krebs, Morbus Alzheimer, Morbus Parkinson, entzündliche Darmerkrankungen, polyzystische Nierenerkrankungen, Arteriosklerose handeln kann bzw. die zur Fertilitätskontrolle eingesetzt werden kann.

26. Verwendung der Arzneimittel gemäß Anspruch 8 zur Behandlung und Prophylaxe von Infektionen der Atmungswege.

27. Verwendung der Verbindungen der allgemeinen Formel I, gemäß den Ansprüchen 1 - 8, in Form eines pharmazeutischen Präparates für die enterale, parenterale, vaginale und orale Applikation.

28. Verfahren zur Herstellung der Verbindungen der allgemeinen Formel (I), **dadurch gekennzeichnet, dass** eine Verbindung der Formel II, 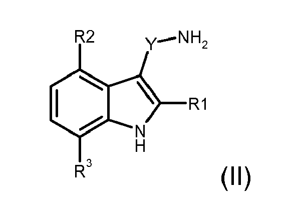 worin R¹, R², R³ und Y die in Anspruch 1 genannte Bedeutungen haben mit einer Säure der allgemeinen Formel III 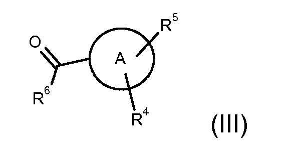 worin A, R⁴ und R⁵ die in Anspruch 1 angegebene Bedeutung aufweist und R⁶ eine Hydroxygruppe, ein Chlor- oder Bromatom oder ein C₁-C₆-Akylrest sein kann, bevorzugt ist Wasserstoff, Chlor, der Methyl- oder Ethyl-Rest, umgesetzt werden und/oder gegebenenfalls benötigte Schutzgruppen anschließend gespalten und/oder gegebenenfalls vorhandene Doppelbindungen hydriert werden und/oder ein Bromid gegen ein Cyanid substituiert wird, zu den Verbindungen der allgemeinen Formel (I) umgesetzt werden.
